# EUROPEAN PATENT APPLICATION

(11) **EP 2 599 860 A1**
(43) Date of publication of application: **05.06.2013**
(21) Application number: 11786682.2
(22) Date of filing: 25.05.2011
(51) Int. Cl.: C12N 5/10, A01K 67/027, A61K 39/00, C12N 15/09, C12Q 1/02, G01N 33/15, G01N 33/50

(54) **INDUCED MALIGNANT STEM CELLS OR PRE-INDUCTION CANCER STEM CELLS CAPABLE OF SELF-REPLICATION OUTSIDE OF AN ORGANISM, PRODUCTION METHOD FOR SAME, AND PRACTICAL APPLICATION FOR SAME**

(30) Priority: 25.05.2010 JP 2010119385
(71) Applicant: National Cancer Center, Tokyo 104-0045 (JP)
(72) Inventor: ISHIKAWA, Tetsuya, Tokyo 104-0045 (JP)
(74) Representative: Cabinet Plasseraud
(86) International application number: PCT/JP2011/062006
(87) International publication number: WO 2011/148983

(57) **Abstract**

The present invention provides an induced cancer cell capable of self-replication in vitro which is useful in cancer therapy research and the research for cancer-related drug discovery, processes for production thereof, cancer cells induced by the malignant cells, and applications of these cells.

The present invention provides an induced cancer stem cell capable of proliferation (self-replication) in vitro, wherein the induced cancer stem cell has the following two characteristics:
(1) expressing the six genes (self-renewal related genes) POU5F1, NANOG, SOX2, ZFP42, LIN28, and TERT selected from a certain group of genes; and
(2) having an aberration which is either (a) a mutation in an endogenous tumor suppressor gene or (b) increased expression of an endogenous cancer-related gene.

## Description

### TECHNICAL FIELD

The present invention relates to induced precancer stem cells or induced malignant stem cells, more particularly, to inducedprecancer stem cells or induced malignant stem cells that are capable of self-renewal *in vitro,* further characterized in that they have aberrations such as mutations in endogenous tumor suppressor genes or increased expression of endogenous cancer-related genes and that self-renewal related genes such as *POU5F1, NANOG, SOX2, ZFP42, LIN28,* and *TERT* are expressed therein (these cells are hereinafter collectively referred to as "induced cancer stem cells"), as well as processes for production thereof, and applications of these cells.

### BACKGROUND ART

In recent years, research on embryonic stem cells (also called "ES cells" but hereinafter referred to as "embryonic stem cells"), as well as research on somatic cell clones directed to the creation of somatic cell clone embryonic stem cells and somatic cell clone animals have led to the postulation that epigenetics (DNA methylation and histone modification) is capable of reprogramming (also called "initializing" but hereinafter referred to as "reprogramming"). As a matter of fact, there is a report of experimental results stating that when the nucleus of a mouse melanoma cell which is a cancer cell was transplanted into an enucleated oocyte, the latter initiated embryogenesis, with the embryonic stem cell from the embryo differentiating into such cells as melanocytes, lymphocytes, and fibroblasts (Non-Patent Document 1).

It has recently been reported that by transduction of *OCT3*/*4* (sometimes designated as *"OCT3", "OCT4"* or *"POU5F1"), SOX2, KLF4,* and *c-MYC* (Patent Document 1) or by transduction of *OCT3*/*4, SOX2,* and *KLF4* in the presence of a basic fibroblast growth factor (Non-Patent Document 2), induced pluripotent stem cells (also called "iPS cells") which are as undifferentiated as embryonic stem cells can be prepared from human somatic cells as the result of reprogramming (Patent Document 2). Human induced pluripotent stem cells are known to have two characteristic features, (1) pluripotency for differentiation into three germ layers which are capable of differentiating into all cells that form a body and (2) self-renewal ability by which the cells can be expanded in passage culture without limit in a culture dish under culture conditions for self-renewal of human embryonic stem cells while remaining undifferentiated. It also has been reported that such human induced pluripotent stem cells are very similar to human embryonic stem cells in terms of morphology, gene expression, cell surface antigen, long-term self-renewing ability, and teratoma (differentiation *in vivo* into three germ layers) forming ability (Non-Patent Documents 3 and 4), and that the genotypes of HLA are completely identical to those of somatic cells which are derived cells (Non-Patent Document 4). In connection with the method of preparing these cells, it is held that a differentiated somatic cell can be "reprogrammed" to an induced pluripotent stem cell by simply introducing the aforementioned genes, (i.e., *OCT3*/*4, SOX2, KLF4,* and *c-MYC,* or *OCT3*/*4, SOX2,* and *KLF4* in the presence of bFGF).

If there occurs a genetic mutation and/or epigenetic aberration, gene expression will increase or decrease or even disappear, and this aberration may generate the carcinogenesis of the cells. It is therefore postulated that by using the above-described reprogramming technology, the cancer cell having various aberrations will be reprogrammed and be returned to the normal cell, losing its cancerous properties.

As a matter of fact, a report recently made at a meeting of the International Society for Stem Cell Research (ISSCR) states as follows: "When two kinds of chemical substance including a cancer-control agent (noncyclic retinoid and tolrestat) were added to cancer stem cells derived from a human hepatocarcinoma cell line (HuH7-derived CD133 positive cells) on a culture dish, 85-90% of the cancer cells were returned normal hepatocytes in 2 days. Upon further addition of two genes *(SOX2* and *KLF4)* and two chemical substances (5-AZAC and TSA), the hepatocytes became induced pluripotent stem cells which, by means of a protocol for differentiation into hepatocytes, could successfully be differentiated into hepatocytes (AFP or ALB positive cells." (See Non-Patent Document 5). There are also a paper describing a successful reprogramming of mouse melanoma cells as cancer cells to induced pluripotent stem cells (Non-Patent Document 6), as well as a report disclosing that as the result of reprogramming by transfer of *OCT3*/*4, SOX2, KLF4,* and *c*-*MYC*, induced pluripotent stem cells having lost BCR-ABL tyrosine kinase dependency were prepared from chronic myeloid leukemia (CML) having BCR-ABL tyrosine kinase activity as an etiology of cancer (Non-Patent Document 7). According to yet another report, when *OCT3*/*4, SOX2, KLF4,* and *c-MYC* were transduced into a cancer cell line, it was reprogrammed to lose drug resistance and tumorigenicity but an extended culture caused canceration involving the activation of exogenous *c-MYC* (Non-Patent Document 8).

The cancer cell lines used in conventional cancer research are those which are first established by culture for cell immortalization through forced expression of SV40, the E6, E7 of HPV, or TERT by tumorigenesis through transfer of oncogenes such as *c-MYC* and *RAS* and further cultured in common conventional media.

However, the cancer cell lines established in common conventional media significantly develop post-culture artificial chromosomal aberrations (e.g. dislocation and deletion), genetic aberrations (genetic mutations), and epigenetic aberrations which may lead to abnormal gene expression and this presents a problem that the aberrations in precancerous cells or cancer cells which were inherent causes of carcinogenesis *in vivo* are difficult to retain as they are. None of these cell lines have been established by culture that permits self-renewal *in vitro.*

In cancer therapy research and the research for cancer-related drug discovery, even if the genetic or epigenetic aberrations in the cancer cell lines established in such conventional media are analyzed, it is extremely difficult to determine whether those aberrations are inherent in mammalian precancerous cells or cancer cells or post-culture artificial aberrations and, hence, it has been impossible to search for cancer etiology, search for a target in drug discovery, screen for an effective anti-cancer therapeutic drug, and the like in appropriate manners.

A further problem is that despite the fact that cancer stem cells are highlighted as an important target in drug discovery, the cancer cells that are contained in a fresh cancer tissue make up a hierarchical and heterogeneous cell population and it is not clear which cancer cells are cancer stem cells. Recently, there was reported a study for identifying cancer stem cells from a cancer cell line or a primary cultured cancer cells (Non-Patent Document 9) but there is no report of successful *self-renewal in vitro* and expansion culture of monoclonal cancer cells, nor has been reported any technology by which they can be self-renewed and subjected to *in vitro* expansion culture until they reach the number necessary for application in drug discovery and for use in cancer research.

### CITATION LIST

### PATENT LITERATURE

Patent Document 1: JP 2008-283972 A
Patent Document 2: JP 2008-307007 A

### NON-PATENT LITERATURE

Non-Patent Document 1: Hochedlinger K, Jaenisch R et al., Genes Dev., 2004, 18:1875-1885
Non-Patent Document 2: Nakagawa M, Yamanaka S et al." Nat Biotechnol., 2008:26,101-106
Non-Patent Document 3: Takahashi K, Yamanaka S, Cell, 2007, 131:861-872
Non-Patent Document 4: Masaki H, Ishikawa T et al., Stem Cell Res., 2008, 1:105-115
Non-Patent Document 5: International Society for Stem Cell Research, 2009, Abstract Number 1739 (page 285)
Non-Patent Document 6: Utikal J et al., J Cell Sci., 2009, 122(Pt 19):3502-3510
Non-Patent Document 7: Carette JE et al., Blood, 2010, 115:4039-4042
Non-Patent Document 8: Nagai K et al., Biochem Biophys Res Commun., 2010, 395:258-263
Non-Patent Document 9: Visvader JE, Lindeman GJ, Nat Rev Cancer., 2008, 8:755-768

### SUMMARY OF INVENTION

### TECHNICAL PROBLEM

Therefore, an object of the present invention is to provide an induced cancer stem cell capable of self-renewal *in vitro* having specific genetic mutations or aberrations in gene expression that are related to carcinogenicity, and a process for producing such induced cancer stem cell.

Another object of the present invention is to provide a method in which the induced cancer stem cell capable of self-renewal *in vitro* is used to perform screening as for a target in anti-cancer drug discovery, an anti-cancer therapeutic drug or a cancer diagnostic drug, or to prvide a method in which the induced cancer stem cell capable of self-renewal *in vitro* is used to prepare an anti-cancer vaccine.

A further object of the present invention is to provide a method of preparing a cancer model animal in which the induced cancer stem cell capable of self-renewal *in vitro* is transplanted to an experimental animal.

### SOLUTION TO PROBLEM

In its first embodiment, the present invention provides an induced cancer stem cell capable of proliferation *in vitro,* wherein the induced cancer stem cell has the following two characteristics:
(1) expressing the six genes (self-renewal related genes) *POU5F1, NANOG, SOX2, ZFP42, LIN28,* and *TERT*; and
(2) having an aberration which is either (a) a mutation in an endogenous tumor suppressor gene or (b) increased expression of an endogenous cancer-related gene.

In this first embodiment of the present invention, it is preferred that the self-renewal related genes as referred to in (1) above are expressed in the induced cancer stem cell in amounts ranging from one-eighth to eight times the amounts of the genes that are expressed in an embryonic stem cell.

The induced cancer stem cell of the present invention may be such that in addition to the increased expressin of an endogenous cancer-related gene as referred to in (b), it has an increased gene expression occurring in at least one endogenous gene selected from the groups of genes consisting of a group of genes related to stress and toxicity, a group of genes for epigenetics of chromatin modifying enzyme, and a group of genes for stem cell transcription factor; alternatively, it may be such that in addition to the increased expressin of an endogenous cancer-related gene as referred to in (b), it has an increased gene expression occurring in at least one endogenous gene selected from the groups of hepatocyte-specific genes.

The induced cancer stem cell of the present invention may further express a gene characteristic of mesendodermal stem cells or endodermal stem cells.

In its second embodiment, the present invention provides a process for producing an induced cancer stem cell capable of self-renewal *in vitro* from a non-embryonic starter somaic cell selected from the group consisting of a somatic cell isolated from a mammal having (a) a mutation in a tumor suppressor gene and a somatic cell that is isolated from a carcinogenic mammal and which has an aberration which is either (a) a mutation in a tumor suppressor gene or (b) increased expressin of a cancer-related gene, the process being characterized by performing an induction step in which the starter somatic cell is brought to such a state that the genetic products of *POU5F1, KLF4,* and *SOX2* are present therein. When the cell is described as being "non-embryonic", it shall be construed as being neither an embryonic stem cell nor an embryo nor a germ cell nor a primordial germ cell.

In the present invention, the genetic products of *POU5F1, KLF4,* and *SOX2* may be such that their relative abundances in the starter somatic cell satisfy the relation of *POU5F1 > SOX2.*

Further in the present invention, it is preferred to use *POU5F1, KLF4,* and *SOX2* or genetic products of these genes, and these genes and their genetic products may be such that their ratio in use satisfies the relation *of POU5F1 > SOX2.*

In addition to the above-described induction step, the present invention may include the step of sorting a single cell in one well and proliferating the cell.

In addition to the above-described induction step, the present invnetion may further include a selection step in which the malignancy or a specific marker of the induced cancer stem cell capable of self-renewal *in vitro* is identified to select the cell of interest. This selection step may be a step in which a cell obtained by induction treatment of a non-embryonic starter somaic cell selected from the group consisting of a somatic cell isolated from a mammal having (a) a mutation in a tumor suppressor gene and a somatic cell that is isolated from a carcinogenic mammal and which has an aberration which is either (a) a mutation in a tumor suppressor gene or (b) increased expressin of a cancer-related gene is compared with an induced mesendodermal stem cell, an induced endodermal stem cell or an induced pluripotent stem cell as induced from a s reference omatic cell isolated from a mammal, or an embryonic stem cell, and the malignancy or a specific marker is identified to select the cell of interest.

In particular, the selection step may be such that the above-mentioned specific marker is identified to select the cell of interest by (b) increased expression of a cancer-related gene within at least one group of genes selected from the groups of genes consisting of a group of genes related to angiogenesis, a group of cancer-related pathway genes, a group of genes related to stromal barrier, a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer.

In its third embodiment, the present invention provides a method of screening that is selected from a method of screening for a target in anti-cancer drug discovery, a method of screening for an anti-cancer therapeutic drug, and a method of screening for a cancer diagnostic drug, wherein the method is characterized by using the induced cancer stem cell according to the first embodiment of the present invention.

In its fourth embodiment, the present invention provides a method of preparing an anti-cancer vaccine which is characterized by using the induced cancer stem cell according to the first embodiment of the present invention, and in its fifth embodiment, the present invention provides a method of preparing a cancer model animal which is characterized in that the induced cancer stem cell according to the first embodiment of the present invention is transplanted to a laboratory animal.

### ADVANTAGEOUS EFFECTS OF INVENTION

According to the present invention, induced cancer stem cells that have an aberration such as (a) a mutation in endogenous tumor suppressor genes or (b) increased expression of endogenous cancer-related genes and which also have self-renewal related genes such as *POU5F1, NANOG, SOX2, ZFP42, LIN28,* and *TERT* expressed therein, as well as processes for production thereof, and applications of these cells can be realized.

The induced cancer stem cells of the present invention not only maintain the aberration inherent in the starter somatic cell such as (a) a mutation in endogenous tumor suppressor genes or (b) increased expression of endogenous cancer-related genes but they also have a distinct feature of stem cells, i.e., being theoretically capable of self-renewal ithout limit. Hence, the induced cancer stem cells of the present invention can effectively be passage cultured for an extended period and can easily be induced to cancer cells having the properties of tissue cells and, as a result, they are extremely useful in cancer therapy research and the research for cancer-related drug discovery, as applied in methods of screening such as a method of screening for targets in anti-cancer drug discovery, a method of screening for anti-cancer therapeutic drugs, and a method of screening for cancer diagnostic drugs, as well as in methods of preparing anti-cancer vaccines and cancer model animals.

### BRIEF DESCRIPTION OF THE DRAWINGS

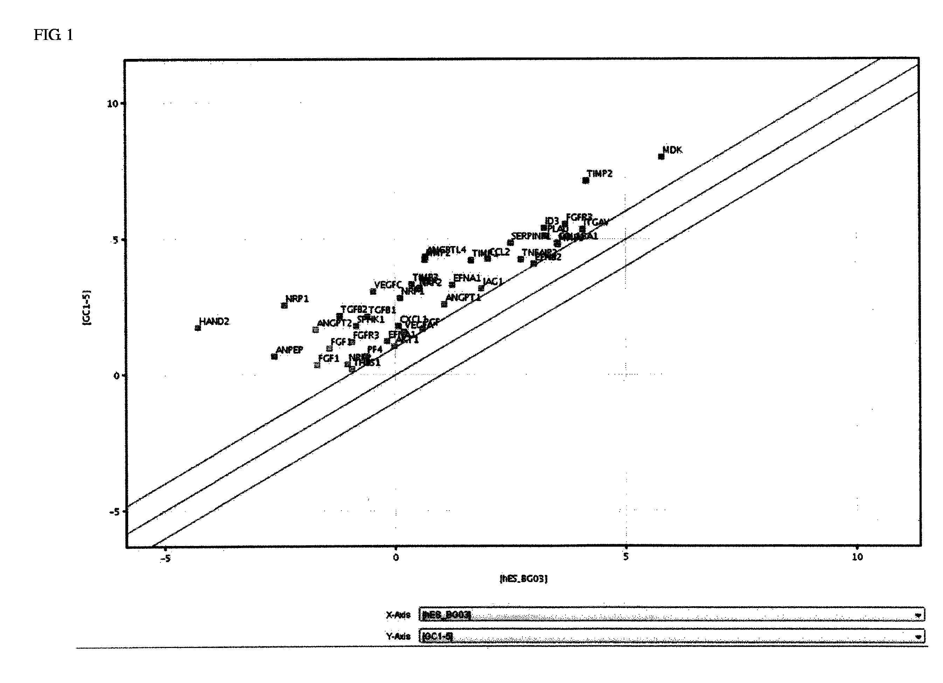

FIG. 1 is a diagram plotting genes related to angiogenesis that were expressed in the human induced malignant stem cell GC1-5 of the present invention in amounts more than twice the amounts expressed in the human embryonic stem cell hES_BG03 (GSM194391); the middle line, the upper line, and the lower line respectively indicate that the level of gene expression was comparable to, twice, and one half the level in the human embryonic stem cell.

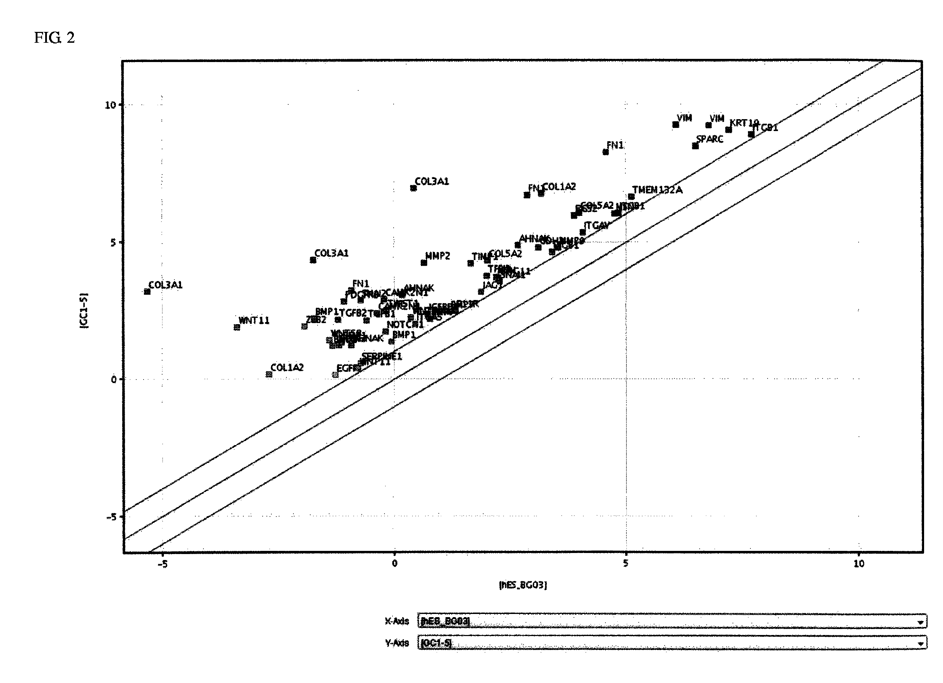

FIG. 2 is a diagram plotting genes related to epithelial-mesenchymal transition that were expressed in the human induced malignant stem cell GC1-5 of the present invention in amounts more than twice the amounts expressed in the human embryonic stem cell hES_BG03 (GSM194391); the middle line, the upper line, and the lower line respectively indicate that the level of gene expression was comparable to, twice, and one half the level in the human embryonic stem cell.

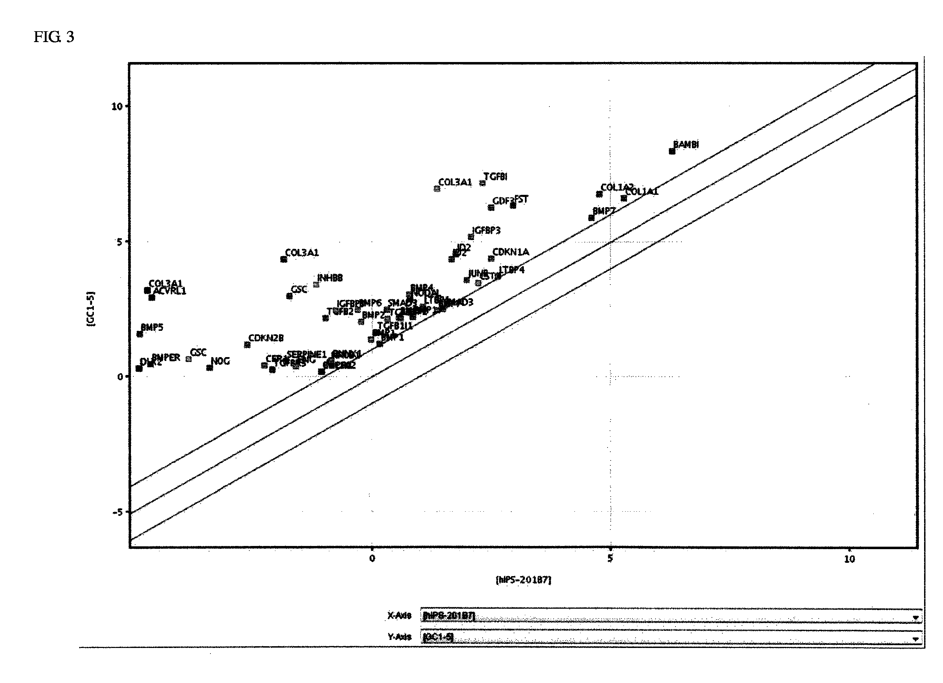

FIG. 3 is a diagram plotting genes related to TGF β/BMP signaling that were expressed in the human induced malignant stem cell GC1-5 of the present invention in amounts more than twice the amounts expressed in the human induced pluripotent stem cell hiPS-201B7; the middle line, the upper line, and the lower line respectively indicate that the level of gene expression was comparable to, twice, and one half the level in the human induced pluripotent stem cell.

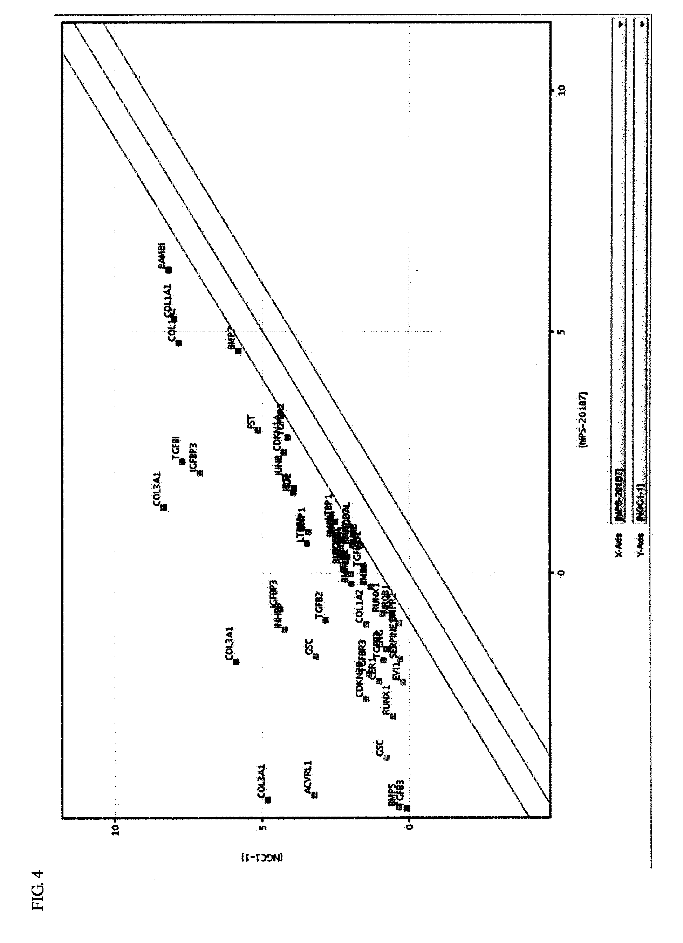

FIG. 4 is a diagram plotting genes related to tissue invasion/metastasis that were expressed in the human induced malignant stem cell NGC1-1 of the present invention in amounts more than twice the amounts expressed in the human induced pluripotent stem cell hiPS-201B7; the middle line, the upper line, and the lower line respectively indicate that the level of gene expression was comparable to, twice, and one half the level in the human induced pluripotent stem cell.

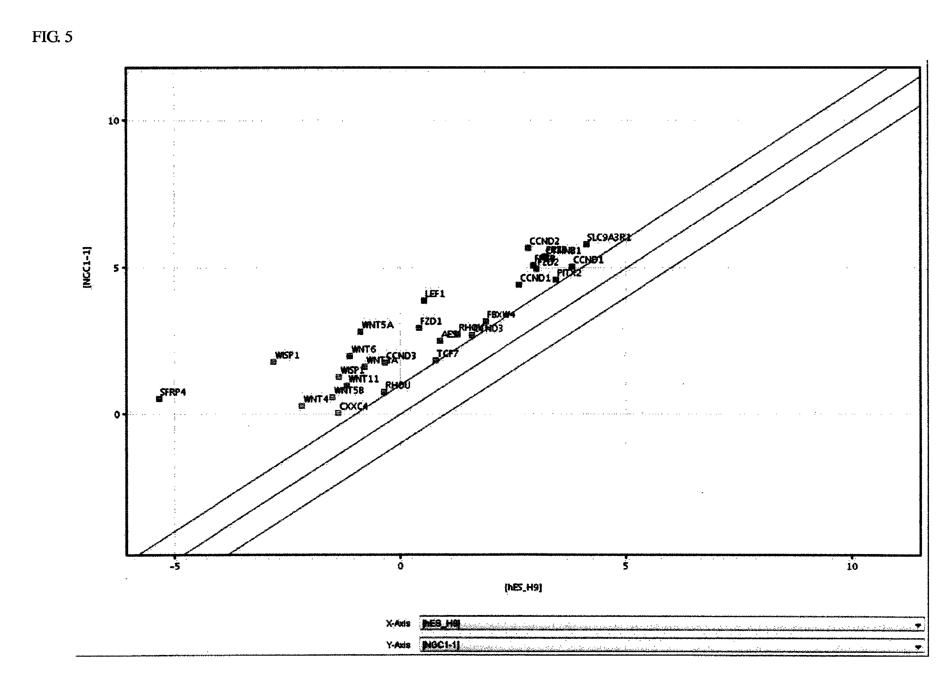

FIG. 5 is a diagram plotting genes related to Wnt signaling that were expressed in the human induced malignant stem cell NGC1-1 of the present invention in amounts more than twice the amounts expressed in the human embryonic stem cell hES_H9 (GSM194390); the middle line, the upper line, and the lower line respectively indicate that the level of gene expression was comparable to, twice, and one half the level in the human embryonic stem cell.

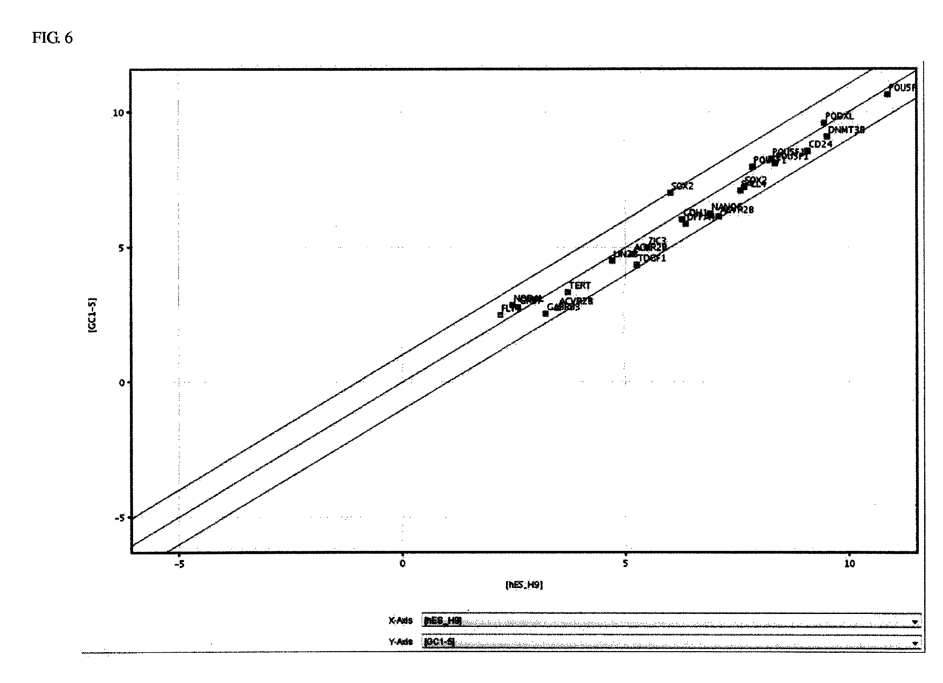

FIG. 6 is a diagram plotting self-renewal related genes that were expressed in the human induced malignant stem cell GC1-5 of the present invention in amounts almost comparable to (one half to twice) the amounts expressed in the human embryonic stem cell hES_H9 (GSM194390); the middle line, the upper line, and the lower line respectively indicate that the level of gene expression was comparable to, twice, and one half the level in the human embryonic stem cell.

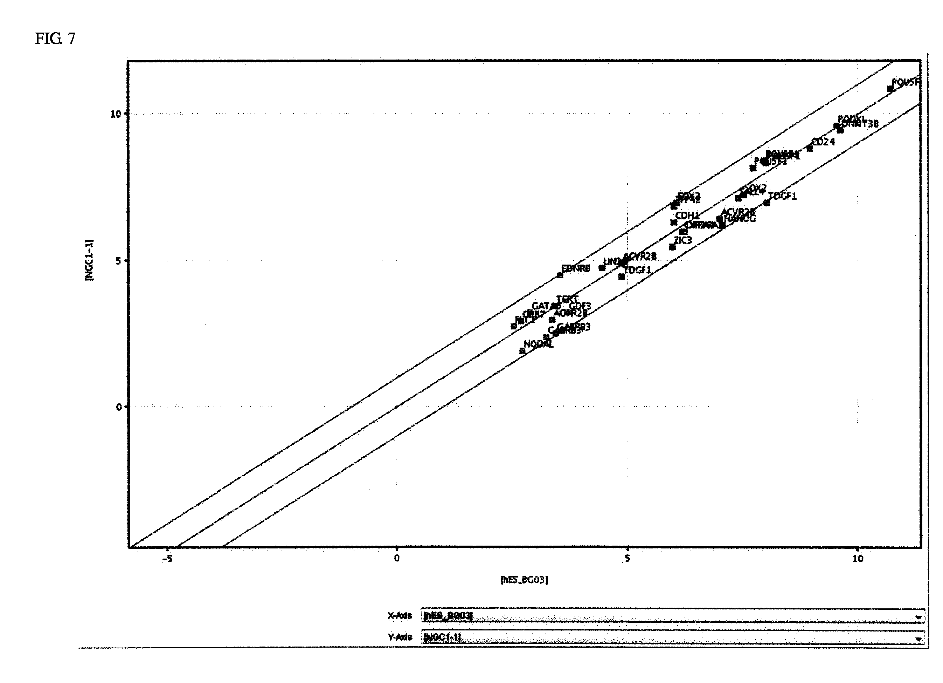

FIG. 7 is a diagram plotting self-renewal related genes that were expressed in the human induced malignant stem cell NGC1-1 of the present invention in amounts almost comparable to (one half to twice) the amounts expressed in the human embryonic stem cell hES_BG03; the middle line, the upper line, and the lower line respectively indicate that the level of gene expression was comparable to, twice, and one half the level in the human embryonic stem cell.

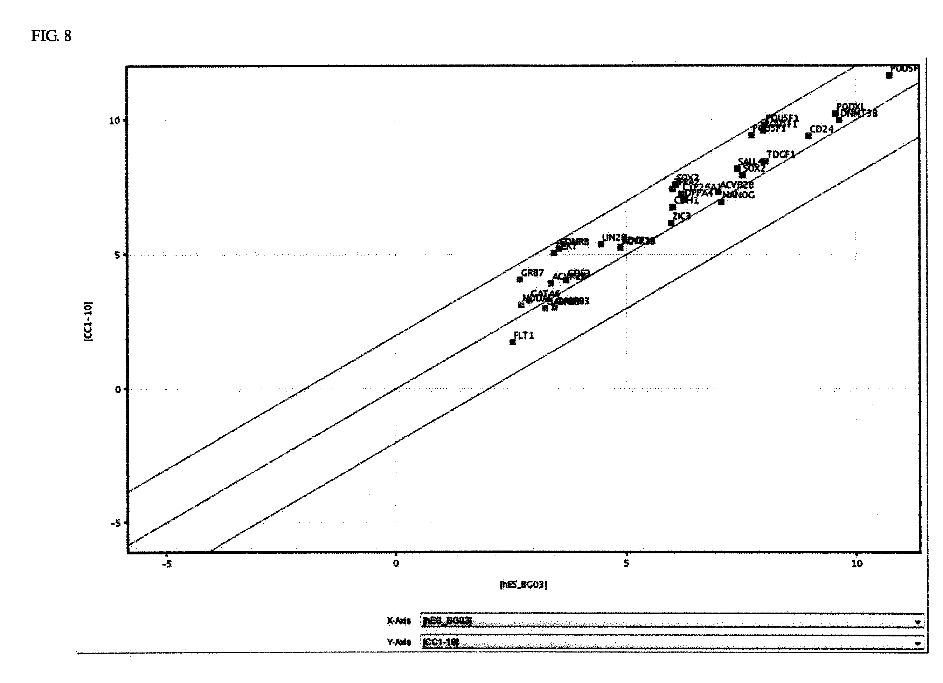

FIG. 8 is a diagram plotting self-renewal related genes that were expressed in the human induced malignant stem cell CC1-10 of the present invention in amounts almost comparable to (one fourth to four times) the amounts expressed in the human embryonic stem cell hES_BG03; themiddle line, the upper line, and the lower line respectively indicate that the level of gene expression was comparable to, four times, and one fourth the level in the human embryonic stem cell.

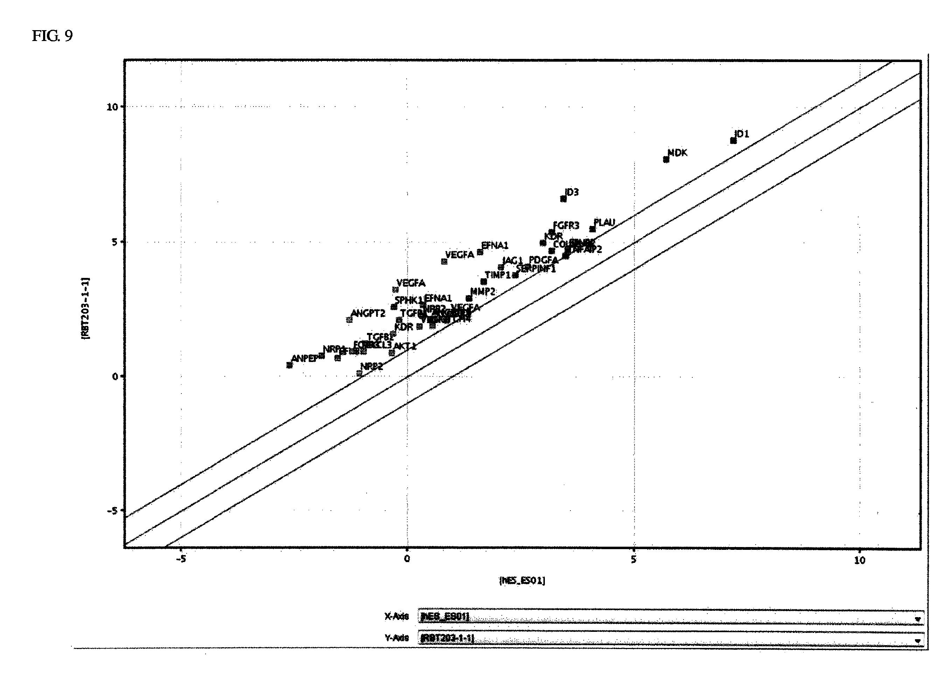

FIG. 9 is a diagram plotting genes related to angiogenesis that were expressed in the human induced malignant stem cell RBT203 (1-1) in amounts more than twice the amounts expressed in the human embryonic stem cell hES_ES01 (GSM194392); themiddle line, the upper line, and the lower line respectively indicate that the level of genetic expression was comparable to, twice, and one half the level in the human embryonic stem cell.

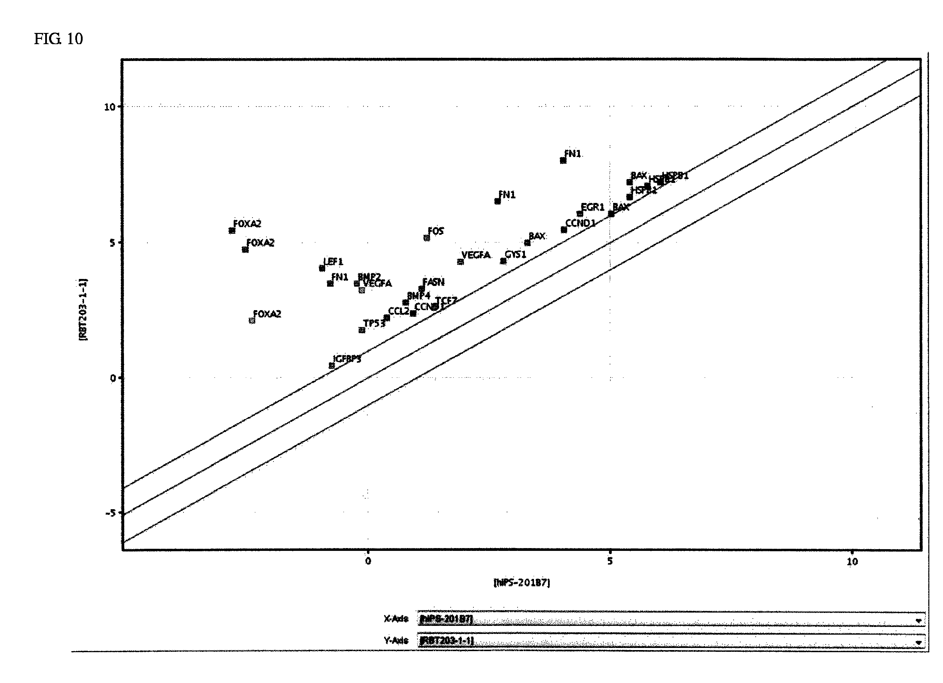

FIG. 10 is a diagram plotting genes related to signal transduction that were expressed in the human induced malignant stem cell RBT203 (1-1) in amounts more than twice the amounts expressed in the human induced pluripotent stem cell hiPS-201B7; the middle line, the upper line, and the lower line respectively indicate that the level of gene expression was comparable to, twice, and one half the level in the human induced pluripotent stem cell.

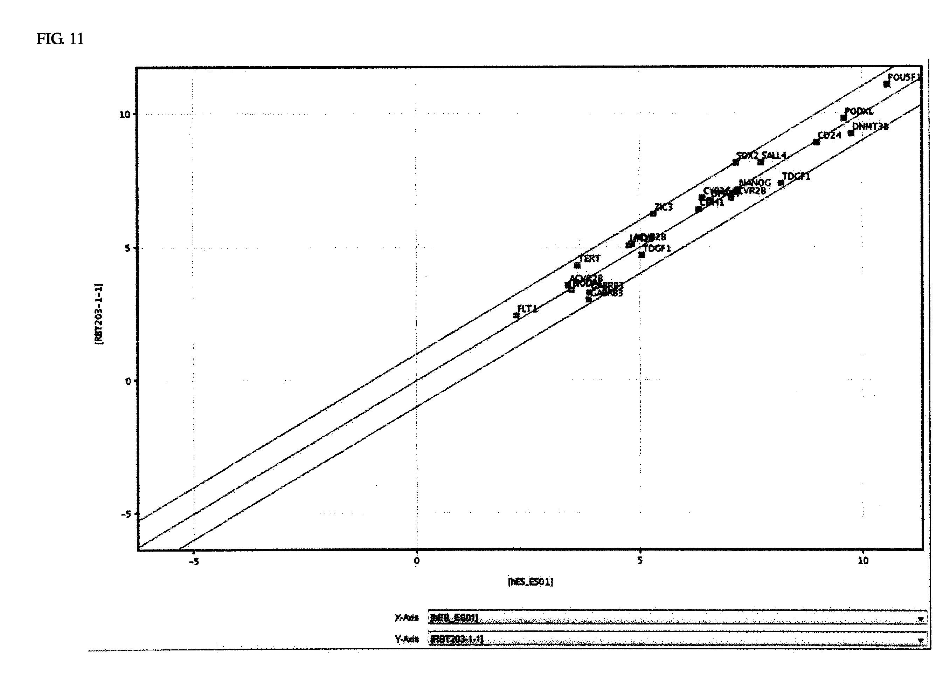

FIG. 11 is a diagram plotting self-renewal related genes that were expressed in the human induced malignant stem cell RBT203 (1-1) in amounts almost comparable to (one half to twice) the amounts expressed in the human embryonict stem cell hES_ES01 (GSM194392); themiddle line, the upper line, and the lower line respectively indicate that the level of gene expression was comparable to, twice, and one half the level in the human embryonic stem cell.

### DESCRIPTION OF EMBODIMENTS

As mentioned earlier, a currently established concept in the art is that just like somatic cells which are reprogrammed to induced pluripotent stem cells, cancer cells can be reverted to normal cells through reprogramming

The present inventors challenged this concept by thinking in the following way: a fresh cancer tissue and a primary cultured cancer cell population are generally both heterogeneous, so a cancer tissue or cancer cell population is likely to include normal cells and non-cancer cells that are identical or approximate in genetics and epigenetics to the normal cells; based on this observation, the present inventors theorized that cancer cells would not be reprogrammed to normal cells but that the non-cancer cells and normal cells contained in the fresh cancer tissue and the primary cultured cancer cell population would be induced to normal induced pluripotent stem cells whereas from the cancer cells that are present in the fresh cancer tissue and the primary cultured cancer cell population and which have mutations in tumor suppressor genes, abnormal gene expression and other aberations, there would be induced cancer stem cells having the mutations in tumor suppressor genes, abnormal gene expression and other aberrations that are derived from said cancer cells.

If this hypothesis is correct, induced cancer stem cells capable of self-renewal *in vitro* can be prepared by making use of techniques for making induced pluripotent stem cells where *POUF5F1, SOX2, KLF4,* and *c-MYC* are transduced or *POU5F1, SOX2,* and *KLF4 are transduced,* and furthermore, by self-renewing the resulting induced cancer stem cells *in vitro,* the induced cancer stem cells capable of self-renewal *in vitro* that maintain the genetic or epigenetic malignancy as cancer could be caused to proliferate without limit under culture conditions.

On the basis of this hypothesis, the present inventors made an intensive study and found that by using as starters both a somatic cell isolated from a mammal having mutations in endogenous tumor suppressor genes and a non-embryonic cell isolated from a carcinogenic mammal and then by causing the genetic products of *POU5F1, KLF4,* and *SOX2* to be present in said starter somatic cell, there could be obtained an induced cancer stem cell capable of self-renewal *in vitro.*

It was also found that when the starter somatic cell was to be placed in the above-described state, the intracellular relative abundances of the genetic products of *POU5F1, KLF4,* and *SOX2* is considered to be one of the important factors that would determine the ultimate course of differentiation.

The present inventors further discovered that by changing the intracellular relative abundances of *POU5F1, KLF4, SOX2,* and like genes, induced mesendodermal stem cells or induced endodermal stem cells could be prepared. More specifically, the present inventors discovered that by changing the intracellular relative abundances of the translation products of *SOX2, POU5F1,* and *KLF4,* the induced cancer stem cells of the present invention, as exemplified by induced mesendodermal precancer stem cells or induced mesendodermal malignant stem cells, induced endodermal precancer stem cells or induced endodermal malignant stem cells, and induced precancerous pluripotent stem cells or induced malignant pluripotent stem cells could be prepared.

What is more, the thus obtained induced cancer stem cells of the present invention can be easily induced to cancer cells by disabling the process of self-renewal through induction of differentiation by means of such methods as culturing in media lacking bFGF or in media other than those for embryonic stem cells or transplanting to laboratory animals.

Thus, the present inventors discovered the induced cancer stem cells of the present invention which not only maintain the aberrations inherent in the starter somatic cell (i.e., genetic mutations or increased gene expression, namely, malignancy as cancer *in vivo*) but are also theoreticaly capable of self-renewal without limit to effectively permit extended passage culture; the present inventors also found that these cells could be applied to drug discovery *in vitro* or used in cancer research. The present invention has been accomplished on the basis of these findings.

As used hereinafter, the "tumor suppressor gene" is a gene that encodes a protein capable of suppressing carcinogenesis and means a gene that has undergone a mutation in the induced cancer stem cells of the present invention. The "cancer-related gene" as used in the present invention is a gene that causes canceration of a cell on account of the aberration of increased gene expression and which relates to canceration of the cell; it means a gene that has undergone increased gene expression in the induced cancer stem cells of the present invention.

On the pages that follow, the induced cancer stem cells of the present invention, the process for producing them, and the applications of these cells are described in detail.

### Induced cancer stem cells

In its first embodiment, the present invention provides an induced cancer stem cell which has the following two characteristics:
(1) expressing the six genes (self-renewal related genes) *POU5F1, NANOG, SOX2, ZFP42, LIN28,* and *TERT*; and
(2) having an aberration which is either (a) a mutation in an endogenous tumor suppressor gene or (b) increased expression of an endogenous cancer-related gene.
It has become clear that the cell having these characteristics is an induced cancer stem cell that is capable of self-renewal *in vitro.*

In the present invention, the term "endogenous" as appearing in the list of the words (1) self-renewal related genes, and (2) (a) an endogenous tumor suppressor gene or (b) an endogenous cancer-related gene, and the like means that these genes are not exogeneous (i.e., having been introduced into the cell as by genetic transduction) but inherent in the cell.

The term "stem cells" as generally used in the technical field contemplated by the present invention refers to cells having both the ability to differentiate into a specific cell (i.e., differentiating ability) and the ability to maintain the same property (differentiating ability) as the original cell even after cell divisions (i.e., self-renwal ability). The term "self-renewal ability" specifically refers to the ability to create the same cell after division, and in the case of the cell of the present invention which has both properties (1) and (2), it means that it can be cultured in an expansion culture condition or in passage culture condition for at least 3 days.

The term "induced cancer stem cells" as used in the present invention means a broad concept covering "induced precancer stem cells" and "induced malignant stem cells". In the present invention, "an induced precancer stem cell" is a precancerous cell at a preliminal stage to canceration and this is a somatic cell in which a genetic aberration that might cause a familial tumor is located on one (an allele) of a pair of alleles ; by expressing the six genes (self-renewal related genes) *POU5F1, NANOG, SOX2, ZFP42, LIN28,* and *TERT,* this cell has been induced to have at least the self-renewal ability.

In the present invention, the term "induced malignant stem cells" means a cell that has increased expression of endogenous cancer-related genes and which has been induced to have at least the self-renewal ability by expressing the six genes (self-renewal related genes) *POU5F1, NANOG, SOX2, ZFP42, LIN28,* and *TERT,* or a somatic cell in which a genetic aberration that might cause a familial tumor is located on at least one (an allele) of a pair of alleles and which has been prepared from a cell derived from a cancer tissue in a patient with a familial tumor and has been induced to have at least the self-renewal ability by expressing the six genes (self-renewal related genes) *POU5F1, NANOG, SOX2, ZFP42, LIN28,* and *TERT.*

The induced cancer stem cells of the present inventin include not only induced cancer stem cells showing pluripotency but also mesendodermal or endodermal induced cancer stem cells.

Stem cells that are "mesendodermal" are those stem cells that have the ability to differentiate into cells pertaining to a mesendodermal or endodermal tissue and which express mesendodermal genes; such cells will differentiate into blood vessels, hematocytes, muscle, bone, cartilage, cardiac muscle, skeletal muscle, stomach, lung, pancreas, liver, small intestine, large intestine, etc.

Stem cells tha are "endodermal" are those stem cells that are below the above-mentioned mesendodermal stem cells in the hierarchy of differentiation, which have the ability to differentiate into cells pertaining to an endodermal tissue, and which express endodermal genes; such cells will differentiate into stomach, lung, pancreas, liver, small intestine, large intestine, etc.

### Gene expression (1) in induced cancer stem cells

The genes (self-renewal related genes) as referred to in (1) above according to the present invention are known as marker genes for embryonic stem cells. These genes are categorized in a group of self-renewal related genes, that specify the induced cancer stem cells of the present invention to be cells that have such a nature that theoretically they are self-renewed without limit and can be cultured in passage-culture condition for an extended period while remaining as the induced cancer stem cells capable of effective self-renewal *in vitro.* Specific examples of such genes are listed in the following Table 1.

**[Table 1]**

| GeneSymbol | GenbankAccession |
|---|---|
| ACVR2B | NM_001106 |
| CD24 | L33930 |
| CDH1 | NM_004360 |
| CYP26A1 | NM_057157 |
| DNMT3B | NM_175850 |
| DPPA4 | NM_018189 |
| EDNRB | NM_003991 |
| FLT1 | NM_002019 |
| GABRB3 | NM_000814 |
| GATA6 | NM_005257 |
| GDF3 | NM_020634 |
| GRB7 | NM_005310 |
| LIN28 | NM_024674 |
| NANOG | NM_024865 |
| NODAL | NM_018055 |
| PODXL | NM_005397 |
| POU5F1 | NM_002701 |
| SALL4 | NM_020436 |
| SOX2 | MM_003106 |
| TDGF1 | NM_003212 |
| TERT | NM_198253 |
| ZFP42 | NM_174900 |
| ZIC3 | NM_003413 |

In the present invention, the six genes consisting of *POU5F1, NANOG, SOX2, ZFP42, LIN28,* and *TERT* mentioned in (1) above (self-renewal related genes) as selected from the group of the genes listed in Table 1 must be expressed but other genes in Table 1 may also be expressed. The six genes in (1) above according to the present invention are known to be particularly typical genes which are expressed in embryonic stem cells specifically and in high yield, and the functions of these genes performed in embryonic stem cells have been well investigated to date.

For the purposes of the present inention, it suffices that the self-renewal related genes referred to in (1) above may be expressed and the amounts of expression of these genes may not be particularly limited; however, from the viewpoint of maintaining the state of the induced cancer stem cells capable of effective self-renewal *in vitro* or from the viewpoint of extended passage culture, the self-renewal related genes in (1) above are preferably expressed in the induced cancer stem cells of the present invention in amounts almost comparable to (i.e., one eighth to eight times, more preferably one fourth to four times) the amounts of the genes expressed in embryonic stem cells (i.e., in either one of hES_H9 (GSM194390), hES_BG03 (GSM194391) and hES_ES01 (GSM194392)), with the range from one half to twice being most preferred.

Among the aforementioned essential genes (six genes), *POU5F1, NANOG,* and *SOX2* are preferably expressed in the induced cancer stem cells of the present invention in amounts ranging from one eighth to eight times, more preferably from one fourth to four times, most preferably from one half to twice, the amounts of the genes expressed in embryonic stem cells.

In the present invention, it is preferred that, among the self-renewal related genes in (1) above that are expressed in the induced cancer stem cells of the present invention, at least five genes are expressed in amounts ranging from one half to twice the amounts of the genes expressed in embryonic stem cells, at least 10 genes being expressed in amounts ranging from one fourth to four times, and at least 20 genes being expressed in amounts ranging from one eighth to eight times, relative to the amounts of the genes expressed in embryonic stem cells.

Among those genes, it is particularly preferred that *NANOG, POU5F1, SOX2, TDGF1, DNMT3B, ZFP42, TERT, GDF3, SALL4, GABRB3, and LIN28* are expressed in amounts ranging from one fourth to four times the amounts of the genes expressed in embryonic stem cells, and most preferably, all of *ACVR2B, CD24, CDH1, CYP26A1, DNMT3B, DPPA4, EDNRB, FLT1, GABRB3, GATA6, GDF3, GRB7, LIN28, NANOG, NODAL, PODXL, POU5F1, SALL4, SOX2, TDGF, TERT, ZFP42, ZIC3* are expressed.

### Gene expression (2) in induced cancer stem cells

The induced cancer stem cells of the present invention have (2) (a) a mutation in an endogenous tumor suppressor gene or (b) increased expression of an endogenous cancer-related gene as an aberration. These aberrations possessed by the induced cancer stem cells of the present invention are identical to the aberrations inherent in the starter somatic cell from which the induced cancer stem cells are derived; in other words, the aberrations inherent in the starter cell have been passed on to the induced cancer stem cells of the present invention.

In this connection, the mutation in an endogenous tumor suppressor gene as referred to in (2) (a) may be any type of mutation, as exemplified by a germline mutation associated with one (an allele) of a pair of alleles in the endogenous tumor suppressor gene.

The increased expression of an endogenous cancer-related gene as referred to in (2) (b) is defined as the case where the yield of expression of that gene is at least twice the yield of expression in embryonic stem cells. The increased expression may be at any yield that is not less than twice the yield of expression in embryonic stem cells, and the greater the difference in expression yield, the more preferred it is, as illustrasted by the following order, in which the degree of preference increases toward the right: at least three times < at least four times, at least five times, at least six times, at least seven times, at least eight times, and so on.

The tumor suppressor gene (a) in which a mutation has taken place and the cancer-related gene (b) which has undergone increased expression are not particularly limited as long as they are known, and they may be exemplified by the following genes.

Examples of the tumor suppressor gene (a) in the present invention include *APC* (GenBank Accession Number: NM_000038.3) and *RB1* (RB1, GenBank Accession Number: NM_000321.2).

The induced tumor stem cells of the presnt invention, if they are confirmed to have a mutation in a causative gene for a familial tumor as (a) a mutation in an endogenous cancer suppressor gene, possess a genetic mutation/gene expression aberration that is related to familial tumor, so they are extremely useful in cancer research as for identifying the carcinogenic mechanism of familial tumors or discovering molecular targets.

The aforementioned cancer-related gene (b) in the present invention may be exemplified by genes that are included within such groups of genes as a group of genes related to angiogenesis, a group of cancer-related pathway genes, a group of genes related to stromal barrier (extracellular matrix and adhesion molecule), a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer. With the induced cancer stem cells of the presnt invention, it is preferred that an increased expression of the endogenous cancer-related gene (b) is recognized to have occurred in at least one gene selected from the groups listed above.

These genes are categorized as groups of genes which are confirmed to increase in gene expression in cancer cells, and by analyzing those induced cancer stem cells which involve aberrations such as (b) increased expression of an endogenous cancer-related gene, one may expect that carcinogenic mechanisms can be identified to offer considerable benefits in cancer research and the research on anti-cancer drug discovery.

The aforementioned cancer-related gene (b) can more specifically be exemplified by the genes listed in the following Tables 2 to 26. GenBank accession numbers corresponding to the respective gene symbols are also listed in these Tables but they are by no means intended to limit the present invention.

The group of genes related to angiogenesis may be exemplified by the genes listed in the following Table 2.

**[Table 2]**

| GeneSymbol | GenbankAccession | GeneSymbol | GenbankAccessian |
|---|---|---|---|
| AKT1 | NM_005163 | ITGB3 | S70348 |
| ANGPT1 | NM_001146 | ITGB3 | NM_000212 |
| ANGPT1 | BC029406 | JAG1 | NM_000214 |
| ANGPT2 | NM_001147 | KDR | NM_002253 |
| ANGPTL3 | NM_014495 | LAMA5 | NM_005560 |
| ANGPTL4 | NM_139314 | LECT1 | NM_00701 |
| ANPEP | NM_001150 | LEP | NM_000230 |
| BAI1 | NM_001702 | MDK | NM_001012334 |
| CCL11 | NM_002986 | MMP2 | NM_004530 |
| CCL2 | NM_002982 | MMP9 | MM_004994 |
| CDH5 | NM_001795 | NOTCH4 | NM_004557 |
| COL18A1 | NM_030582 | NRP1 | MM_003873 |
| COL4A3 | NM_000091 | NRP1 | AF280547 |
| CXCL1 | NM_001511 | NRP2 | MM_201264 |
| CXCL1 0 | NM_001565 | NRP2 | NM_201266 |
| CXCL3 | MM_002090 | NRP2 | MM_018534 |
| CXCL5 | NM_002994 | PDGFA | NM_002607 |
| CXCL6 | NM_002993 | PECAM1 | NM_000442 |
| CXCL9 | NM_002416 | PF4 | NM_002619 |
| EFNA1 | NM_004428 | PGF | NM_002632 |
| EFNA3 | NM_004952 | PLAU | NM_002658 |
| EFNB2 | NM_004093 | PLG | NM_000301 |
| EGF | MM_001963 | PLXDC1 | NM_020405 |

| | | | |
|---|---|---|---|
| ENG | NM_000118 | PROK2 | NM_021935 |
| EPHB4 | NM_004444 | PTGS1 | NM_000962 |
| EREG | NM_001432 | SERPINF1 | NM_002615 |
| FGF1 | AF211169 | SPHK1 | NM_021972 |
| FGF1 | NM_000800 | STAB1 | NM_015136 |
| FGF2 | NM_002006 | TEK | NM_000459 |
| FGFR3 | NM_000142 | TGFA | NM_003236 |
| FIGF | NM_004469 | TGFB1 | NM_000660 |
| FLT1 | NM_002019 | TGF2 | NM_003238 |
| SHAND2 | NM_021973 | TGFBR1 | NM_004612 |
| HGF | NM_001010931 | THBS1 | NM_003246 |
| HIF1A | NM_181054 | THBS2 | NM_003247 |
| HPSE | MM_006665 | THBS2 | L12350 |
| ID1 | NM_002165 | TIMP1 | NM_003254 |
| ID3 | NM_002167 | TIMP2 | AK057217 |
| IFNB1 | NM_002176 | TIMP2 | NM_003255 |
| IFNG | NM_000619 | TIMP3 | NM_000362 |
| IGF1 | NM_000618 | TNF | NM_000594 |
| IL1B | MM_000576 | TNFAIP2 | NM_006291 |
| IL6 | NM_000600 | VEGFA | NM_001025366 |
| IL8 | NM_000584 | VEGFA | NM_003376 |
| IL8 | X77737 | VEGFC | NM_005429 |
| ITGAV | NM_002210 | | |

The group of cancer-related pathway genes may be exemplified by the genes listed in the following Table 3.

**[Table 3]**

| GeneSymbol | GenbankAccession | GeneSymbol | GenbankAccession |
|---|---|---|---|
| AKT1 | NM_005163 | ITGB1 | MM_002211 |
| ANGPT1 | NM_001146 | ITCB3 | NM_000211 |
| ANGPT1 | B0029406 | ITGB3 | S70348 |
| ANGPT2 | NM_001147 | ITGB5 | NM_002213 |
| APAF1 | MM_181861 | JUN | NM_002228 |
| ATM | MM_000051 | MAP2K1 | NM_002755 |
| ATM | BC022307 | MCAM | NM_006500 |
| BAD | MM_004322 | MDM2 | MM_002392 |
| BAX | MM_138764 | MDM2 | NM_006879 |
| BAX | NM_138765 | MET | NM_000245 |
| BAX | NM_138763 | MMP1 | NM_002421 |
| BCL2 | M13995 | MMP2 | NM_004530 |
| BCL2 | NM_000633 | MMP9 | NM_004994 |
| SCL2L1 | NM_138578 | MTA1 | NM_004689 |
| BCL2L1 | MM_001191 | MTA2 | NM_004739 |
| BRCA1 | NM_007295 | MTSS1 | NM_014751 |
| CASP8 | NM_033356 | MYC | NM_002467 |
| CASP8 | MM_033358 | MYC | M13930 |
| CCNE1 | NM_001238 | NFKB1 | NM_003998 |
| CDC25A | NM_001789 | NFKBIA | NM_020529 |
| CDK2 | NM_001798 | NME1 | NM_198175 |
| CDK4 | NM_000075 | NME4 | NM_005009 |
| CDKN1A | NM_076467 | PDGFA | NM_002607 |
| CDKM1A | NM_000389 | PDGFB | MM_002608 |

| | | | |
|---|---|---|---|
| CDKN2A | NM_058197 | PIK3R1 | NM_181523 |
| CFLAR | NM_003879 | PLAU | NM_002658 |
| CFLAR | AF009616 | PLAUR | NM_001005377 |
| CHEK2 | NM_001005735 | PNN | NM_002687 |
| COL18A1 | NM_030582 | RAF1 | NM_002880 |
| E2F1 | NM_005225 | RB1 | NM_000321 |
| EPDR1 | NM_017549 | S100A4 | NM_002961 |
| ERB62 | NM_001005862 | SERPINB5 | NM_002639 |
| ETS2 | NM_005239 | SERPINE1 | NM_000602 |
| FAS | NM_000043 | SNCG | NM_003087 |
| FGFR2 | NM_022970 | SYK | NM_003177 |
| FGFR2 | MM_000141 | TEK | NM_000459 |
| FOS | NM_005252 | TERT | NM_198253 |
| GZMA | NM_006144 | TGFB1 | NM_000660 |
| HTATIP2 | AF092095 | TGFBR1 | NM_004612 |
| HTATIP2 | NM_006410 | THBS1 | NM_003246 |
| IFNB1 | NM_002176 | TIMP1 | NM_003254 |
| IGF1 | NM_000618 | TIMP3 | NM_000362 |
| IL8 | NM_000584 | TNF | NM_000594 |
| IL8 | X77737 | TNFRSF10B | NM_003842 |
| ITGA1 | NM_181501 | TNFRSF1A | NM_001065 |
| ITGA2 | NM_002203 | TNFRSF25 | NM_148965 |
| ITGA3 | NM_002204 | TP53 | NM_000546 |
| ITGA4 | NM_000885 | TWST1 | NM_000474 |
| ITGAV | NM_002210 | VEGFA | NM_001025366 |
| ITGB1 | NM_133376 | VEGFA | NM_003376 |
| ITGB1 | AF086249 | | |

The group of genes related to stromal barrier may be exemplified by the genes listed in the following Table 4.

**[Table 4]**

| GeneSymbol | GenbankAccession | GeneSymbol | GenebankAccession |
|---|---|---|---|
| ADAMTS1 | NM_006988 | ITGB4 | NM_000213 |
| ADAMTS13 | NM_139025 | ITGB5 | NM_002213 |
| ADAMTS13 | NM_139027 | KAL1 | NM_000216 |
| ADAMTS8 | NM_007037 | LAMA1 | NM_005559 |
| CD44 | NM_000610 | LAMA1 | AF351616 |
| CDH1 | NM_004360 | LAMA2 | NM_000426 |
| CLEC3B | NM_003270 | LMA3 | NM_000227 |
| CNTN1 | NM_001843 | LAMA3 | NM_198129 |
| COL11A1 | NM_080629 | LAMB1 | NM_002291 |
| GOL12A1 | NM_004370 | LAMB3 | NM_001017402 |
| COL14A1 | NM_021110 | LAMC1 | NM_002293 |
| COL15A1 | NM_001855 | MMP1 | NM_002421 |
| COL16A1 | NM_001856 | MMP10 | NM_002425 |
| COL1A1 | Z74615 | MMP11 | NM_005940 |
| COL4A2 | NM_001846 | MMP12 | MM_002426 |
| COL5A1 | NM_000093 | MMP13 | NM_002427 |
| COL6A1 | NM_001848 | MMP14 | NM_004995 |
| COL6A2 | NM_001849 | MMP15 | NM_002428 |
| COL6A2 | NM_058175 | MMP16 | NM_005941 |
| COL7A1 | NM_000094 | MMP16 | NM_022564 |
| GOL8A1 | NM_001850 | MMP2 | NM_004530 |
| GTGF | NM_001901 | MMP3 | MM_002422 |

| | | | |
|---|---|---|---|
| CTNNA1 | NM_001903 | MMP7 | NM_002423 |
| CTNNB1 | NM_001904 | MMP8 | NM_002424 |
| CTNND1 | NM_001331 | MMP9 | NM_004994 |
| CTNND1 | CR749275 | NCAM1 | NM_001076682 |
| CTNNC2 | NM_001332 | NGAM1 | NM_000615 |
| ECM1 | NM_004425 | PECAM1 | NM_000442 |
| FN1 | NM_212482 | SELE | NM_000450 |
| FN1 | NM_054034 | SELL | NM_000655 |
| HAS1 | NM_001523 | SELP | NM_003005 |
| ICAM1 | NM_000201 | SGCE | NM_003919 |
| ITGA1 | NM_181501 | SPARC | NM_0031181 |
| ITG3A2 | NM_002203 | SPG7 | NM_003119 |
| ITGA3 | NM_1002204 | SPG7 | NM_199367 |
| ITGA4 | NM_000885 | SPP1 | NM_000582 |
| ITGA5 | NM_002205 | TGFB1 | NM_000358 |
| ITGA6 | NM_000210 | THBS1 | NM_003246 |
| ITGA7 | NM_002206 | THBS2 | NM_003247 |
| ITGA8 | NM_003638 | THBS2 | L12350 |
| ITGAL | NM_002209 | THBS3 | NM_007112 |
| ITGAM | NM_006632 | TIMP1 | NM_003254 |
| ITGAV | NM_002210 | TIMP2 | AK057217 |
| ITGB1 | NM_133370 | TIMP2 | NM_003255 |
| ITGB1 | AF086249 | TEMP3 | NM_000362 |
| ITGB1 | NM_002211 | TNC | NM_002160 |
| ITGB2 | NM_000211 | VGAM1 | NM_001078 |
| ITGB3 | NM_000212 | VCAN | NM_000638 |
| ITGB3 | S70348 | VTN | NM_000638 |

The group of genes related to epithelial-mesenchymal transition may be exemplified by the genes listed in the following Table 5.

**[Table 5]**

| GeneSymbol | GenbankAccession | GeneSymbol | GenebankAccession |
|---|---|---|---|
| AHNAK | NM_001620 | MITF | NM_198177 |
| AHNAK | NM_024060 | MMP2 | NM_004530 |
| AKT1 | NM_00563 | MMP3 | NM_002422 |
| BMP1 | NM_001199 | MMP9 | NM_004994 |
| BMP1 | NM_006129 | MSN | NM_002444 |
| BMP1 | NM_006128 | MST1R | NM_002447 |
| BMP7 | NM_001719 | NODAL | NM_018055 |
| CALD1 | NM_0033138 | NOTCH1 | NM_0017617 |
| CALD1 | AK022222 | NUDT13 | NM_015901 |
| CALD1 | AF247820 | OCLN | NM_002538 |
| CAMK2N1 | NM_018584 | PDGFRB | NM_002609 |
| CAMK2N1 | AF116637 | PLEK2 | NM_016445 |
| CAV2 | NM_001233 | PTK2 | NM_153831 |
| CDH1 | NM_004360 | PTP4A1 | NM_003463 |
| CDH2 | NM_001792 | RAC1 | NM_198829 |
| COL1A2 | NM_000089 | RGS2 | NM_002923 |
| COL3A1 | NM_000090 | SERPINE1 | NM_000602 |
| COL5A2 | NM_000393 | SIP1 | NM_003616 |
| CTNNB1 | NM_001904 | SMAD2 | NM_005901 |
| DSC2 | NM_024422 | SMAD2 | NM_001003652 |
| DSP | NM_004415 | SNAI1 | NM_005985 |
| EGFR | NM_005228 | SNAI2 | U97060 |
| ERBB3 | U88357 | SNAI2 | NM_003068 |
| ERBB3 | U88360 | SNAI3 | NM_178310 |
| ERBB3 | NM_001982 | SOX10 | NM_006941 |

| | | | |
|---|---|---|---|
| ESR1 | NM_000125 | SPARC | NM_003118 |
| ESR1 | U68068 | SPP1 | NM_000582 |
| F11R | NM_144503 | STAT3 | NM_213662 |
| FGF8P1 | NM_005130 | STAT3 | BC029783 |
| FN1 | NM_212482 | STEAP1 | NM_012449 |
| FN1 | NM_054034 | TCF3 | NM_003200 |
| FOXC2 | NM_005251 | TCF4 | NM_003199 |
| FZD7 | NM_003507 | TFPI2 | NM_006528 |
| GNG11 | NM_004129 | TGFB1 | NM_000660 |
| GSC | NM_173849 | TGFB2 | NM_003238 |
| GSK3B | NM_002093 | TGFB3 | NM_003239 |
| IGFBP4 | NM_001552 | TIMP1 | NM_003254 |
| IL1RN | NM_173842 | TMEFF1 | NM_003692 |
| IL1RN | BC068441 | TMEM132A | NM_017870 |
| ILK | NM_001014795 | TSPAN13 | NM_014399 |
| ITGA5 | NM_002205 | TMST1 | NM_000474 |
| ITGAV | NM_002210 | VCAN | NM_004385 |
| ITGB1 | NM_133376 | VIM | NM_003380 |
| ITGB1 | AF086249 | VPS13A | NM_033305 |
| ITGB1 | NM_002211 | VPS13A | NM_015186 |
| JAG1 | NM_000214 | WNT11 | NM_004626 |
| KRT14 | NM_000526 | WNT5A | NM_003392 |
| KRT19 | NM_002276 | WNT5B | NM_030775 |
| KRT7 | NM_005556 | ZEB1 | NM_030751 |
| MAP1B | NM_005909 | ZEB2 | NM_014795 |
| MITF | NM_198159 | | |

The group of genes related to stomach cancer may be exemplified by the genes listed in the following Table 6.

**[Table 6]**

| GeneSymbol | GenbankAccession |
|---|---|
| CCND2 | NM_001759 |
| CDH1 | NM_004360 |
| CDH13 | NM_001257 |
| CDKN2A | NM_058197 |
| CHFR | NM_018223 |
| DKK2 | NM_014421 |
| FHIT | NM_002012 |
| KLF4 | NM_004235 |
| LOX | NM_002317 |
| MGMT | NM_002412 |
| MLH1 | NM_000249 |
| NID1 | NM_002508 |

| | |
|---|---|
| OPCML | NM_001012393 |
| PRKCDBP | NM_145040 |
| PTGS2 | NM_000963 |
| RARB | NM_000965 |
| RASSF1 | NM_170713 |
| RB1 | NM_000321 |
| RUNX3 | NM_004350 |
| SFN | NM_006142 |
| SFRP2 | NM_003013 |
| SFRP5 | NM_003015 |
| TIMP3 | NM_000362 |
| TMEFF2 | AB004064 |
| TMEFF2 | NM_016192 |

The group of genes related to autonomous growth may be exemplified by the genes listed in the following Table 7.

**[Table 7]**

| GeneSymbol | GenbankAccession | GeneSymbol | GenbankAccession |
|---|---|---|---|
| AMH | NM_000479 | ICF2 | NM_000612 |
| BDNF | NM_170735 | IL10 | NM_000572 |
| BMP1 | NM_001199 | IL11 | NM_000641 |
| BMP1 | NM_006128 | IL12B | NM_002187 |
| BMP1 | NM_006128 | IL18 | NM_001562 |
| BMP10 | NM_014482 | IL1A | NM_000575 |
| BMP2 | NM_001200 | IL1B | NM_000576 |
| BMP3 | NM_001201 | IL2 | NM_000586 |
| BMP4 | NM_001202 | IL3 | NM_000588 |
| BMP5 | NM_021073 | IL4 | NM_000589 |
| BMP6 | NM_001718 | INHA | NM_002191 |
| BMP7 | NM_001719 | INHBA | NM_002192 |
| BMP8B | NM_001720 | INHBB | NM_002193 |
| CECR1 | NM_017424 | JAG1 | NM_000214 |
| CECR1 | NM_177405 | JAG2 | NM_00226 |
| CLC | NM_001828 | LEFTY1 | NM_020997 |
| CSF1 | NM_172212 | LEFTY2 | NM_003240 |
| CSF1 | NM_00172210 | LIF | NM_002309 |
| CSF2 | NM_000758 | LTBP4 | NM_003573 |
| CSF3 | NM_000759 | MDK | NM_001012334 |
| CSPG5 | NM_006574 | NDP | NM_000266 |
| CXCL1 | NM_001511 | NODAL | NM_018055 |

| | | | |
|---|---|---|---|
| DKK1 | NM_012242 | NRG1 | NM_013959 |
| EREG | NM_001432 | NRG1 | NM_013961 |
| FGF1 | AF211169 | NRG1 | NM_013962 |
| FGF1 | NM_000800 | NRG1 | NM_013957 |
| FGF11 | NM_004112 | NRG2 | NM_004883 |
| FGF13 | NM_004114 | NRG2 | NM_013982 |
| FGF14 | NM_175929 | NRTN | NM_004558 |
| FGF17 | NM_003867 | NTF3 | NM_002527 |
| FGF19 | NM_005117 | OSGIN1 | NM_013370 |
| FGF2 | NM_002006 | PDGFC | NM_016205 |
| FGF22 | NM_020637 | PGF | NM_002632 |
| FGF23 | NM_020638 | PSPN | NM_004158 |
| FGF5 | NM_004464 | PTN | NM_002825 |
| FGF6 | NM_020996 | SLCO1A2 | NM_005075 |
| FGP7 | NM_002009 | SLCO1A2 | NM_134431 |
| FGF9 | NM_0002010 | SPP1 | NM_000582 |
| FIGF | NM_004469 | TDGF1 | NM_003212 |
| GDF10 | NM_004962 | TGFB1 | NM_000660 |
| GDF11 | NM_005811 | THPO | NM_000460 |
| GDNF | NM_000514 | TNNT1 | BC107798 |
| GP1 | NM_000175 | VEGFA | NM_001025366 |
| HBEGF | NM_001945 | VEGFA | NM_003376 |
| IGF1 | NM_000618 | VEGFC | NM_005429 |
| IGF2 | NM_001007139 | | |

The group of genes related to TGF β/BMP signaling may be exemplified by the genes listed in the following Table 8.

**[Table 8]**

| GeneSymbol | GenebankAccession | GeneSymbol | GenbankAccession |
|---|---|---|---|
| ACVR1 | NM_001105 | IGFBP3 | NM_001013398 |
| ACVR2A | NM_001616 | IL6 | NM_000600 |
| ACVRL1 | NM_000020 | INHA | NM_002191 |
| AMH | NM_000479 | INHBA | NM_002192 |
| AMHR2 | NM_020547 | INHBB | NM_002193 |
| BAMBI | NM_012342 | ITGB5 | NM_002213 |
| BGLAP | NM_199173 | ITGB7 | NM_00889 |
| BMP1 | NM_001199 | JUN | NM_002228 |
| BMP1 | NM_006129 | JUNB | NM_002229 |
| BMP1 | NM_006128 | LEFTY1 | NM_020997 |
| BMP2 | NM_001200 | LTBP1 | NM_206943 |
| BMP3 | NM_001201 | LTBP2 | NM_000428 |
| BMP4 | NM_001202 | LTBP4 | NM_003573 |
| BMP5 | NM_021073 | MYC | NM_002467 |
| BMP6 | NM_001718 | MYC | M13930 |
| BMP7 | NM_001719 | NBL1 | NM_182744 |
| BMPER | NM_133468 | NODAL | NM_018055 |
| BMPR1A | NM_004329 | NOG | NM_005450 |
| BMRR1B | NM_001203 | NR0B1 | NM_000475 |
| BMPR2 | NM_001204 | PDGFB | NM_002608 |
| CDC25A | NM_001789 | PLAU | NM_002658 |

| | | | |
|---|---|---|---|
| CDKN1A | NM_078467 | RUNX1 | NM_001001890 |
| CDKN1A | NM_000389 | RUNX1 | X90978 |
| CDKN2B | NM_004936 | SERPINE1 | NM_000602 |
| CDKN2B | NM_078487 | SMAD1 | NM_005900 |
| CER1 | NM_005454 | SMAD2 | NM_005901 |
| CHRD | NM_003741 | SMAD2 | NM_001003652 |
| COL1A1 | Z74615 | SMAD3 | NM_005902 |
| COL1A2 | NM_000089 | SMAD3 | U68019 |
| COL3A1 | NM_000090 | SMAD4 | NM_005359 |
| CST3 | NM_000099 | SMAD5 | NM_001001419 |
| DLX2 | NM_004405 | SMURF1 | NM_020429 |
| ENG | NM_000118 | SOX4 | NM_003107 |
| EVI1 | NM_005241 | STAT1 | NM_139266 |
| EVI1 | BX640908 | STAT1 | NM_007315 |
| FKBP1B | NM_054033 | TGFB1 | NM_000660 |
| FOS | NM_005252 | TGFB111 | NM_0015927 |
| FST | NM_013409 | TGFB2 | NM_003239 |
| GDF2 | NM_016204 | TGFB3 | NM_003239 |
| GDF3 | NM_020634 | TGFB1 | NM_000358 |
| GDF5 | NM_000557 | TGFBR1 | NM_004612 |
| GDF6 | NM_001001557 | TGFBR2 | NM_003242 |
| GSC | NM_173849 | TGFBR2 | NM_001024847 |
| HIPK2 | NM_022740 | TGFBR3 | NM_003244 |
| ID1 | NM_002165 | TGFBRAP1 | NM_004257 |
| ID2 | NM_002166 | TGIF1 | NM_170695 |
| IGF1 | NM_000618 | TSC22D1 | NM_183422 |

The group of genes related to tissue invasion/metastasis may be exemplified by the genes listed in the following Table 9.

**[Table 9]**

| GeneSymbol | GenbankAccession | GeneSymbol | GenbankAccession |
|---|---|---|---|
| APC | NM_000038 | MDM2 | NM_006879 |
| BRMS1 | NM_015399 | MET | NM_000245 |
| CCL7 | NM_006273 | METAP2 | NM_006838 |
| CD44 | NM_000610 | MGAT5 | NM_002410 |
| CD82 | NM_002231 | MMP10 | NM_002425 |
| CDH1 | NM_004360 | MMP11 | NM_005940 |
| CDH11 | NM_001797 | MMP13 | NM_002427 |
| CDH6 | NM_004932 | MMP2 | NM_004530 |
| CDKN2A | NM_058197 | MMP3 | NM_002422 |
| CHD4 | NM_001273 | MMP7 | NM_002423 |
| COL4A2 | NM_001846 | MMP9 | NM_004994 |
| CST7 | NM_003650 | MTA1 | NM_004689 |
| CTBP1 | AL137853 | MTSS1 | NM_014751 |
| CTBP1 | NM_001012614 | MYC | NM_002467 |
| CTNNA1 | NM_001903 | MYC | M13930 |
| CTSK | NM_000396 | MYCL1 | NM_005376 |
| CTSL1 | NM_001912 | NF2 | NM_181831 |
| CXCL12 | NM_199168 | NF2 | NM_181832 |
| CXCL12 | AK090482 | NME1 | NM_198175 |
| CXCL12 | NM_000609 | NME2 | NM_002512 |
| CXCR4 | NM_001008540 | NME4 | NM_005009 |
| DENR | NM_0003677 | NR4A3 | NM_173198 |
| EPHB2 | NM_004442 | NR4A3 | NM_173198 |
| ETV4 | NM_001986 | PLAUR | NM_001005377 |

| | | | |
|---|---|---|---|
| EWSR1 | BC000527 | PMN | NM_002687 |
| EWSR1 | NM_013986 | PTEN | NM_000314 |
| FGFR4 | NM_213647 | RB1 | NM_000321 |
| FLT4 | NM_182925 | RORB | BX647070 |
| FLT4 | NM_002020 | RORB | NM_006914 |
| FN1 | NM_212482 | RPSA | BC010054 |
| FN1 | NM_054034 | SET | NM_003011 |
| FXYD5 | NM_144779 | SMAD2 | NM_005901 |
| GNRH1 | NM_000825 | SMAD2 | NM_001003652 |
| HGF | NM_001010931 | SMAD4 | NM_005359 |
| HPSE | NM_006665 | SRC | NM_005417 |
| HRAS | NM_005343 | SSTR2 | NM_001050 |
| HTATIP2 | AF092095 | SYK | NM_003177 |
| HTATIP2 | NM_006410 | TCF20 | NM_005650 |
| IGF1 | NM_000618 | TGFB1 | NM_000660 |
| IL18 | NM_001562 | TIMP2 | AK057217 |
| IL1B | NM_000576 | TIMP2 | AK051217 |
| IL8RB | NM_001557 | TIMP3 | NM_000362 |
| ITGA7 | NM_002206 | TIMP4 | NM_003256 |
| ITGB3 | NM_000212 | TNFSF10 | NM_003810 |
| ITGB3 | S70348 | TP53 | NM_000546 |
| KISS1 | NM_002256 | TRPM1 | NM_002420 |
| KISS1R | NM_032551 | TSHR | NM_001018036 |
| KRAS | NM_033360 | TSHR | NM_000369 |
| KRAS | BC029545 | VEGFA | NM_001025366 |
| MCAM | NM_006500 | VEGFA | NM_003376 |
| MDM2 | NM_002392 | | |

The group of genes related to Wnt signaling may be exemplified by the genes listed in the following Table 10.

**[Table 10]**

| GeneSymbol | GenbankAccession | GeneSymbol | GenbankAccession |
|---|---|---|---|
| AES | NM_198969 | JUN | NM_002228 |
| AES | NM_198970 | KREMEN1 | NM_153379 |
| APC | NM_000038 | KREMEN1 | NM_001039570 |
| AXIN1 | NM_003502 | LEF1 | NM_016269 |
| BCL9 | MM_004326 | LRP5 | NM_002335 |
| BTRC | NM_033637 | LRP6 | NM_002336 |
| CCND1 | NM_053056 | MYC | NM_002467 |
| CCND2 | NM_001759 | MYC | M13930 |
| CCND3 | NM_001760 | NKD1 | NM_033119 |
| CSNK1A1 | AF447582 | NLK | NM_016231 |
| CSNK1A1 | NM_001025105 | PITX2 | NM_153426 |
| CSNK1A1 | NM_001892 | PORCN | MM_203473 |
| CSNK1D | AB209463 | PPP2CA | NM_002715 |
| CSNK1D | NM_001893 | PPP2R1A | NM_014225 |
| CSNK1G1 | NM_022048 | PYGO1 | AL049925 |
| CSNK2A1 | NM_177559 | PYGO1 | NM_015617 |
| CTBP1 | AL137653 | RHOU | NM_021205 |
| CTBP1 | NM_001012614 | SENP2 | AF151697 |
| CTBP2 | NM_022802 | SENP2 | NM_021627 |
| CTBP2 | NM_001329 | SFRP1 | NM_003012 |
| CTNNB1 | NM_001904 | SFRP4 | NM_003014 |
| CTNNBIP1 | NM_020248 | SLC9A3R1 | NM_004252 |
| CXXC4 | NM_025212 | SOX17 | NM_022454 |

| | | | |
|---|---|---|---|
| DAAM1 | NM_014992 | T | NM_003181 |
| DIXDC1 | NM_033425 | TCF7 | NM_003202 |
| DKK1 | NM_012242 | TCF7L1 | NM_031283 |
| DVL1 | NM_181870 | TLE1 | NM_005077 |
| DVL2 | NM_004422 | TLE2 | NM_003260 |
| EP300 | NM_001429 | WIF1 | MM_007191 |
| FBXW11 | NM_012300 | WISP1 | NM_080838 |
| FBXW2 | NM_012164 | WISP1 | NM_003882 |
| FBXW4 | NM_022039_{'} | WNT1 | NM_005430 |
| FGF4 | NM_002007 | WNT10A | NM_025216 |
| FOSL1 | NM_005438 | WNT11 | NM_004626 |
| FOXN1 | NM_003593 | WNT16 | NM_057168 |
| FRAT1 | NM_005479 | WNT2 | NM_003391 |
| FRZB | NM_001463 | WNT2B | NM_004185 |
| FSHB | NM_000510 | WNT3 | NM_030753 |
| FZD1 | NM_003505 | WNT3A | NM_033131 |
| FZD2 | NM_001466 | WNT4 | NM_030761 |
| FZD3 | NM_017412 | WNT5A | NM_003392 |
| FZD4 | NM_012193 | WNT5B | NM_030775 |
| FZD5 | NM_003468 | WNT6 | NM_006522 |
| FZD6 | NM_003506 | WNT7A | NM_004625 |
| FZD7 | NM_003507 | WNT7B | NM_058238 |
| FZD8 | NM_031866 | WNT8A | NM_058244 |
| GSK3A | NM_019884 | WNT9A | NM_003395 |
| GSK3B | NM_002093 | | |

The group of genes related to signal transduction may be exemplified by the genes listed in the following Table 11.

**[Table 11]**

| GeneSymbol | GenbankAccession | GeneSymbol | GenbankAccession |
|---|---|---|---|
| ATF2 | AK128731 | IKBKB | NM_001558 |
| ATF2 | NM_001880 | IL1A | NM_000575 |
| BAX | NM_138764 | IL2 | NM_000586 |
| BAX | NM_138765 | IL4 | NM_000589 |
| BAX | NM_138763 | IL4R | NM_000418 |
| BCL2 | M13995 | IL8 | NM_000584 |
| BCL2 | NM_000633 | IL8 | X77737 |
| BCL2A1 | NM_004049 | IRF1 | NM_002198 |
| BCL2L1 | NM_138578 | JUN | NM_002228 |
| BCL2L1 | NM_001191 | KLK2 | NM_005551 |
| BIRC2 | NM_001166 | KLK2 | AF336106 |
| BIRC3 | NM_001165 | KLK2 | NM_001002231 |
| BMP2 | NM_001200 | LEF1 | NM_016289 |
| BMP4 | NM_001202 | LEP | NM_000230 |
| BRCA1 | NM_007295 | LTA | NM_000595 |
| CCL2 | NM_002982 | MDM2 | UM_002392 |
| CGL20 | NM_004591 | MDM2 | NM_006879 |
| CCND1 | MM_053056 | MMP10 | MM_002425 |
| CD5 | NM_014207 | MMP7 | NM_002423 |
| CDK2 | NM_001798 | MYC | NM_002467 |
| CDKN1A | NM_078467 | MYC | M13930 |
| CDKN1A | NM_000389 | NAIP | NM_004536 |
| CDKN1B | NM_004064 | NFKB1 | NM_003998 |

| | | | |
|---|---|---|---|
| CDKN2A | NM_058197 | NRIP1 | NM_003489 |
| CDKN2B | NM_004936 | ODC1 | NM_002539 |
| ODKN2B | NM_078487 | PECAM1 | NM_000442 |
| CEBPB | NM_005194 | PPARG | NM_138711 |
| CXCL9 | NM_000758 | PRKCA | NM_002737 |
| CYP19A1 | NM_002416 | PRKCE | NM_005400 |
| CYP19A1 | NM_031226 | PTCH1 | NM_000264 |
| CYP19A1 | BC035714 | PTGS2 | NM_000963 |
| EGR1 | NM_001964 | RBP1 | NM_002899 |
| EN1 | NM_001426 | SELE | NM_000450 |
| FAS | NM_000043 | SELPLG | NM_003006 |
| FASLG | NM_000639 | TANK | NM_004180 |
| FASN | NM_004104 | TANK | NM_133484 |
| FASN | NM_212482 | TCF7 | NM_003202 |
| FN1 | MM_054034 | TERT | NM_198253 |
| FOS | NM_005252 | TFRC | NM_003234 |
| FOXA2 | NM_021784 | TNF | NM_000594 |
| GADD45A | NM_001924 | TP53 | NM_000546 |
| GREB1 | MM_014668 | TP5313 | NM_00488 |
| GREB1 | NM_148903 | VCAM1 | NM_001078 |
| GYS1 | NM_002103 | VEGFA | NM_001025366 |
| HK2 | MM_000189 | VEGFA | NM_003376 |
| HOXA1 | MM_153620 | WISP1 | NM_080838 |
| HSF1 | NM_153620 | WISP1 | NM_003882 |
| HSPB1 | NM 001540 | WNT1 | NM_005430 |
| ICAM1 | NM_000201 | WNT2 | NM_003391 |
| IGFBP3 | NM_001013398 | | |

The group of genes related to Notch signaling may be exemplified by the genes listed in the following Table 12.

**[Table 12]**

| GeneSymbol | GenbankAccession | GeneSymbol | GenbankAccession |
|---|---|---|---|
| ADAM10 | NM_001110 | LRP5 | MM_002335 |
| ADAM17 | MM_003183 | MAP2K7 | BC005365 |
| AES | NM_198969 | MAP2K7 | NM_145185 |
| AES | NM_198970 | MFNG | NM_002405 |
| AXIN1 | MM_003502 | MMP7 | NM_002423 |
| CBL | NM_005188 | MYCL1 | NM_005376 |
| CCND1 | NM_053056 | NCOR2 | NM_006312 |
| CCNE1 | NM_001238 | NEURL | NM_004210 |
| CD44 | NM_000610 | NFKB1 | NM_003998 |
| CDC16 | NM_003903 | NFKB2 | NM_002502 |
| ODKN1A | NM_078467 | NOTCH1 | NM_017617 |
| CDKN1A | NM_000389 | NOTCH2 | MM_024408 |
| CFLAR | AF009616 | NTCH2NL | NM_303458 |
| CFLAR | NM_001278 | NOTCH2NL | AK075065 |
| CHUK | MM_001278 | NOTCH2NL | AK022008 |
| CTNNB1 | NM_001904 | NOTCH3 | NM_000435 |
| DLL1 | NM_005618 | NOTCH4 | HM_004557 |
| DTX1 | NM_004418 | NR4A2 | NM_006186 |
| EP300 | MM_001429 | NUMB | NM_001005743 |
| ERBB2 | NM_001005862 | PAX5 | U62539 |
| FIGF | NM_004469 | PAX5 | NM_016734 |
| FGS | NM_005252 | PDPK1 | NM_002613 |
| FOSL1 | NM_005438 | POFUT1 | MM_172236 |

| | | | |
|---|---|---|---|
| FZD1 | NM_003505 | POFUT1 | NM_015352 |
| FZD2 | NM_001468 | PPARG | NM_138711 |
| FZD3 | NM_017412 | PSEN1 | NM_000021 |
| FZD4 | NM_012193 | PSEN1 | AJ008005 |
| FZD6 | NM_003506 | PSEN2 | NM_000447 |
| FZD7 | NM_003507 | PSEN2 | NM_0012486 |
| GBP2 | NM_004120 | PSENEN | NM_172341 |
| GLI1 | NM_005269 | PTCRA | NM_138296 |
| GSK3B | NM_002093 | RFNG | NM_002917 |
| HDAC1 | NM_004964 | RUNX1 | NM_001001890 |
| HES1 | NM_005524 | RUNX1 | X90978 |
| HEY1 | NM_012258 | SEL1L | NM_005065 |
| HEYL | NM_014571 | SH2D1A | NM_002351 |
| HOXB4 | NM_024015 | SHH | NM_000193 |
| HR | NM_005144 | SMO | NM_005631 |
| IFNG | NM_000619 | SNW1 | NM_012245 |
| IL17B | MM_014443 | STAT6 | NM_003153 |
| IL2RA | NM_000417 | STIL | HM_003035 |
| JAG1 | NM_000214 | SUFU | MM_016169 |
| JAG2 | NM_002226 | TEAD1 | NM_021961 |
| KRT1 | NM_006121 | TLE1 | NM_005077 |
| LFNG | NM_001040167 | WISP1 | NM_080838 |
| LFNG | NM_001040168 | WISP1 | MM_003882 |
| LMO2 | NM_005574 | WNT11 | NM_004626 |
| LOR | NM_000427 | ZIC2 | NM_007129 |

The group of genes related to breast cancer and estrogen receptor signaling may be exemplified by the genes listed in the following Table 13.

**[Table 13]**

| GeneSymbol | GenbankAccession | GeneSymbol | GenbankAccession |
|---|---|---|---|
| AR | NM_000044 | IL6 | NM_000600 |
| BAD | NM_004322 | IL6R | NM_000565 |
| BAG1 | NM_004323 | IL6ST | NM_002184 |
| BCL2 | M13995 | IL6ST | U58146 |
| BCL2 | NM_000633 | ITGA6 | NM_000210 |
| BCL2L2 | NM_004050 | ITGB4 | NM_000213 |
| C3 | NM_000064 | JUN | NM_002228 |
| CCNA1 | NM_003914 | KIT | NM_000222 |
| CCNA2 | NM_001237 | KLF5 | NM_001730 |
| CCND1 | NM_053056 | KLK5 | NM_012427 |
| CCNE1 | NM_001238 | KRT18 | NM_000224 |
| CD44 | NM_000610 | KRT18 | L32537 |
| CDH1 | NM_004360 | KRT19 | NM_002276 |
| CDKN1A | NM_078467 | MAP2K7 | BC005365 |
| CDKN1A | NM_000389 | MAP2K7 | NM_145185 |
| CDKN1B | NM_004064 | MKI67 | NM_002417 |
| CDKN2A | NM_058197 | MT3 | NM_005954 |
| CLDN7 | NM_001307 | MUC1 | M_002456 |
| CLU | NM_203339 | HFYB | NM_006166 |
| COL6A1 | NM_001848 | NGFR | NM_002507 |
| CTNNB1 | NM_001904 | NME1 | NM_198175 |
| CTSB | NM_147780 | PAPPA | NM_002581 |
| CTSD | NM_001909 | PGR | NM_000926 |
| CYP19A1 | NM_031226^{.} | PLAU | NM_002658 |
| CYP19A1 | BC035714 | PTEN | NM_000314 |

| | | | |
|---|---|---|---|
| DLC1 | NM_024767 | PTGS2 | NM_000963 |
| DLC1 | NM_182643 | RAC2 | NM_002872 |
| EGFR | NM_005228 | RPL27 | MM_000988 |
| ERBB2 | NM_001005862 | SCGB1D2 | NM_006551 |
| ESR1 | U68068 | SGGB2A1 | NM_002407 |
| ESR1 | NM_000125 | SERPINA3 | NM_001085 |
| ESR2 | NM_{_}001437 | SERPINB5 | NM_002639 |
| FAS | NM_000043 | SERPINE1 | NM_000602 |
| FASLG | NM_000639 | SLC7A5 | NM_003486 |
| FGF1 | AF211169 | SPRR1B | NM_003125 |
| FGF1 | NM_000800 | STC2 | NM_003714 |
| FLRT1 | NM_013280 | TFF1 | MM_003225 |
| FOSL1 | NM_005438 | TGFA | MM_003236 |
| GASRP | NM_01421 | THBS1 | NM_003246 |
| GATA3 | NM_001002295 | THBS2 | NM_003247 |
| GNAS | NM_080425 | THBS2 | L12350 |
| GNAS | NM_016592 | TIE1 | NM_005424 |
| GSN | NM_198252 | TNFAIP2 | NM_006291 |
| HMGB1 | NM_002128 | TOP2A | NM_001067 |
| HSPB1 | NM_001540 | TP53 | NM_000546 |
| ID2 | NM_002166 | VEGFA | NM_001025366 |
| IGFBP2 | NM_000597 | VEGFA | NM_003376 |
| IL2RA | NM_000417 | | |

The group of genes related to colon cancer may be exemplified by the genes listed in the following Table 14.

**[Table 14]**

| **GeneSymbol** | **GenbankAccession** |
|---|---|
| APC | NM_000038 |
| CDH1 | NM_004360 |
| CDKN2A | NM_058197 |
| DJKK2 | NM_014421 |
| DKK3 | NM_015881 |
| HIC1 | NM_006497 |
| HIC1 | BY798288 |
| HS3ST2 | NM_006043 |
| IGF2 | NM_001007139 |
| IGF2 | NM_000612 |
| MLH1 | NM_000249 |
| OPCML | NM_001012393 |
| PCDH10 | NM_020815 |
| PCDH10 | NM_032961 |
| RASSF1 | NM_170713 |
| RUNX3 | NM_004350 |
| SFRP1 | MM_003012 |
| SFRP2 | NM_003013 |
| SFRP5 | NM_003015 |
| SPARC | NM_003118 |
| TMEFF2 | AB004064 |
| TMEFF2 | NM_016192 |
| UCHL1 | NM_004181 |
| WIF1 | NM_007191 |
| WT1 | NM_024424 |

The group of genes related to hypoxic signaling may be exemplified by the genes listed in the following Table 15.

**[Table 15]**

| GeneSymbol | GenbankAccession | GeneSymbol | GenbankAccession |
|---|---|---|---|
| ADM | NM_001124 | IL6 | NM_000600 |
| AGPAT2 | NM_006412 | IL6ST | NM_002184 |
| AGTPBP1 | NM_015239 | IL6ST | U58146 |
| AGTPBP1 | AJ437018 | IQGAP1 | MM_003870 |
| ANGPTL4 | NM_139314 | KHSRP | NM_003685 |
| ARD1A | NM_003491 | KIT | NM_000222 |
| ARNT2 | NM_014862 | LCT | NM_002299 |
| BAX | NM_138764 | LEP | NM_000230 |
| BAX | NM_138765 | MAN2B1 | NM_000528 |
| BAX | NM_138763 | MAN2B1 | U60266 |
| BIRC5 | NM_001012271 | MOCS3 | NM_014484 |
| CA1 | NM_001738 | MT3 | NM_005954 |
| CASP1 | NM_033292 | MYBL2 | NM_002466 |
| CAT | NM_001752 | NOTCH1 | NM_017617 |
| CDC42 | NM_044472 | NPY | NM_000905 |
| CDC42 | NM_001791 | NUDT2 | NM_001161 |
| CHGA | NM_001275 | PDIA2 | NM_006849 |
| COL1A1 | Z74615 | PEA15 | NM_003768 |
| CREBBP | NM_004380 | PLAU | NM_002658 |
| CSTB | NM_000100 | PLOD3 | NM_001084 |
| CYGB | NM_134268 | PPARA | NM_0050036 |
| DAPK3 | NM_001348 | PPARA | L02932 |
| DCTN2 | NM_006400 | PPP2CB | NM_01009552 |
| DR1 | NM_001938 | PRKAA1 | NM_206907 |

| | | | |
|---|---|---|---|
| ECE1 | NM_001397 | PRPF40A | B0027178 |
| EEF1A1 | NM_001402 | PSMB3 | NM_002795 |
| ENO1 | NM_001428 | PTX3 | NM_002852 |
| EP300 | NM_001429 | RARA | NM_000964 |
| EPAS1 | NM_001430 | RPL28 | NM_000991 |
| EPO | NM_000799 | RPL32 | NM_001007074 |
| GNA11 | L40630 | RPS2 | NM_002952 |
| GNA11 | NM_002067 | RPS2 | BC020336 |
| GPI | NM_00175 | RPS2 | AB065089 |
| GPX1 | NM_20139 | RPS7 | NM_001011 |
| HBB | NM_000518 | SAE1 | NM_005500 |
| HIF1A | NM_181054 | SLC2A1 | NM_006516 |
| HIF1AN | NM_017902 | SLC2A4 | MM_001042 |
| HIF3A | NM_152794 | SPTBN1 | NM_178313 |
| HIF3A | AK024095 | SPTBN1 | NM_003128 |
| HIF3A | NM_022462 | SSSCA1 | NM_006395 |
| HK2 | NM_000189 | SUMO2 | NM_006937 |
| HMOX1 | NM_002133 | TH | NM_199293 |
| HYOU1 | NM_006389 | TST | NM_003312 |
| IGF2 | NM_001007139 | TUBA4A | NM_006000 |
| IGF2 | NM_000612 | UCP2 | NM_003355 |
| IGFBP1 | NM_000596 | VEGFA | NM_001025360 |
| IL1A | NM_000575 | VEGFA | NM_003376 |

The group of genes related to GPCR signaling may be exemplified by the genes listed in the following Table 16.

**[Table 16]**

| **GeneSymbol** | **GenbankAccession** | **GeneSymbol** | **GenbankAccession** |
|---|---|---|---|
| ADCY5 | MM_183357 | GALR2 | NM_003557 |
| ADORA2A | NM_000675 | GCGR | NM_000160 |
| ADRB1 | NM_000684 | GNAQ | NM_002072 |
| ADRB2 | NM_000024 | GNAS | NM_080425 |
| AGT | NM_000029 | GNAS | NM_016592 |
| AGTR1 | D13814 | GRM1 | NM_000838 |
| AGTR1 | NM_031850 | GRM2 | NM_000839 |
| AGTR2 | NM_000686 | GRM4 | NM_000841 |
| AGTRAP | NM_020350 | GRM5 | NM_000842 |
| AKT1 | NM_005163 | GRM7 | NM_181874 |
| ARRB1 | NM_004041 | GRM7 | NM_181875 |
| ARRB2 | NM_004313 | ICAM1 | NM_000201 |
| BAI1 | NM_001702 | IL1B | NM_000576 |
| BCL2 | M13995 | IL1R1 | NM_000877 |
| BCL2 | NM_000633 | IL1R2 | NM_004633 |
| BCL2L1 | NM_138578 | IL2 | NM_000586 |
| BCL2L1 | NM_001191 | JUN | NM_0012228 |
| CALCR | NM_001742 | JUNB | NM_002229 |
| CALCRL | NM_005795 | LHCGR | NM_000233 |
| CASR | NM_000388 | MAX | NM_197957 |
| CCL2 | NM_002982 | MAX | NM_145113 |
| CCL4 | NM_002984 | MAX | NM_145114 |

| | | | |
|---|---|---|---|
| CCND1 | NM_053056 | MMP9 | NM_004994 |
| CCNE1 | NM_001238 | MYC | NM_002467 |
| CCNE2 | NM_057749 | MYC | M13930 |
| CDKN1A | NM_078467 | OPRD1 | MM_000911 |
| CDKN1A | NM_000389 | OPRK1 | NM_000912 |
| CDKN1B | NM_004064 | PDPK1 | NM_002613 |
| CFLAR | NM_003879 | PIK3CG | NM_002649 |
| CFLAR | AF009616 | PRKCA | NM_002737 |
| COL1A1 | Z74615 | PTGDR | NM_000953 |
| CRHR1 | AK124894 | PTGS2 | NM_000963 |
| CRHR1 | NM_004382 | RGS2 | NM_002923 |
| CRHR2 | NM_001883 | RHO | NM_000539 |
| CTGF | NM_001901 | SCTR | NM_002980 |
| CYP19A1 | NM_031226 | SERPINE1 | NM_000602 |
| CYP19A1 | BC035714 | SOCS1 | NM_003745 |
| DRD1 | NM_000794 | TNF | NM_000594 |
| DRD2 | NM_000795 | TSHR | NM_001018036 |
| DUSP14 | NM_007026 | TSHR | NM_000369 |
| EDN1 | NM_001955 | UCP1 | NM_021833 |
| EGR1 | MM_001964 | VCAM1 | NM_001078 |
| ELK1 | NM_005229 | VEGFA | NM_001025366 |
| ELK4 | NM_001973 | VEGFA | NM_003376 |
| FGF2 | NM_002006 | YWHAZ | NM_145690 |
| FOS | NM_005252 | YWHAZ | NM_145690 |

The group of genes related to drug resistance may be exemplified by the genes listed in the following Table 17.

**[Table 17]**

| **GeneSymbol** | **GenbankAccession** | **GeneSymbol** | **GenbankAccession** |
|---|---|---|---|
| ABCA1 | NM_005502 | SLC19A3 | NM_025243 |
| ABCA1 | AK024328 | SLC22A1 | NM_153187 |
| ABCA12 | NM_173076 | SLC22A2 | NM_003058 |
| ABCA13 | NM_152701 | SLC22A3 | NM_021977 |
| ABCA2 | NM_001606 | SLC22A6 | NM_153277 |
| ABCA3 | NM_001089 | SLC22A7 | NM_153320 |
| ABCA4 | NM_000350 | SLC22A8 | NM_004254 |
| ABCA9 | NM_080283 | SLC22A9 | NM_080866 |
| ABCB1 | NM_000927 | SLC25A13 | NM_014251 |
| ABCB11 | NM_003742 | SLC28A1 | NM_004213 |
| ABCB4 | NM_018850 | SLC28A2 | NM_004212 |
| ABCB5 | NM_178559 | SLC28A3 | NM_022127 |
| ABCB6 | NM_005689 | SLC29A1 | NM_004955 |
| ABCC1 | NM_019862 | SLC29A | MM_001532 |
| ABCC10 | NM_033450 | SLC2A1 | NM_006516 |
| ABCC11 | NM_033151 | SLC2A2 | NM_000340 |
| ABCC12 | NM_033226 | SLC2A3 | NM_006931 |
| ABCC2 | NM_000392 | SLC31A1 | NM_001859 |
| ABCC3 | NM_003786 | SLC38A2 | NM_018976 |
| ABCC4 | NM_005845 | SLC38A5 | NM_033518 |
| ABCC5 | NM_005688 | SLC3A1 | NM_000341 |
| ABCC6 | NM_001079528 | SLC3A2 | NM_002394 |
| ABCC6 | NM_001171 | SLC5A1 | NM_000343 |

| | | | |
|---|---|---|---|
| ABCD1 | NM_000033 | SLC5A4 | NM_014227 |
| ABCD3 | NM_002858 | SLC7A11 | NM_014331 |
| ABCD4 | NM_005050 | SLC7A5 | NM_003486 |
| ABCF1 | NM_001090 | SLC7A6 | NM_003983 |
| ABCG2 | NM_004827 | SLC7A7 | NM_003982 |
| ABCG8 | NM_022437 | SLC7A8 | NM_182728 |
| AQP1 | NM_198098 | SLC7A9 | NM_014270 |
| AQP7 | NM_001170 | SLCO1A2 | NM_005075 |
| AQP9 | NM_020980 | SLCO1A2 | NM_134431 |
| ATP6V0C | NM_001694 | SLCO1B1 | NM_006446 |
| ATP7B | NM_000053 | SLCO1B3 | NM_019844 |
| MVP | NM_017458 | SLCO2A1 | NM_005630 |
| SLC10A1 | NM_003049 | SLCO2B1 | NM_007256 |
| SLC10A2 | NM_000452 | SLCO3A1 | XM_001132480 |
| SLC15A1 | NM_005073 | SLCO3A1 | NM_013272 |
| SLC15A1 | AB001328 | SLCO4A1 | NM_016354 |
| SLC15A2 | NM_021082 | TAP1 | NM_000593 |
| SLC16A1 | NM_003051 | TAP2 | NM_018833 |
| SLC16A2 | NM_006517 | TAP2 | NM_000544 |
| SLC16A3 | NM_004207 | VDAC1 | NM_003374 |
| SLC19A1 | NM_194255 | VDAC2 | NM_003375 |
| SLC19A2 | NM_006996 | | |

The group of genes related to Hedgehog signaling may be exemplified by the genes listed in the following Table 18.

**[Table 18]**

| **GeneSymbol** | **GenbankAccession** | **GeneSymbol** | **GenbankAccession** |
|---|---|---|---|
| BMP2 | NM_001200 | NPC1 | NM_000271 |
| BMP4 | NM_001202 | NPC1L1 | NM_013389 |
| BMP5 | NM_021073 | NUMB | NM_0010005743 |
| BMP6 | NM_001718 | OTX2 | NM_021728 |
| BMP7 | NM_001719 | PRKACA | NM_002730 |
| BMP8A | NM_181809 | PRKACB | NM_207578 |
| BMP8B | NM_001720 | PRKACB | NM_002731 |
| BTRC | NM_0336337 | PRKACG | NM_002732 |
| C18orf8 | NM_013326 | PRKX | NM_0050044 |
| CDON | NM_016952 | PRKY | NM_002760 |
| CEP76 | NM_024899 | PTCH1 | NM_000264 |
| CRIM1 | NM_016441 | PTCH2 | NM_003738 |
| CSNK1A1 | AF447582 | PTCHD1 | NM_173495 |
| CSNK1A1 | NM_001025105 | PTCHD1 | BX107899 |
| CSNK1A1 | NM_001892 | PTCHD2 | AL117235 |
| CSNK1A1L | NM_145203 | RAB23 | NM_016277 |
| CSNK1D | AB209463 | SFRP1 | NM_003012 |
| CSNK1D | NM_001893 | SHH | NM_000193 |
| CSNK1E | NM_152221 | SIAH1 | NM_003031 |
| CSNK1G1 | NM_022048 | SMO | NM_005631 |
| CSNK1G2 | NM_{_}001319 | STK36 | NM_015690 |
| CTNNB1 | NM_001904 | SUFU | NM_016169 |

| | | | |
|---|---|---|---|
| DHH | NM_021044 | WIF1 | NM_007191 |
| ERBB4 | NM_005235 | WNT1 | NM_005430 |
| FBXW11 | NM_012300 | WNT10A | NM_025216 |
| FGF9 | NM_002010 | WNT10B | NM_003394 |
| FGFR3 | NM_000142 | WNT11 | NM_004626 |
| FKBP8 | NM_012181 | WNT16 | NM_057168 |
| FOXE1 | X94553 | WNT2 | NM_003391 |
| FOXE1 | NM_004473 | WNT2B | NM_004185 |
| GAS1 | NM_002048 | WNT3 | NM_030753 |
| GLI1 | MM_005269 | WNT3A | NM_033131 |
| GLI2 | NM_005270 | WNT4 | NM_030761 |
| GLI3 | NM_000168 | WNT5A | NM_003392 |
| GREM1 | NM_013372 | WNT5B | NM_030775 |
| GSK3B | NM_002093 | WNT6 | NM_006522 |
| HHAT | NM_018194 | WNT7A | NM_004625 |
| HHIP | AK074711 | WNT7B | NM_058238 |
| HHIP | NM_022475 | WNT8A | NM_058244 |
| IFT52 | NM_016004 | WNT8B | NM_003393 |
| KCTD11 | NM_001002914 | WNT9A | NM_003395 |
| LRP₂ | NM_004525 | WNT9B | NM_003396 |
| MAPK1 | NM_138957 | ZIC1 | NM_003412 |
| MAPK1 | NM_002745 | ZIC2 | NM_007129 |
| MTSS1 | NM_014751 | | |

The group of genes related to PI3K-AKT signaling may be exemplified by the genes listed in the following Table 19.

**[Table 19]**

| **GeneSymbol** | **GenbankAccession** | **GeneSymbol** | **GenbankAccession** |
|---|---|---|---|
| ADAR | NM_001111 | MAPK14 | NM_139013 |
| AKT1 | NM_005163 | MAPK14 | NM_001315 |
| AKT2 | NM_J01626 | MAPK3 | NM_002746 |
| AKT3 | NM_181690 | MAPK8 | NM_139047 |
| AKT3 | NM_005465 | MTCP1 | NM_014221 |
| APC | NM_000038 | MYD88 | NM_002468 |
| BAD | NM_004322 | NFKB1 | NM_003998 |
| BTK | NM_000061 | NFKBIA | NM_020529 |
| CASP9 | NM_001229 | PABPC1 | NM_002588 |
| CCND1 | NM_053056 | PAK1 | NM_002576 |
| CD14 | NM_000591 | PDGFRA | AA599881 |
| CDC42 | NM_044472 | PDGFRA | BC015188 |
| CDC42 | NM_001791 | PDGFRA | Nm_006206 |
| CDKN1B | NM_004084 | PDK1 | NM_002610 |
| CHUK | NM_001278 | PDK2 | NM_002611 |
| CSNK2A1 | NM_177559 | PDPK1 | NM_002813 |
| CTNNB1 | NM_001904 | PIK3CA | NM_006218 |
| EIF2AK2 | NM_002759 | PIK3CG | NM_002649 |
| EIF4B | NM_001417 | PIK3R1 | NM_181523 |
| EIF4E | NM_001968 | PIK3R2 | NM_005027 |
| EIF4EBP1 | NM_004095 | PRKCA | NM_002737 |
| EIF4G1 | NM_182917 | PRKCZ | NM_002744 |
| ELK1 | NM_005229 | PRKCZ | AB007974 |
| FASLG | NM_000639 | PTEN | NM_000314 |

| | | | |
|---|---|---|---|
| PKBP1A | NM_000801 | PTK2 | NM_153831 |
| FKBP1A | NM_054014 | PTPN11 | NM_002834 |
| FOS | NM_005252 | RAC1 | NM_198829 |
| FOXO1 | NM_002015 | RAF1 | NM_002880 |
| FOXO3 | NM_001455 | RASA1 | NM_002890 |
| FRAP1 | NM_004958 | RBL2 | NM_005611 |
| GJA1 | NM_000185 | RHEB | BC009638 |
| GRB10 | NM_001001555 | RHEB | NM_005614 |
| GRB2 | NM_002086 | RHOA | NM_001664 |
| GSK3B | NM_002093 | RPS6KA1 | NM_002953 |
| HRAS | NM_00O5343 | RPS6KB1 | BC036033 |
| HSPB1 | NM_001540 | RPS6KB1 | NM_003161 |
| IGF1 | NM_000618 | SHC1 | NM_003029 |
| IGF1R | NM_000875 | SHC1 | NM_183001 |
| IGF1R | AF020763 | SOS1 | NM_005633 |
| ILK | NM_001014795 | SRF | NM_003131 |
| IRAK1 | NM_001569 | TCL1A | NM_021986 |
| IRS1 | NM_005544 | TIRAP | NM_148910 |
| ITGB1 | NM_133376 | TIRAP | NM_001039661 |
| ITGB1 | AF086249 | TLR4 | NM_138554 |
| ITGB1 | NM_002211 | TOLLIP | NM_019009 |
| JUN | NM_002228 | TSC1 | NM_000368 |
| MAP2K1 | NM_002755 | TSC2 | NM_000548 |
| MAPK1 | NM_138957 | WASL | NM_003941 |
| MAPK1 | NM_002745 | YWHAH | NM_003405 |

The group of drug metabolism genes may be exemplified by the genes listed in the following Table 20.

**[Table 20]**

| Gene Symbol | GenbankAccession | GeneSymbol | GenbankAccession |
|---|---|---|---|
| ABCB1 | NM_000927 | GCKR | NM_001486 |
| ABCC1 | NM_019862 | GGT1 | NM_005265 |
| ABP1 | NM_001091 | GGT1 | NM_013430 |
| ADH1C | NM_000669 | GPI | NM_000175 |
| ADH4 | NM_000670 | GPX1 | NM_201397 |
| ADH5 | NM_000671 | GPX2 | NM_002083 |
| ADH6 | BC039065 | GPX3 | NM_002084 |
| ADH6 | NM_000672 | GBX4 | NM_002085 |
| AHR | NM_001621 | GPX5 | NM_001509 |
| ALAD | NM_001003945 | GSR | BC035691 |
| ALDH1A1 | NM_000689 | GSR | NM_000637 |
| ALOX12 | NM_000697 | GSTA3 | NM_000847 |
| ALOX15 | M95923 | GSTA4 | NM_001512 |
| ALOX15 | NM_001140 | GSTM2 | NM_000848 |
| ALOX5 | NM_000698 | GSTM3 | NM_000849 |
| APOE | NM_00041 | GSTM5 | NM_000851 |
| ARNT | NM_001668 | GSTP1 | NM_000852 |
| ASNA1 | NM_004317 | GSTT1 | NM_000853 |
| BLVRA | NM_000712 | GSTZ1 | NM_145870 |
| BLVRB | NM_000713 | HK2 | NM_000189 |
| CES2 | NM_198061 | HSD17B1 | BC033110 |
| CES2 | MNM_003869 | HSD17B1 | NM_000413 |
| CES4 | AF106005 | HSD17B2 | NM_002153 |
| CHST1 | NM_003654 | HSD17B3 | NM_000197 |

| | | | |
|---|---|---|---|
| COMT | NM_000754 | LPO | NM_006151 |
| CYB5R3 | NM_000398 | MARCKS | NM_002356 |
| CYB5R3 | NM_007326 | MGST1 | NM_145791 |
| CYP11B2 | NM_000498 | MGST2 | NM_002413 |
| CYP17A1 | NM_000102 | MGSI3 | NM_004528 |
| CYP19A1 | NM_031226 | MPO | NM_000250 |
| CYP19A1 | BC035714 | MT2A | NM_005953 |
| CYP1A1 | NM_000499 | MT3 | NM_005954 |
| CYP2B6 | NM_000767 | MTHFR | NM_005957 |
| CYP2C19 | NM_000769 | NAT1 | NM_000662 |
| CYP2C8 | NM_000770 | NAT2 | NM_000015 |
| CYP2C9 | NM_000171 | NOS3 | NM_000603 |
| CYP2D6 | NM_000106 | NQO1 | NM_000903 |
| CYP2E1 | NM_000773 | PKLR | NM_000298 |
| CYP2F1 | NM_000774 | PKM2 | NM_182470 |
| CYP2J2 | NM_000775 | PON1 | NM_000446 |
| CYP3A5 | NM_000777 | PON2 | NM_000305 |
| CYP3A5 | AF355801 | PON3 | NM_000940 |
| EPHX1 | NM_000120 | SMARCAL1 | NM_014140 |
| FAAH | NM_001441 | SNN | NM_003498 |
| FBP1 | NM_000507 | SRD5A1 | NM_001047 |
| GAD1 | NM_000817 | SHD5A2 | NM_000348 |
| GAD1 | NM_013445 | | |

The group of genes related to molecular mechanism of cancer may be exemplified by the genes listed in the following Table 21.

**[Table 21]**

| GeneSymbol | GenbankAccession | GeneSymbol | GenbankAccession | GeneSymbol | GenbankAccession |
|---|---|---|---|---|---|
| ABL1 | NM_005157 | DVL1 | NM_181870 | MAPK14 | NM_001315 |
| ABL1 | NM_007313 | E2F1 | NM_005225 | MAPK3 | NM_002746 |
| AKT1 | NM_005163 | EGFR | NM_005228 | MAPK8 | NM_139047 |
| AKT2 | NM_001626 | ELK1 | NM_005229 | MAX | NM_197957 |
| APC | NM_000038 | ERBB2 | NM_001005862 | MAX | NM_145113 |
| BAX | NM_138764 | FADD | NM_003824 | MAX | NM_145114 |
| BAX | NM_138765 | FAS | NM_000043 | MDM2 | NM_002392 |
| BAX | NM_138763 | FASLG | NM_000639 | MDM2 | NM_006879 |
| BCAR1 | NM_014567 | FGF2 | NM_002006 | MYC | NM_002467 |
| BCL2 | M13995 | FN1 | NM_212482 | MYC | M13930 |
| BCL2 | NM_000633 | FN1 | NM_054034 | NFKB1 | NM_003998 |
| BCL2L1 | NM_138578 | FOS | NM_005252 | NFKB2 | NM_002502 |
| BCL2L1 | NM_001191 | FYN | NM_002037 | NFKBIA | NM_020529 |
| BCL2L11 | NM_138621 | FZD1 | NM_003505 | NRAS | NM_002524 |
| BCL2L11 | AB071195 | GRB2 | NM_002086 | PIK3CA | NM_006218 |
| BIB | NM_197966 | GSK3B | NM_002093 | PIK3R1 | NM_181523 |
| BRAF | NM_004333 | HGF | NM_001010931 | PTEN | NM_000314 |
| GASP8 | NM_033356 | HRAS | NM_005343 | PTK2 | NM_0153831 |
| CASP8 | NM_033358 | IGF1 | NM_000618 | PTK2B | NM_173174 |
| CASP9 | NM_001229 | IGF1R | NM_000875 | RAC1 | NM_198829 |

| | | | | | |
|---|---|---|---|---|---|
| CCND1 | NM_053056 | IGF1R | AF020763 | RAF1 | NM_002880 |
| CCND2 | NM_001759 | ITGA2B | NM_000419 | RB1 | NM_000321 |
| CCND3 | NM_001760 | ITGAV | NM_002210 | RELA | BC014095 |
| CCNE1 | NM_001238 | ITGB1 | NM_133376 | RHOA | NM_001664 |
| CDC42 | NM_044472 | ITGB1 | AF086249 | SHC1 | NM_003029 |
| CDC42 | NM_001791 | ITGB1 | NM_002211 | SHC1 | NM_183001 |
| CDH1 | NM_004360 | ITGB3 | NM_000212 | SMAD4 | NM_005359 |
| CDK2 | NM_001798 | ITGB3 | S70348 | SOS1 | NM_005633 |
| CDK4 | NM_000075 | JUN | NM_002228 | SPP1 | NM_000582 |
| CDKN1A | NM_078467 | KDR | NM_002253 | SRC | NM_005417 |
| CDKN1A | NM_000389 | KIT | NM_000222 | TCF3 | NM_003200 |
| CDKN1B | NM_004064 | KRAS | NM_033360 | TGFB1 | NM_000660 |
| CDKN2A | NM_058197 | KRAS | BC029545 | TGFBR1 | NM_004612 |
| CDKN2B | NM_004936 | LEF1 | NM_016269 | TGFBR2 | NM_003242 |
| CDKN2B | NM_078487 | MAP2K1 | NM_002755 | TGFBR2 | NM_001024847 |
| COL1A1 | Z74615 | MAP3K5 | NM_005923 | TP53 | NM_000546 |
| CRK | NM_016823 | MAPK1 | NM_138957 | VEGFA | NM_001025366 |
| CTNNB1 | NM_001904 | MAPK1 | NM_002745 | VEGFA | NM_003376 |
| CYCS | NM_018947 | MAPK14 | NM_139013 | WNT1 | NM_005430 |

The group of genes related to SMAD signaling network may be exemplified by the genes listed in the following Table 22.

**[Table 22]**

| GeneSymbol | GenbankAccession | GeneSymbol | GenbankAccession | GeneSymbol | GenbankAccession |
|---|---|---|---|---|---|
| ACTA1 | NM_001100 | HDAC4 | NM_006037 | SIN3B | BC063531 |
| ACTA2 | NM_001613 | HDAC5 | NM_001015053 | SKI | NM_003036 |
| ACTB | NM_001101 | HDAC6 | BC011498 | SKIL | NM_005414 |
| ACTG1 | NM_001614 | HDAC6 | NM_006044 | SMAD2 | NM_005901 |
| ACTG2 | NM_001615 | HDAC8 | NM_018486 | SMAD2 | NM_001003652 |
| AXIN1 | NM_003502 | HDAC9 | NM_178423 | SMAD3 | NM_005902 |
| BMP1 | NM_001199 | HDAC9 | NM_058177 | SMAD3 | U68019 |
| BMP1 | NM_006129 | HDAC9 | NM_014707 | SMAD4 | NM_005359 |
| BMP1 | NM_006128 | HDAG9 | NM_058176 | SMAD7 | NM_005904 |
| BMP10 | NM_014482 | HECW1 | NM_015052 | SMURF1 | NM_020429 |
| BMP15 | NM_005448 | ITCH | NM_031483 | SMURF2 | NM_0227739 |
| BMP2 | NM_001200 | KPNB1 | NM_002265 | SMURF2 | AK002019 |
| BMP3 | NM_001201 | LEFTY2 | NM_003240 | SNX6 | NM_021249 |
| BMP4 | NM_001202 | PSMA2 | NM_002787 | STUB1 | NM_005861 |
| BMP5 | NM_021073 | PSMA3 | NM_002788 | TGFB1 | NM_000660 |
| BMP6 | NM_001718 | PSM4 | NM_002789 | TGFB2 | NM_003238 |
| BMP7 | NM_001719 | PSMA6 | NM_002791 | TGFB3 | NM_003239 |

| | | | | | |
|---|---|---|---|---|---|
| CREBBP | NM_004380 | PSMA7 | NM_002792 | TGFBR1 | NM_004612 |
| CTBP1 | AL137653 | PSMB10 | NM_002801 | TGFBR2 | NM_003242 |
| CTBP1 | NM_001012614 | PSMB4 | NM_002796 | TGFBR2 | NM_001024847 |
| CTBP2 | NM_022802 | PSMB5 | NM_002797 | TGFBRAP1 | NM_004257 |
| CTBP2 | NM_001329 | PSMB8 | NM_004159 | TGIF1 | NM_170695 |
| DAB2 | NM_001343 | PSMB9 | NM_002800 | UBB | NM_018955 |
| EP300 | NM_001429 | PSMC2 | NM_002803 | UBC | NM_021009 |
| FLNA | NM_001456 | PSMC3 | NM_002804 | UBB | NM_006398 |
| FLNB | NM_001457 | PSMC4 | NM_006503 | UBE3A | NM_130839 |
| FLNB | AK022486 | PSMC5 | NM_002805 | UBE3B | NM_183415 |
| FLNC | NM_001458 | PSMD4 | NM_002810 | UBE3C | NM_014671 |
| FOXH1 | NM_003923 | PSMD4 | NM_153822 | UBR1 | NM_174916 |
| HACE1 | NM_020771 | RAB5A | NM_004162 | UBR2 | NM_015255 |
| HDAC1 | NM_004964 | RAB5B | NM_002868 | WWP1 | NM_007013 |
| HDAC10 | NM_032019 | RAB5B | X54871 | WWP2 | NM_199423 |
| HDAC10 | AL512711 | RAB5C | NM_201434 | WWP2 | NM_199424 |
| HDAC11 | NM_024817 | RAN | NM_006325 | XRO1 | NM_003400 |
| HDAC2 | NM_001527 | RNF8 | NM_003858 | ZFYVE9 | NM_004799 |
| HDAC3 | NM_003883 | SIN3A | NM_015477 | ZFYVE9 | NM_007323 |

The group of genes related to pancreatic cancer may be exemplified by the genes listed in the following Table 23.

**[Table 23]**

| GeneSymbol | GenbankAccession | GeneSymbol | GenbankAccession |
|---|---|---|---|
| AKT1 | NM_005163 | MAPK1 | NM_138957 |
| AKT2 | NM_001626 | MAPK1 | NM_002745 |
| AKT3 | NM_181690 | MAPK3 | NM_002746 |
| AKT3 | NM_005465 | MDM2 | NM_002392 |
| ARHGEF7 | NM_145735 | MDM2 | NM_006879 |
| ARHGEF7 | NM_003899 | MMP1 | NM_002421 |
| BCL2 | M13995 | MMP2 | NM_004530 |
| BCL2 | NM_000633 | MMP3 | NM_002422 |
| BCL2L1 | NM_138578 | MMP7 | NM_002423 |
| BCL2L1 | NM_001191 | MMP9 | NM_004994 |
| BIRC5 | NM_001012271 | NFKB1 | NM_003998 |
| BRAF | NM_004333 | NFKB2 | NM_002502 |
| BRCA2 | NM_000059 | NOTCHI | NM_017617 |
| CCNA2 | NM_001237 | PIK3CA | NM_006218 |
| CCNB1 | NM_031966 | PIK3CB | NM_006219 |
| CCND1 | NM_053056 | PIK3CD | NM_005026 |
| OCND2 | NM_001759 | PIK3R1 | NM_181523 |
| CCNE1 | NM_001238 | PIK3R2 | NM_005027 |
| CCNE2 | NM_057749 | PTGS2 | NM_000963 |
| CDC42 | NM_044472 | RAC1 | NM_199829 |
| GDC42 | NM_001791 | RAC2 | NM_002872 |
| CDK2 | NM_001798 | RAF1 | NM_002880 |
| CDK4 | NM_000075 | RB1 | NM_000321 |
| CDKN1A | NM_078467 | REL | NM_002908 |
| CDKN1A | NM_000389 | RELA | BC014095 |
| CDKN1B | NM_004064 | RELB | NM_006509 |
| CDKN2A | NM_058197 | RHOA | NM_001664 |

| | | | |
|---|---|---|---|
| CDKN2B | NM_004936 | RHOB | NM_004040 |
| CDKN2B | NM_078487 | SMAD2 | NM_005901 |
| CDKN2C | NM_001262 | SMAD2 | NM_001003652 |
| CDKN2D | NM_001800 | SMAD3 | NM_005902 |
| CYP2E1 | NM_000773 | SMAD3 | U68019 |
| E2F1 | NM_005225 | SMAD4 | NM_005359 |
| E2F3 | NM_001949 | SOS1 | NM_005633 |
| E2F4 | NM_001950 | SRC | NM_005417 |
| EGF | NM_001963 | STAT1 | NM_139266 |
| EGFR | NM_005228 | STAT1 | NM_007315 |
| ELK1 | NM_005229 | STAT2 | NM_005419 |
| ERBB2 | NM_001005862 | STAT3 | NM_213662 |
| FIGF | NM_004469 | STAT3 | BC029783 |
| GRB2 | NM_002086 | STAT5B | NM_012448 |
| HBEGF | NM_001945 | STAT5B | BC020868 |
| HSP90AA1 | NM_005348 | STAT6 | NM_003153 |
| IGF1 | NM_000618 | TGFA | NM_003236 |
| IL6 | NM_000600 | TGFB1 | NM_000660 |
| JAK1 | NM_002227 | TGFB2 | NM_003238 |
| JAK2 | NM_004972 | TGFB3 | NM_003239 |
| JAK3 | NM_000215 | TGFBR1 | NM_004612 |
| JAK3 | BC028068 | TGFBR2 | NM_003242 |
| KDR | NM_002253 | TGFBR2 | NM_001024847 |
| KIT | NM_000222 | TP53 | NM_000546 |
| KRAS | NM_033360 | VEGFA | NM_001025366 |
| KRAS | BC029545 | VEGFA | NM_003376 |
| MAP2K1 | NM_002755 | VEGFB | NM_003377 |
| MAP2K2 | NM_030662 | VEGFC | NM_005429 |

The group of genes related to prostate cancer may be exemplified by the genes listed in the following Table 24.

**[Table 24]**

| **GeneSymbol** | **GenbankAccession** |
|---|---|
| APC | NM_000038 |
| AR | NM_000044 |
| CAV1 | NM_001753 |
| CCNA1 | NM_003914 |
| ODH1 | NM_004360 |
| CDKN2A | NM_058197 |
| DKK3 | NM_015881 |
| DLC1 | NM_024767 |
| DLC1 | NM_182643 |
| EDNRB | NM_003991 |
| GPX3 | NM_002084 |
| GSTP1 | NM_000852 |
| MGMT | NM_002412 |
| MSX1 | NM_002448 |
| OPCML | NM_001012393 |
| PDLIM4 | NM_003687 |
| PTGS2 | NM_000963 |
| RARB | NM_000965 |
| RASSF1 | NM_170713 |
| SFRP1 | NM_003012 |
| SLC5A8 | NM_145913 |
| TIMP2 | AK057217 |
| TIMP2 | NM_003255 |
| TNFRSF10D | NM_003840 |
| ZNF185 | AK095258 |

The group of genes related to liver cancer may be exemplified by the genes listed in the following Table 25.

**[Table 25]**

| **GeneSymbol** | **GenbankAccession** |
|---|---|
| CCND2 | NM_001759 |
| CDH1 | NM_004360 |
| CDKN1A | NM_078467 |
| CDKN1A | NM_000389 |
| CDKN1B | NM_004064 |
| CDKN2A | NM_058197 |
| DAB2IP | NM_138709 |
| DAB2IP | NM_032552 |
| DLC1 | NM_024767 |
| DLC1 | NM_182643 |
| DLEC1 | NM_007335 |
| E2F1 | NM_005225 |
| EP300 | NM_001429 |
| FHIT | NM_002012 |
| GSTP1 | NM_000852 |
| MSH2 | NM_000251 |
| MSH3 | NM_002439 |
| OPCML | NM_001012393 |
| PYCARD | MM_013258 |
| RASSF1 | NM_17013 |
| RELN | NM_005045 |
| RUNX3 | NM_004350 |
| SFRP2 | NM_003013 |
| SOCS1 | NM_003745 |
| TNFRSF10D | NM_003840 |
| WT1 | NM_024424 |

The group of genes related to lung cancer may be exemplified by the genes listed in the following Table 26.

**[Table 26]**

| GeneSymbol | GenbanAccession |
|---|---|
| APBA1 | NM_001163 |
| APC | NM_000038 |
| CADM1 | NM_014333 |
| CDH1 | NM_004360 |
| CDH13 | NM_001257 |
| CDKN1C | NM_000076 |
| CDKN2A | NM_058197 |
| CDKN2B | NM_004936 |
| CDKN2B | NM_078487 |
| CXCL12 | NM_199168 |
| CXCL12 | AK090482 |
| CXCL12 | NM_000609 |
| CYP1B1 | NM_000104 |
| DLC1 | NM_024767 |
| DLC1 | NM_182643 |
| FHIT | NM_002012 |
| MGMT | NM_002412 |
| MLH1 | NM_000249 |
| MTHFR | NM_005957 |
| OPCML | NM_001012393 |
| PAX5 | U62539 |
| PAX5 | NM_016734 |
| PRDM2 | NM_015866 |
| PRDM2 | NM_012231 |
| RASSF1 | NM_170713 |
| RASSF2 | NM_014737 |
| SFRP1 | NM_003012 |
| TCF21 | NM_003206 |

### Additional genes in the induced cancer stem cells

It is further preferred with the induced cancer stem cells of the present invention that, in addition to the aforementioned endogenous cancer-related genes (b), at least one endogenous gene selected from the groups of genes consisting of a group of genes related to stress and toxicity, a group of genes for epigenetics of chromatin modifying enzyme, and a group of genes for stem cell transcription factor has caused experienced an increased expression.

These groups of genes can specifically be exemplified by the genes listed in the following Tables 27 to 29. GenBank accession numbers corresponding to the respective gene symbols are also listed in these Tables but they are by no means intended to limit the present invention.

The group of genes related to stress and toxicity may be exemplified by the genes listed in the following Table 27.

**[Table 27]**

| GeneSymbol | GenbankAccession | GeneSymbol | GenbankAccession |
|---|---|---|---|
| ANXA5 | NM_001154 | HMOX1 | NH_002133 |
| ATM | NM_000051 | HSF1 | NM_005526 |
| ATM | BC022307 | HSP90AB1 | NM_007355 |
| BAX | NM_138764 | HSPA1A | NM_005345 |
| BAX | NM_138765 | HSPA1L | NM_005527 |
| BAX | NM_138763 | HSPA2 | NM_021979 |
| BCL2L1 | NM_138578 | HSPA4 | NM_002154 |
| BSL2L1 | NM_001191 | HSPA5 | NM_005347 |
| CASP1 | NM_033292 | HSPA6 | NM_002155 |
| CASP10 | NM_032977 | HSPA8 | NM_006597 |
| CASP10 | NM_032974 | HSPA8 | NM_153201 |
| CASP8 | NM_033356 | HSPB1 | NM_001540 |
| GASP8 | NM_033358 | HSPD1 | NM_002156 |
| CAT | NM_001752 | HSPE1 | NM_002157 |
| CCL21 | NM_002989 | HSPH1 | NM_006644 |
| CCL3 | 000044 | IGFBP6 | NM_002178 |
| CCL4 | NM_002984 | IL18 | NM_001562 |
| CCNC | NM_005190 | IL1A | NM_000575 |
| CCND1 | NM_053056 | IL1B | NM_000576 |
| CCNG1 | NM_004060 | IL6 | NM_000600 |
| CDKN1A | NM_078467 | LTA | NM_000595 |
| CDKN1A | NM_000389 | MDM2 | NM_002392 |

| | | | |
|---|---|---|---|
| CHEK2 | NM_001005735 | MDM2 | NM_006879 |
| CRYAB | NM_001885 | MIF | NM_002415 |
| CSF2 | NM_000758 | MT2A | NM_005953 |
| CXCL10 | NM_001565 | NFKB1 | NM_003998 |
| CYP1A1 | NM_000499 | NFKB1A | NM_020529 |
| CYP2E1 | NM_000773 | PCNA | NM_002592 |
| CYP7A1 | NM_000780 | POR | NM_000941 |
| DDB1 | NM_001923 | PRDX1 | NM_002574 |
| DDIT3 | NM_004083 | PRDX2 | NM_181738 |
| DNAJA1 | NM_001539 | PRDX2 | NM_005809 |
| DNAJB4 | NM_007034 | PTGS1 | NM_000962 |
| E2F1 | NM_005225 | RAD23A | NM_005053 |
| EGR1 | NM_001964 | RAD50 | NM_005732 |
| EPHX2 | NM_001979 | SERPINE1 | NM_000602 |
| ERCC1 | NM_001983 | SOD1 | NM_000454 |
| ERCC3 | NM_000122 | SOD2 | BC016934 |
| FASLG | NM_000639 | SOD2 | NM_000636 |
| FMO1 | NM_002021 | TNF | NM_000594 |
| FMO5 | NM_001461 | TNFRSF1A | NM_001065 |
| GADD45A | NM_001924 | TNFSF10 | NM_003810 |
| GDF15 | NM_004864 | TP53 | NM_000546 |
| GPX1 | NM_201397 | UNG | NM_003362 |
| GSR | BC035691 | XRCC1 | NM_006297 |
| GSR | NM_000637 | XRCC2 | CR749256 |
| GSTM3 | NM_000849 | XRCC2 | NM_005431 |

The group of genes for epigenetics of chromatin modifying enzyme may be exemplified by the genes listed in the following Table 28.

**[Table 28]**

| GeneSymbol | GenbankAccession | GeneSymbol | GenebankAccession |
|---|---|---|---|
| ASH1L | NM_018489 | MYST3 | AK027361 |
| ATF2 | AK128731 | MYST4 | NM_012330 |
| ATF2 | NM_001880 | NCOA1 | NM_147233 |
| AURKA | NM_198433 | NCOA3 | NM_181659 |
| AURKB | NM_004217 | NCOA6 | NM_014071 |
| AURKC | NM_001015878 | NEK6 | NM_014387 |
| CDYL | NM_170752 | NSD1 | NM_022455 |
| CIITA | NM_000246 | PAK1 | NM_002576 |
| CIITA | U18288 | PRMT1 | NM_198319 |
| CIITA | U18259 | PRMT2 | NM_206962 |
| CSRP2BP | NM_020536 | PRMT3 | NM_005788 |
| DNMT1 | NM_001379 | PRMT5 | NM_0006109 |
| DNMT3A | NM_175629 | PRMT6 | NM_018137 |
| DNMT3A | NM_175630 | PRMT7 | NM_019023 |
| DNMT3B | NM_175850 | PRMT8 | NM_019854 |
| DOT1L | NM_032482 | RNF2 | NM_007212 |
| DZIP3 | AB014575 | RNF20 | NM_019592 |
| DZIP3 | NM_014648 | RPS6KA3 | NM_004586 |
| EHMT2 | NM_006709 | RPS6KA5 | NM_182398 |
| EHMT2 | NM_025256 | RPS6KA5 | NM_004755 |
| ESCO1 | NM_052911 | SETD1A | NM_014712 |
| ESCO2 | NM_001017420 | SETD1B | |

| | | | |
|---|---|---|---|
| HAT1 | NM_003642 | SETD2 | NN_014159 |
| HDAC1 | NM_004964 | SETD3 | NM_032233 |
| HDAC10 | NM_032019 | SETD4 | NM_001007259 |
| HDAC10 | AL512711 | SETD4 | NM_017438 |
| HDAC11 | NM_024827 | SETD5 | BX648380 |
| HDAC2 | NM_001527 | SETD5 | NM_001080517 |
| HDAC3 | NM_003883 | SETD6 | NM_024860 |
| HDAC4 | NM_006037 | SETD7 | NM_030648 |
| HDAC5 | NM_001015053 | SETD8 | NM_020382 |
| HDAC6 | BC011488 | SETDB1 | NM_012432 |
| HDAC6 | NM_006044 | SETDB2 | NM_031915 |
| HDAC8 | NM_018486 | SMYD3 | NM_022743 |
| HDAC9 | NM_178423 | SUV39H1 | NM_003173 |
| HDAC9 | NM_058177 | SUV420H1 | NM_017635 |
| HDAC9 | NM_014707 | SUV420H1 | NM_016028 |
| HDAC9 | NM_058176 | UBE2A | NM_003336 |
| MBD2 | NM_003927 | UBE2B | NM_003337 |
| MBD2 | NM_015832 | UBE2B | BC001694 |
| MLL | NM_005933 | USP16 | NM_006447 |
| MLL | AF487905 | USP21 | NM_012475 |
| MLL | AF272382 | USP21 | NM_001014443 |
| MLL3 | NM_170606 | USP22 | BC110499 |
| MLL5 | NM_018682 | USP22 | AB028988 |
| MYSM1 | AB067502 | WHSC1 | NM_133334 |
| MYST1 | NM_032188 | WHSC1 | NM_133330 |
| MYST2 | NM_007067 | WHSC1 | NM_007331 |
| MYST3 | NM_006766 | WHSC1 | NM_133336 |

The group of genes for stem cell transcription factor may be exemplified by the genes listed in the following Table 29.

**[Table 29]**

| GeneSymbol | GenbankAccession | GeneSymbol | GenbankAccession |
|---|---|---|---|
| CDX2 | NM_001265 | MSX2 | NM_002449 |
| DACH1 | NM_080759 | MYC | NM_002467 |
| DLX1 | NM_178120 | MYC | M13930 |
| DLX2 | NM_004405 | NANOG | NM_024865 |
| DNMT3B | NM_175850 | NEUROD1 | NM_002500 |
| EGR3 | NM_004430 | NFATC1 | NM_172387 |
| ESR1 | NM_000125 | NFATC1 | NM_172390 |
| ESR1 | U68068 | NKX2-2 | NM_002509 |
| EZH2 | NM_004456 | NOTCH2 | NM_024408 |
| FOXA1 | NM_004496 | NR2F2 | NM_021005 |
| FOXA2 | NM_021784 | OLIG2 | NM_005806 |
| FOXP1 | NM_032882 | PAX1 | NM_006192 |
| FOXP2 | NM_014491 | PAX5 | U62539 |
| FOXP2 | NM_148900 | PAX5 | NM_016734 |
| FOXP3 | NM_014009 | PAX6 | NM_001604 |
| GATA1 | NM_002049 | PAX9 | U59828 |
| GATA6 | NM_005257 | PAX9 | NM_006194 |
| GLI2 | NM_005270 | PCNA | NM_002592 |
| HAND1 | NM_004821 | PITX2 | NM_153426 |
| HOXA10 | NM_018951 | PITX3 | NM_005029 |
| HOXA10 | S69027 | POU4F1 | NM_006237 |
| HOXA11 | NM_005523 | POU4F2 | NM_004575 |

| | | | |
|---|---|---|---|
| HOXA2 | NM_006735 | POU5F1 | NM_002701 |
| HOXA3 | NM_153631 | PPARG | NM_138711 |
| HOXA7 | NM_006896 | RB1 | NM_000321 |
| HOXA9 | NM_152739 | RUNX1 | NM_001001890 |
| HOXB1 | NM_002144 | RUNX1 | X90978 |
| HOXB13 | NM_006361 | SIX2 | NM_016932 |
| HOXB3 | NM_002146 | SMAD2 | NM_005901 |
| HOXB5 | NM_002147 | SMAD2 | NM_001003652 |
| HOXB8 | NM_024016 | SOX2 | NM_003106 |
| HOXC10 | NM_017409 | SOX6 | NM_03326 |
| HOXC12 | NM_173860 | SOX9 | NM_000346 |
| HOXC4 | NM_014620 | SP1 | NM_138473 |
| HOXC5 | NM_018953 | STAT1 | NM_139266 |
| HOXC6 | NM_153693 | STAT1 | NM_007315 |
| HOXC9 | NM_006897 | STAT3 | NM_213662 |
| HOXD1 | NM_024501 | STAT3 | BC029783 |
| HOXD10 | NM_002148 | TBX5 | NM_080718 |
| HOXD4 | NM_014621 | TBX5 | NM_000192 |
| HTR7 | NM_019859 | TDGF1 | NM_003212 |
| IRX4 | NM_016358 | TERT | NM_198253 |
| ISL1 | NM_002202 | TLX3 | NM_021025 |
| JUN | NM_00228 | VDR | NM_001017535 |
| KLF2 | NM_016270 | WRN | NM_000553 |
| KLF4 | NM_004235 | WT1 | NM_024424 |
| LIN28B | NM_001004317 | ZFPM2 | NM_012082 |
| LMX1B | NM_002316 | ZIC1 | NM_003412 |

It is also within the scope of the present invention that, in addition to the aforementioned endogenous cancer-related genes (b), at least one endogenous gene selected from the group of hepatocyte specific genes has caused an increased expression.

The group of hepatocyte specific genes may be exemplified by the following genes associated with the functions of the liver. Since each of these genes may function as a gene associated with a property of cancer, it is preferred with the induced cancer stem cells of the present invention that, in addition to the aforementioned cancer-related genes (b), genes of the group of hepatocyte specific genes have been confirmed to cause an increase in expression.

The group of hepatocyte specific genes can specifically be exemplified by the group of hepatocyte related genes (Hepa) listed in the following Table 30. GenBank accession numbers corresponding to the respective gene symbols are also listed in this Table but they are by no means intended to limit the present invention.

**[Table 30]**

| GeneSymbol | GenbankAccession | GeneSymbol | GenbankAccession |
|---|---|---|---|
| A2M | NM_000014 | HHEX | NM_002729 |
| ACE2 | NM_021804 | HMGCS2 | NM_005518 |
| AFP | NM_001134 | HP | NM_005143 |
| AGT | NM_000029 | HPX | NM_000613 |
| AHSG | NM_001622 | HSD17B2 | NM_002153 |
| AK074614 | IAK074614 | IGF2 | NM_001007139 |
| AK124281 | AK124281 | IL32 | NM_001012631 |
| AK126405 | AK126405 | INHBB | NM_002193 |
| ALB | NM_000477 | KYNU | NM_003937 |
| ALDH1A1 | NM_000689 | LGALS2 | NM_006498 |
| ANXA8 | NM_001630 | LOC132205 | AK091178 |
| APOA1 | NM_000039 | LOC285733 | AK091900 |
| APOA2 | NM_001643 | M27126 | M27126 |
| APOA4 | NM_000482 | MAF | AF055376 |
| APOB | NM_000384 | MTTP | NM_000253 |
| AREG | NM_001657 | NNMT | NM_006169 |
| ART4 | NM_021071 | NTF3 | NM_002527 |
| ASGR2 | NM_080912 | PAG1 | NM_018440 |
| ATAD4 | NM_024320 | PDZK1 | NM_002614 |
| BC018589 | BC018589 | PLG | NM_000301 |

| | | | |
|---|---|---|---|
| C11orf9 | NM_013279 | PRG4 | NM_005807 |
| C13orf15 | NM_014059 | PSMAL | NM_153696 |
| C3 | NM_000064 | PTGDS | NM_000954 |
| C5 | NM_001735 | RASD1 | NM_016084 |
| CA414006 | CA414006 | RBP4 | NM_006744 |
| COLEC11 | NM_199235 | RNF43 | NM_017763 |
| CXCR4 | NM_001008540 | RRAD | NM_004165 |
| CXCR7 | NM_020311 | S100A14 | NM_020672 |
| DLK1 | NM_003836 | SEPP1 | NM_005410 |
| F10 | NM_000504 | SERINC2 | NM_178865 |
| F2 | NM_000506 | SERPINA1 | NM_001002236 |
| FABP1 | NM_001443 | SERPINA3 | NM_001085 |
| FGA | NM_021871 | SERPINA5 | NM_000624 |
| FGA | NM_000508 | SLC13A5 | NM_177550 |
| FGB | NM_005141 | SLC40A1 | NM_014585 |
| FGG | NM_000509 | SLPI | NM_003064 |
| FLRT3 | NM_198391 | STARD10 | NM_006645 |
| FOXA1 | NM_004496 | TDO2 | NM_005651 |
| FTCD | NM_206965 | TF | NM_001063 |
| GATA4 | NM_002052 | TTR | NM_000371 |
| GATM | NM_001482 | UBD | NM_006398 |
| GJB1 | NM_000166 | UGT2B11 | NM_001073 |
| GLT1D1 | NM_144669 | UGT2B7 | NM_001074 |
| GPRC5C | NM_022036 | VCAM1 | NM_001078 |
| GSTA3 | NM_000847 | VIL1 | NM_007127 |
| H19 | NR_002196 | VTN | NM_000638 |

It is also preferred in the case of the induced cancer stem cells of the present invention that the cells express genes characteristic of mesendodermal or endodermal stem cells, and it is particularly preferred that they are expressed in greater amounts than the genes in the undifferentiated induced pluripotent stem cell which serves as a reference for control. As such reference cell, hiPS-201B7 can be used. Gene expression data for this cell is accessible from the aforementioned Gene expression Omnibus [GEO].

The genes characteristic of mesendodermal or endodermal stem cells are not particularly limited as long as they are characteristic of the respective stem cells. To be more specific, preferred examples of the genes characteristic of mesendodermal stem cells include *GSC,* etc., and preferred examples of the genes characteristic of endodermal stem cells include *GSC, GATA4, FOXA2, SOX17,* etc.

The induced cancer stem cells of the present invention have such a nature that it is easy to induce their differentiation into cancer cells having the properties of specific tissue cells, so they can be induced for differentiation into cells that become malignant in familial tumors, for example, retinoblasts or intestinal epithelial cells, from which cancer cells as in retinoblastoma or polyposis in large intestine can be induced.

What is more, the induced cancer stem cells of the present invention can be expansion-cultured or passage-cultured for at least 3 days but they are induced cancer stem cells capable of self-renewal *in vitro* that can effectively be proliferated for at least a month, half a year or even one year and longer; this means that they are theoretically capable of self-renewal without limit.

### Media to be used and culture methods

Media for expansion culture or passage culture of the induced cancer stem cells of the present invention are not particularly limited as long as they permit the expansion culture or passage culture of embryonic stem cells, pluripotent stem cells, and the like; media suitable for the culture of embryonic stem cells, pluripotent stem cells, and the like are preferably used. Examples of such media include, but are not limited to, an ES medium [40% Dulbecco's modified Eagle medium (DMEM), 40% F12 medium (Sigma), 2 mM L-glutamine or GlutaMAX (Sigma), 1% non-essential amino acid (Sigma), 0.1 mM β-mercaptoethanol (Sigma), 15-20% Knockout Serum Replacement (Invitrogen), 10 µg/ml of gentamicin (Invitrogen), and 4-10 ng/ml of FGF2 factor]; medium which are prepared by supplementing 0.1 mM β-mercaptoethanol and 10 ng/ml of FGF2 to a conditioned medium that is the supernatant of a 24-hr culture of mouse embryonic fibroblasts (hereinafter referred to as MEF) on an ES medium lacking 0.1 mM β-mercaptoethanol (this medium is hereinafter referred to as MEF conditioned ES medium), an optimum medium for iPS cells (iPSellon), an optimum medium for feeder cells (iPSellon), StemPro (registered trademark) hESC SFM (Invitrogen), mTeSR1 (STEMCELL Technologies/VERITAS), an animal protein free, serum-free medium for the maintenance of human ES/iPS cells, named TeSR2 [ST-05860] (STEMCELL Technologies/VERITAS), a medium for primate ES/iPS cells (ReproCELL), ReproStem (ReproCELL), ReproFF (ReproCELL), and ReproFF2 (ReproCELL). For human cells, media suitable for culturing human embryonic stem cells may be used. Extracellular matrices that may be used to coat the culture dish include gelatin, collagen, Matrigel, laminin, Synthemax, etc.

The techniques for effecting expansion culture or passage culture of the induced cancer stem cells of the present invention are not particularly limited if they are methods commonly used by the skilled artisan to culture embryonic stem cells, pluripotent stem cells, and the like. A specific example that may be given is the following: the medium is eliminated from the cells, which is washed with PBS(-); a dissociation solution is added and after standing for a given period, the dissociation solution is removed; after adding a D-MEM (high glucose) medium supplemented with 1X antibiotic-antimycotic and 10% FBS, the cells are subjected to centrifugation is performed and the supernatant is removed; thereafter, 1X antibiotic-antimycotic, mTeSR and Y-27632 are added and the cell suspension is seeded on an MEF-seeded gelatin or collagen coat for effecting passage culture.

Preferably, FGF2 (bFGF) is further added to the above-mentioned media, and the preferred amount of addition ranges from 1 to 100 ng/mL. FGF2 (bFGF) is selected depending on the type of the somatic cell to be induced and there can be used FGF2 (bFGF) derived from human, mouse, bovine, equine, porcine, zebrafish, etc. What is more, the aforementioned pituitary gland extract, serum, LIF, Z-VAD-FMK, ALK5 inhibitor, PD032591, CHIR00921, etc. can be added.

Furthermore, inhibitors of Rho associated kinase (Rho-associated coiled coil containing protein kinase), such as Y-27632 (Calbiochem; water soluble) and Fasudil (HA1077: Calbiochem) can also be added to the medium during passage.

Other inhibitors that can be added include: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD 184352, and CHIR99021], two low-molecular weight inhibitors of MEK/ERK pathway and GSK3 [PD0325901 and CHIR99021], a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, an inhibitor of p53 pathway, etc.

Further, the induced cancer stem cells of the present invention can be frozen or thawed according to known methods. An exemplary method of freezing that may be used is the following: the medium is eliminated from the cells, which is washed with PBS(-); a dissociation solution is added and after standing for a given period, the dissociation solution is removed; after adding a D-MEM (high glucose) medium supplemented with 1X antibiotic-antimycotic and 10% FBS, the cells are subjected to centrifugation and the supernatant is removed; thereafter, a stock solution for freezing is added and the mixture is distributed into cryogenic vials, frozen overnight at -80 °C and thereafter stored in liquid nitrogen. An exemplary method of thawing is the following: the frozen sample is thawed in a thermostated bath at 37 °C and then suspended in a D-MEM (high glucose) medium supplemented with 1X antibiotic-antimycotic and 10% FBS before use.

### Method of producing induced cancer stem cells

In its second embodiment, the present invention provides a process for producing the induced cancer stem cell, wherein an induced cancer stem cell capable of self-renewal *in vitro* is produced from a non-embryonic starter somatic cell selected from the group consisting of a somatic cell isolated from a mammal having (a) a mutation in a tumor suppressor gene and a somatic cell that is isolated from a carcinogenic mammal and which has an aberration which is either (a) a mutation in a tumor suppressor gene or (b) increased expressin of a cancer-related gene.

This process is characterized in that the starter somatic cell is brought to such a state that the genetic products of *POU5F1, KLF4,* and *SOX2* are present therein. As a result, the aforementioned gene (1) (self-renewal related gene) which is inherent in the starter somatic cell is expressed, whereupon the induced cancer stem of the present invention is eventually induced. The term "bringing the starter somatic cell to such a state" should be understood as a broad concept that covers not only the case of adjusting the cell to have such a state but also the case of selecting a cell that has been brought to such a state and conditioning the same.

### Starter cell for the induced cancer stem cell

Since the induced cancer stem cell of the present invention inherit the aberration that was inherent in the starter somatic cell serving as its source, the starter somatic cells have (a) a mutation in a tumor suppressor gene or (b) increased expresstion of a cancer-related gene; hence, the starter somatic cell, or the somatic cell that serves as the starter, must be a somatic cell selected from the group consisting of a somatic cell isolated from a mammal having (a) a mutation in a tumor suppressor gene and a somatic cell that is isolated from a carcinogenic mammal and which has an aberration which is either (a) a mutation in a tumor suppressor gene or (b) increased expressin of a cancer-related gene.

The mammal from which the starter somatic cell is to be isolated is not particularly limited as long as it is a mammal and may be exemplified by rat, mouse, guinea pig, dog, cat, porcine such as minipig, bovine, equine, primates such as monkeys including a cynomolgus monkey, and human, with rat, mouse, guinea pig, dog, cat, minipig, equine, cynomolgus monkey, and human being preferred, and human is used with particular preference.

The starter somatic cell to be used in the production process of the present invention must be a non-embryonic cell, namely, a cell derived from a non-reproductive tissue. Therefore, cells derived from reproductive tissues are not encompassed by the starter somatic cell to be used in the present invention.

Such non-embryonic starter somatic cells are not particularly limited if they are non-embryonic cells as noted above and it is possible to use somatic cells isolated from various tissues of mammals at various stages of development. Specific examples are mentioned below but they are not intended to limit the present invention: they include not only somatic cells isolated from various organs such as the brain, liver, esophagus, stomach, duodenum, small intestine, large intestine, colon, pancreas, kidney, lung, and mammary gland but also somatic cells isolated from bone marrow fluid, adipose tissue, peripheral blood, skin, and skeletal muscle. Most of these cells are readily available as medical waste, typically during operation in cancer therapy.

It is also possible to use tissues that accompany childbirth such as umbilical cord tissues (umbilical cord and umbilical cord blood), amnion, placenta, and cells derived from amniotic fluid; in particular, there may be used tissues just after birth such as various tissues ofneonates (e.g., neonatal skin), as well as umbilical cord tissues (umbilical cord and umbilical cord blood) such as tissues derived from blood vessels derived from umbilical cord.

The somatic cell that is isolated from a mammal and which has a mutation in a tumor suppressor gene as referred to in (a) is not particularly limited if it has such aberration and an example that can be used is a somatic cell isolated from a mammal having a genetic aberration that can trigger a familial tumor.

The somatic cell isolated from a mammal having a genetic aberration may be exemplified by a somatic cell isolated from a mammal manifesting a familial tumor, as well as a somatic cell that is isolated from an individual in a kin relationship to said mammal and which has a genetic aberration that can trigger a familial tumor. These somatic cells may be such that the genetic aberration triggering a familial tumor is located on one (an allele) of a pair of alleles (precancerous cell) or on both alleles (malignant cell). If a precancerous cell is used, the induced precancer stem cell of the present invention is induced, and if a malignant cell is used, the induced malignant stem cell of the present invention is induced.

The above-mentioned somatic cell having a genetic aberration on one (an allele) of a pair of alleles may be exemplified by a somatic cell isolated other than from a cancerous tissue in a mammal manifesting a familial tumor, as well as a somatic cell that is isolated from an individual in a kin relationship to said mammal and which has a genetic aberration that can trigger a familial tumor (precancerous cell). In contrast, the somatic cell having a genetic aberration on both of a pair of alleles (malignant cell) may be exemplified by a cancer cell in a mammal that manifests a familial tumor.

Since it is difficult to isolate only cancer cells from a tissue, cells in a cancer tissue which is substantially made up of cancer cells are preferably used in practice. Another option is to use cells in a non-cancer tissue involving cancer cells.

Germ layers as the source of the starter somatic cell to be used in the production of the induced cancer stem cell of the present invention are not particularly limited. If the induced cancer stem cell to be produced in the present invention is endodermal, a somatic cell that is an endodermal cell as derived from the liver, stomach, large intestine, or colon may be used as the starter somatic cell, and a somatic cell derived from the stomach or colon is used with particular preference.

The starter somatic cell to be used in the production process of the present invention may be somatic cancer cells as isolated from a caricinogenic mammal. Such cells have aberrations peculiar to cancer cells, as exemplified by (a) a mutation in a tumor suppressor gene, abnormal gene expression, and the like. These somatic cells are isolated from a carcinogenic mammal, especially from a cancr tissue involving cancer cells and precancerous cells or from a non-cancer tissue involving cancer cells and precancerous cells but, in practice, it is difficult to isolate only cancer cells or precancerous cells. Nevertheless, whether the cells used as the starter were cancer cells or precancerous cells or whether they were normal cells or non-cancer cells can be verified by determining whether the finally obtained induced cancer stem cell of the rpesent invention has such aberrations as (a) a mutation in a tumor suppressor gene and abnormal gene expression (note that a germline mutation can be identified even in the starter cell.) This is because if the finally obtained induced cancer stem cell of the rpesent invention has such aberrations as (a) a mutation in a tumor suppressor gene and abnormal gene expression, it is recognized that these aberrations have been inherited from the starter cell.

The tissue as the source of the starter somatic cell that is to be used in the process for producing the induced cancer stem cells of the present inventin is not particularly limited. For example, if somatic cells isolated from a carcinogenic mammal are to be used, they may be the following; in the case of producing induced mesendodermal malignant stem cells or induced endodermal malignant stem cells, mesendodermal or endodermal somatic cells may respectively be used. Hence, any somatic cells that have been isolated from the liver, stomach, duodenum, small intestine, large intestine, colon, pancreas, lung, etc. can be induced to give rise to induced mesendodermal malignant stem cells or induced endodermal malignant stem cells.

The types of cancers in carcinogenic mammals are not particularly limited and they may be any cancers such as malignant tumor, solid cancer, carcinoma, sarcoma, brain tumor, hematopoietic organ cancer, leukemia, lymphoma, multiple myeloma, and the like. More specific examples include oral cancer, cancer of the throat, cancer of upper airway, lung cancer, lung cell cancer, esophageal cancer, stomach cancer, duodenal cancer, pancreatic cancer, liver cancer, gallbladder cancer, biliary tract cancer, bowel cancer, colon cancer, rectal cancer, breast cancer, thyroid cancer, uterine body cancer, cervical cancer, ovary cancer, testis cancer, kidney cancer, bladder cancer, prostate cancer, skin cancer, malignant melanoma, brain tumor, bone sarcoma, and blood cancer.

The starter somatic cells that are to be used in the production process of the present invention may be used immediately after being isolated from mammals or they can be used after being stored, cultured or otherwise treated by known methods. In the case of culturing, the number of passages is not particularly limited.
The gene symbols for *POU5F1, KLF4,* and *SOX2,* as well as the corresponding Genbank accession numbers are given in Table 31.

**[Table 31]**

| GoneSymbol | GenbankAccession |
|---|---|
| KLF4 | NM_004235 |
| POU5F1 | NM_002701 |
| SOX2 | NM_003106 |

In the aforementioned step of inducing the induced cancer stem cell of the present invention, it suffices that the aforementioned starter somatic cell is brought to such a state that the genetic products of *POU5F1, KLF4,* and *SOX2* are present therein. Methods for doing this are exemplified by, but are not limited to, those which are known as induction techniques for giving rise to induced pluripotent stem cells.

In the aforementioned step of inducing the induced cancer stem cell of the present invention, the desired induced cancer stem cell can also be produced by ensuring that the genetic products of *POU5F1, KLF4,* and *SOX2* are present in specified proportions within the starter somatic cell as it is being induced to give rise to the induced cancer stem cell of the present invention.

More specifically, if the intracellular relative abundances of *POU5F1, KLF4,* and *SOX2* in the aforementioned starter smatic cell are adjusted to satisfy the relation of *POU5F1 > SOX2,* there are induced endodermal induced cancer stem cells as in the stomach, large intestine, liver, lung, pancreas, etc.

In the case of inducing endodermal induced cancer stem cells, it is preferred to make adjustment to satisfy the relation of *POU5F1 > KLF4 > SOX2;* this is preferred from the viewpoint of inducing the induced cancer stem cell of the present invention in high efficiency. It is particularly preferred that the ratio in use between *POU5F1, KLF4,* and *SOX2* is 4:2:1. In the methods of preparation of induced pluripotent stem cells, as disclosed in Takahashi et al. (Takahashi K, Yamanaka S et al., Cell, 2007, 131, 861-872; Non-Patent Document 3) and Masaki et al. (Masaki H, Ishikawa T et al., Stem Cell Res., 2008, 1, 105-115: Non-Patent Document 4), the respective genes are used in equal amounts, i.e., at a ratio of 1:1:1, and the standard protocol information available from the Center for iPS Cell Research and Application (CiRA), Kyoto University also recommends the use of those genes in equal amounts.

In the aforementioned step of inducing the induced cancer stem cell of the present invention, genes that may be used to elevate the intensity of expression of *POU5F1, KLF4,* and *SOX2* are *POU5F1, KLF4,* and *SOX2* per se. If the above-mentioned *POU5F1, KLF4,* or *SOX2* is expressed only insufficiently in the aforementioned starter somatic cell, the insufficient gene or genetic product is transduced into the same cell, and if the above-mentioned *POU5F1, KLF4,* or *SOX2* is expressed in the aforementioned cell, other gene or a genetic product thereof may be transduced in place of the above-mentioned *POU5F1, KLF4,* or *SOX2.*

If the cell has already strongly expressed *POU5F1, KLF4,* or *SOX2,* the induced cancer stem cell of the present invention can be induced by transducing other genes that are known to give rise to induced pluripotent stem cells, as exemplified by *NANOG, LIN28, TBX3, PRDM14, L-MYC, c-MYC, N-MYC, SALL1, SALL4, UTFI , ESRRB, NR5A2, REM2 GTPase, TCL-1A,* the Yes-associated protein (YAP) gene, the E-cadherin gene, the p53 dominant negative mutant gene, *p53shRNA,* etc.

The gene symbols for *NANOG, LIN28, TBX3,* and *c-MYC,* as well as the corresponding Genbank accession numbers are given in Table 32.

**[Table 32]**

| **GeneSymbol** | Genbank Accession |
|---|---|
| NANOG | NM_024865 |
| LIN28 | NM_024674 |
| TBX3 | NM_016569 |
| C-MYC | NM_002467 |

It is believed that by bringing the genetic products of the aforementioned genes *POU5F1, KLF4,* and *SOX2* to such a state that they are present in the starter somatic cell, the group of self-renewal related genes changes their chromatin structure and the intracellular genetic products of *POU5F1, KLF4,* and *SOX2* induce the expression of the group of endogenous self-renewal related genes, whereupon the cell starts to be self-renewed.

Methods by which proteins, mRNAs or the like that are genetic products of the aforementioned genes *POU5F1, KLF4,* and *SOX2* or genes that are substitutes for these genes can be transduced into the aforementioned starter somatic cell include, but are not limited to, those which are known as induction techniques for giving rise to induced pluripotent stem cells. For example, proteins, mRNAs or the like that are genetic products of these genes may be added to culture media.

In the aforementioned induction step, in order to increase the efficiency of induction to the induced cancer stem cell, compounds that are known to give rise to induced pluripotent stem cells may further be added to the culture media used to give rise to the induced hepatic stem cell of the present invention, and these compounds are exemplified by inhibitors including: three low-molecular weight inhibitors of FGF receptor tyrosine kinase, MEK (mitogen activated protein kinase)/ERK (extracellular signal regulated kinases 1 and 2) pathway, and GSK (Glycogen Synthase Kinase) 3 [SU5402, PD184352, and CHIR99021], two low-molecular weight inhibitors of MEK/ERK pathway and GSK3 [PD0325901 and CHIR99021], a low-molecular weight compound as an inhibitor of the histone methylating enzyme G9a [BIX-01294 (BIX)], azacitidine, trichostatin A (TSA), 7-hydroxyflavone, lysergic acid ethylamide, kenpaullone, an inhibitor of TGF-β receptor I kinase/activin-like kinase 5 (ALK5) [EMD 616452], inhibitors of TGF-β receptor 1 (TGFBR1) kinase [E-616452 and E-616451], an inhibitor of Src-family kinase [EI-275], thiazovivin, PD0325901, CHIR99021, SU5402, PD184352, SB431542, anti-TGF-β neutralizing antibody, A-83-01, Nr5a2, a p53 inhibiting compound, siRNA against p53, an inhibitor of p53 pathway, ctc. If necessary, hypoxic culture may be performed to achieve efficient induction of the induced cancer stem cell of the present invention.

As described above, in addition to the aforementioned genes *POU5F1, KLF4,* and *SOX2,* as well as their genetic products, the following may typically be used in order to enhance the efficiency of induction of the aforementioned induced cancer stem cell, as have been noted earlier: genes such as the aforementined *NANOG, LIN28, TBX3, PRDM14, L-MYC, c-MYC, N-MYC, SALL1, SALL4, UTF1, ESRRB, NR5A2, REM2 GTPase, TCL-1A,* Yes-associated protein (YAP) gene, E-cadherin gene, p53 dominant negative mutant gene, *p53shRNA,* as well as their genetic products and compounds; bFGF; as well as the ALK inhibitor (e.g. A-83-01), TGF-beta RI inhibitor, and TGF-beta RI kinase inhibitor.

To produce the induced cancer stem cells of the present invention from the aforementioned starter somatic cell, genes may be transduced into the aforementioned mammalian cell by any known methods without particular limitation, and vectors that can be used include viral vectors, plasmids, artificial chromosomes (HAC), episomal vectors (EBV), micircle vectors, polycistronic expression vectors, vectors as an application of the Cre/loxP system, vectors making use of a phage integrase, and a transposon such as a piggyback.

Viral vectors that can be used to transduce genes into the aforementioned starter somatic cell may be of any known types. Examples include, but are not limited to, lentiviral vectors, retroviral vectors, adenoviral vectors, monkey immunodeficiency virus vectors (DNAVEC Corporation), adeno-associated viral vectors (DNAVEC Corporation), Sendai virus vectors having no residual exogenous genes in the genome (DNAVEC Corporation, and MEDICAL & BIOLOGICAL LABORATORIES CO., LTD.), Sendai mini vectors (DNAVEC Corporation), and HVJ. Retroviral vectors include Moloney murine leukemia virus-derived retroviral vectors.

Viral vector plasmids that can be used may be of any known types of viral vector plasmids. For example, a vector preferably used as retroviral vector plasmids are pMXs, pMXs-IB, pMXs-puro, and pMXs-neo (pMXs-IB being prepared by replacing a blasticidin resistance gene with the puromycin resistance gene in the pMXs-puro) [Toshio Kitamura et. al., "Retrovirus-mediated gene transfer and expression cloning: Powerful tools in functional genomics", Experimental Hematology, 2003, 31(11):1007-14], and other examples include MFG [Proc. Natl. Acad. Sci. USA, 92, 6733-6737 (1995)], pBabePuro [Nucleic Acids Research, 18, 3587-3596 (1990)], LL-CG, CL-CG, CS-CG, CLG [Journal of Virology, 72, 8150-8157 (1998)], etc. Adenoviral vector plasmids include pAdex1 [Nucleic Acids Res., 23, 3816-3821 (1995)], etc.

### Additional step in the preparation of induced cancer stem cells

In addition to the above-described induction step, the production process of the present inventin may further include the step of sorting a single cell in one well and proliferating the cell. This is a step in which cells, either stained or not stained with any one antibody selected from the group consisting of an anti-ALB antibody, an anti-FABP1 antibody, an anti-IGF-II antibody, an anti-DLK1 antibody, an anti-PDGFR α antibody, an anti-VEGFR2 antibody, an anti-E-cadherin antibody, an anti-CXCR4 antibody, an anti-PDGFR β antibody, an anti-cadherin 11 antibody, an anti-CD34 antibody, and an anti-IGF-R1, are proliferated with a single cell being sorted in one well.

In an exemplary method, the induced cancer stem cells of the present invention are stained with one of specific antibodies against the above-mentioned E-cadherin and, then, using PERFLOW^{™} Sort (Furukawa Electric Co., Ltd.), the specific antibody stained cells are single-sorted on a 96-well plate or the like such that one cell is contained in one well. It is also possible to use unstained cells instead of the cells stained with the specific antibody.

The production process of the present invention may further include a selection step in which the malignancy or a specific marker of the induced cancer stem cell capable of self-renewal *in vitro* is identified to select the cell of interest.

The term "malignancy" as used herein refers to various properties of cancer cells that are associated with their ability to proliferate without limit, invasion, metastasis, resistance, and recurrence. The term "specific marker" refers to properties by which cancer cells can be identified and they include proteins (e.g. secreted proteins) or specific proteins or sugar-chain antigens that are located on the surfaces of cancer cells. An exemplary specific marker that can be used is (b) increased expression of cancer-related genes. Included among the cancer-related genes referred to in (b) are a group of genes related to angiogenesis, a group of cancer-related pathway genes, a group of genes related to stromal barrier, a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer, and an increased expression of an endogenous cancer-related gene selected from at least one of these groups of genes may be given as an example of the specific marker.

The aforementioned selection step may be a step of comparing a cell obtained by induction treatment of a non-embryonic starter somaic cell selected from the group consisting of a somatic cell isolated from a mammal having (a) a mutation in a tumor suppressor gene and a somatic cell that is isolated from a carcinogenic mammal and which has an aberration which is either (a) a mutation in a tumor suppressor gene or (b) increased expressin of a cancer-related gene with an induced mesendodermal stem cell, an induced endodermal stem cell or an induced pluripotent stem cell as induced from a reference somatic cell isolated from a mammal, or an embryonic stem cell.

The above-mentioend reference somatic cell isolated from a mammal is not particularly limited if it is a somatic cell isolated from various tissues of the mammal at various stages of growth. Such tissues of the reference mammal may be exemplified by the various tissues listed earlier as examples of the tissues from which the starter somatic cell can be obtained.

The above-mentioend reference somatic cell isolated from a mammal is not particularly limited if it is a normal cell or non-cancer cell having no aberration as is found in the starter somatic cell to be used in the presnt invention; examples that can be used are somatic cells derived from adults, neonates, or neonatal skins, or somatic cells obtained from carcinogenic mammals but which are non-cancer cells or somatic cells in carcinogenic individuals that are substantially free of aberrations that are found in the starter somatic cell to be used in the presnt invention. It is especially recommended to use the somatic cells derived from adults, neonates, or neonatal skins since these are considered to involve fewer aberrations that are found in the starter somatic cell to be used in the presnt invention.

However, since it is difficult to achieve isolation of a single normal cell or non-cancer cell from a tissue, a cell group that is recognized to be a normal or non-cancer tissue is used in practice.

If the starter somatic cell is a cancer cell from a carcinogenic mammal, a normal or a non-cancer cell in the same individual as the carcinogenic mammal can be used as the aforementioned reference somatic cell isolated from a mammal. In particular, if a cell isolated from the same organ in the same individual is used, the difference in malignancy between the two cells (i.e., the starter somatic cells and the refernece somatic cells) is distinct because of the commonality of the features that are unique to the individual or organ. Hence, the above-described step of making comparison with the tissue of the same individual as the one from which the starter somatic cell has been isolated does more than identifying the malignancy or specific marker of the induced cancer stem cell; it also serves as a useful analysis tool that may be applied to identify carcinogenic mechanisms and its utility even covers use as a method of screening for a target in drug discovery (for details, see below.)

As already noted, it is difficult to isolate only a single cancer cell from a tissue, so a cell group in a cancer tissue or a non-cancer tissue in a carcinogenic mammal is used in practice.

In addition, the mammal from which the reference somatic cell is to be isolated may be the same as the mammal from which the starter somatic cell has been isolated, and a human is particularly preferred.

In addition, the induced mesendodermal stem cell and the induced endodermal stem cell as induced from the reference somatic cell isolated from a mammal are not particularly limited if they have been induced from the reference somatic cell isolated from a mammal, but it should be noted that those which are obtained by the same method of induction as employed to give rise to the induced cancer stem cell of the present invention are preferably used.

In addition, the induced pluripotent stem cell as induced from the referene somatic cell isolated from a mammal is not particularly limited if it has been prepared by known methods of giving rise to induced pluripotent stem cells, but those which are obtained by the same method of induction as employed to give rise to the induced cancer stem cell of the present invention are preferably used. Other examples that can be used include: the induced pluripotent stem cells that are described in Patent Documents 1 and 2, as well as in "Methods of establishing human iPS cells", Center for iPS Cell Research and Application, Institute for Integrated Cell-Material Sciences, Kyoto University, CiRA/M&M, p. 1-14,2008, 7.4; induced pluripotent stem cells that are available from known supply sources such as RIKEN BioResource Center and Kyoto University; and known gene expression data for induced pluripotent stem cells that are available from the aforementioned Gene expression Omnibus [GEO].

Further in addition, embryonic stem cells can also be used as the reference for comparison and any such cells that have been prepared by known methods can be used. It is also possible to use undifferentiated embryonic stem cells obtained by the methods descried in Thomson JA et al., "Embryonic stem cell lines derived from human blastocysts", Science, 1998 Nov 6, 282 (5391): 1145-7, Erratum in Science, 1998 Dec 4, 282 (5395): 1827 and Hirofumi Suemori et al., "Efficient establishment of human embryonic stem cell lines and long term maintenance with stable karyotype by enzymatic bulk passage", Biochemical and Biophysical Research Communications, 345, 926-32 (2006)); undifferentiated embryonic stem cells available from known supply sources such as RIKEN BioResource Center and Institute for Frontier Medical Sciences, Kyoto University; and known gene expression data such as hES_H9 (GSM194390), hES_BG03 (GSM194391), and hES_ES01 (GSM194392). These gene expression data are available from the aforementioned Gene expression Omnibus [GEO].

The induced cancer stem cell of the present invention is selected as such if (a) a mutation is verified and identified in a tumor suppressor gene. It suffices for the purposes of the present invention that (a) a mutation is verified in a tumor suppressor gene and there is no need to perform analysis for the entire genome.

The induced cancer stem cell of the present invention can also be selected as such if (b) an increased expression of a cancer-related gene is verified and identified in comparison with the reference cell.

Transcriptome analysis refers to analysis of all mRNAs (or the (primary) transcripts) that are found in a single organism cell or proliferated, similarly differentiated cells of organism under given cell upon biological conditions. Since mRNA changes variously on account of accumulating extracellular effects that occur in the process of development, analysis of the transctiptome makes it possibel to determine the properties of the current cell in details. Specifically, analysis is performed using microarrays and the like.

For example, the induced cancer stem cell of the present invention can be selected as such if mRNA corresponding to (a) a mutated tumor suppressor gene or mRNA corresponding to (b) a cancer-related gene is found in said cell in greater amounts than in the reference cell.

In one preferred embodiment of the present invention, transcriptome analysis (on microarrays) is performed to measure (b) an increased expression of a cancer-related gene, for example, an increased expression of at least one cancer-related gene as selected from the groups of genes that consist of a group of genes related to angiogenesis, a group of cancer-related pathway genes, a group of genes related to stromal barrier, a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer; based on the result of this measurement, a specific marker can be identified to select the cell of interest. In addition to these genes, at least one other gene selected from the groups of genes that consist of a group of genes related to stress and toxicity, a group of genes for epigenetics of chromatin modifying enzyme, a group of genes for stem cell transcription factor, and a group of hepatocyte specific genes may be subjected to a measurement for determining if it has undergone an increased expression; the increased expressions of such at least two genes are preferably measured to effect overall rating.

### Methods of screening using the induced cancer stem cell

In its third embodiment, the present invention provides a method of screening characterized by using the induced cancer stem cell according to its first embodiment, and it is advantageously used as a method of screening for a target of anti-cancer drug discovery, a method of screening for an anti-cancer therapeutic drug, or as a method of screening for a cancer diagnostic drug.

The screening method of the present invention preferably involves a step of contacting both the induced cancer stem cell of the present invention and the reference cell such as an induced mesendodermal stem cell, an induced endodermal stem cell or an induced pluripotent stem cell as induced from the somatic cell isolated from a mammal, or an embryonic stem cell with the test substance.

In the case where this method is used to screen for a target of anti-cancer drug discovery, a gene or protein that is a potential target of anti-cancer drug discovery can be searched for by comparing the induced cancer stem cell according to the first embodiment of the present invention with an induced mesendodermal stem cell, an induced endodermal stem cell or an induced pluripotent stem cell as induced from the reference somatic cell isolated from a mammal, or an embryonic stem cell.

If, as the result of the search, antisense RNA and siRNA that suppress the expression of a certain gene to be a putative target in drug discovery or specific inhibitors of proteins (e.g. enzymes) translated from this gene are added to a culture dish on which the induced cancer stem cell of the presnt invention has been cultured and thereafter the properties and the like of the cell are examined to determine if the gene can be used as a target of drug discovery.

In the case where this method is used to screen for an anti-cancer therapeutic drug, a medicine that is a candidate for an anti-cancer agent or vaccine (e.g. anti-cancer vaccine) is added to a Culture dish on which the induced cancer stem cell of the presnt invention has been cultured and thereafter the properties and the like of the cell are evaluated to determine the efficacy of the medicine.

In the case where this method is used to screen for a cancer diagnostic drug, it is possible to evaluate as to whether a possible cancer diagnostic drug is effective as the cancer diagnostic drug by adding various types of the induced cancer stem cell of the presnt invention to the possible cancer diagnostic drug and by checking to see if they are accurately diagnosed as cancerous.

### Method of preparing an anti-cancer vaccine using the induced cancer stem cell

In its fourth embodiment, the present invention provides a method of preparing an anti-cancer vaccine using the induced cancer stem cell according to its first embodiment.

More specifically, anti-cancer vaccines useful in CTL therapy, dendritic cell therapy, cancer peptide vaccine therapy, and other therapies can be prepared by using the induced cancer stem cell according to the first embodiment of the present invention.

CTL (cytotoxic T-lymphocyte) therapy is a therapeutic method in which lymphocytes isolated from a patient are activated through their artificial learning the features of the cancer to be attacked and then a large amount of the cytotoxic T lymphocytes (CTL cells) are returned into the body of the patient.

In CTL therapy, learning by lymphocytes is generally achieved by using the antigen of cancer cells present in the patient or by using an artificial antigen. Using the antigen of cancer cells present in the patient is considered to have the greater efficacy. However, the need for isolating cancer cells exerts a great physical burden on the patient and, what is more, the isolated cancer cells need to be preliminarily proliferated to an adequate number *ex vivo,* but then they are difficult to culture; hence, this method is only applicable to the case where a relatively large tumor has been extracted by surgery and the antigen isolated successfully.

The induced cancer stem cell of the present invention is capable of self-renewal *in vitro,* so induced cancer stem cells can be made available in the required amount and, in addition, the physical burden to be exerted on the cancer patient by the process of isolating cancer cells can be sufficiently reduced to provide significant utility.

In a more specific production process, T cells capable of attacking cancer cells are extracted from a patient's blood as by component blood sampling to which the induced cancer stem cells of the present invention, a lysate of these cells, as well as a cancer antigen protein or peptide obtained on the basis of these cells are added, so that they will learn the cancer antigen. Subsequently, the T cells are activated by an anti-CD3 antibody or the like and then cultured in the presence of interleukin 2 or the like to prepare a large amount of cytotoxic T lymphocytes which can serve as an anti-cancer vaccine. In the case of using induced cancer stem cells or a lysate of these cells as a cancer antigen, a preferred source of supply for the induced cancer stem cells is a cancer tissue extracted by surgery from the patient to be treated or cancer cells isolated from the ascites or the like of the patient.

Dendritic cell therapy is a therapeutic method in which dendritic cells isolated from the patient are caused to learn the features of the cancer to be attacked and are then returned into the body of the patient; the dendritic cells returned into the patient's body stimulate the T lymphocytes so that they become killer T cells which in turn attack the cancer cells for cancer treatment.

This therapeutic method has the same problem as the aforementioned CTL therapy in that it is only applicable to the case where a relatively large tumor has been extracted by surgery and the antigen isolated successfully. In contrast, the induced cancer stem cell of the present invention is capable of self-renewal *in vitro,* so induced cancer stem cells can be made available in the required amount and, in addition, the physical burden to be exerted on the cancer patient by the process of isolating cancer cells can be sufficiently reduced to provide significant utility.

In a more specific production process, dendritic cells are extracted as by component blood sampling to which the induced cancer stem cells of the present invention, a lysate of these cells, as well as a cancer antigen protein or peptide obtained on the basis of these cells are added, so that they will learn the cancer antigen to become an anti-cancer vaccine. In the case of using induced cancer stem cells or a lysate of these cells as a cancer antigen, a preferred source of supply for the induced cancer stem cells is a cancer tissue extracted by surgery from the patient to be treated or cancer cells isolated from the ascites or the like of the patient.

The aforementioned dendritic cells are such that even a single dendritic cell is capable of stimulating from several hundred to several thousdand lymphocytes, so the therapeutic method in which the dendritic cells are caused to learn the features of the target cancer and then returned into the body of the patient is believed to be extremely efficient. However, dendritic cells account for only about 0.1 to 0.5% of leucocytes in number, so instead of using them directly, monocytes that are abundant in the blood and which can change to dendritic cells are acquired in large quantities by a separated component blood sampling method and cultured in the presence of a cell stimulating substance such as cytokine to grow into dendritic cells for use in therapy.

Cancer peptide vaccine therapy is a therapeutic method in which a peptide (peptide vaccine) as a specific antigen possessed by cancer cells is injected into the patient so that the immunity of the patient is sufficiently enhanced to suppress the growth of the cancer. Specifically, when the peptide (a small one consisting of 9 or 10 amino acids) is administered into the body of the paitnet, killer T cells stimulated by the peptide are activated and further proliferated to attack the cancer cells; cancer peptide vaccine therapy uses this nature of the peptide to eliminate (regress) the cancer.

Since the induced cancer stem cell of the present invention is capable of self-renewal *in vitro* and enables various types of induced cancer stem cells to be amplified in large quantities, the induced cancer stem cell of the present invention as prepared from cancer tissues or the like that are derived from patients with various types of cancer can be cultured in large quantities to prepare the desired anti-cancer vaccines. The thus obtained anti-cancer vaccines can also be used in CTL therapy or dendritic cell therapy.

The anti-cancer vaccines described above are extremely useful in preventive cancer therapy or for preventing possible recurrence after the application of standard therapies including chemotherapy, radiation therapy and surgical therapy.

### Method of preparing a cancer model animal using the induced cancer stem cell

In its fifth embodiment, the present invention provides a method of preparing a cancer model animal using the induced cancer stem cell according to its first embodiment.

According to this method of preparing a cancer model animal, the induced cancer stem cell according to the first embodiment of the present invention may be transplanted to a laboratory animal such as mouse to thereby prepare tumor bearing mice, which are then administered with an anti-cancer agent, an antibody, a vaccine and the like; their pharmacological efficacy can be verified by subjecting the tumor bearing animals to a blood test, a urine test, autopsy, and the like.

The induced cancer stem cell of the present invention finds various other applications than in the aforementioned methods of screening, methods of preparing anti-cancer vaccines, and methods of preparing cancer model animals.

For example, secretory proteins and membrane proteins are screened exhaustively from the genetic information about induced cancer stem cells and those secretory proteins and membrane proteins that are specific for the induced cancer stem cell of the present invention and which hence are useful as cancer diagnostic markers are identified to prepare therapeutic or diagnostic antibodies. An exemplary method for exhaustive screening of secretory proteins and membrane proteins is the "signal sequence trapping method" (JP Patent Nos. 3229590 and 3499528) which is characterized by gene identification targeted to a signal sequence that is common to the secretory proteins and membrane proteins.

On the pages that follow, the present invention is described more specifically by means of Examples but it should be understood that the scope of the present invention is by no means limited by those Examples.

### Example 1 Preparation of retroviral vectors

Three retroviral vector plasmids for the three genes, POU5F1-pMXs, KLF4-pMXs, and SOX2-pMXs, were introduced into packaging cells for preparing a pantropic retroviral vector, namely Plat-GP cells, using Fugene HD (Roche; Cat No. 4709691) to thereby prepare a retroviral vector solution. The gene vector plasmids POU5F1-pMXs, KLF4-pMXs, and SOX2-pMXs were used at a ratio of 4:2:1 in that order so as to enture that the relation of POU5F1 > SOX2 was achieved. The details of the procedure are as described below.

### <Preparation of a retroviral vector solution for transducing the genes into cells derived from stomach cancer patient's cancer tissues>

The vectors POU5F1-pMXs, KLF4-pMXs, and SOX2-pMXs were constructed vectors (Table 33 below).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs (Cell Biolab), and 45 µL of FuGENE HD.

### <Preparation of a retroviral vector solution for transducing the genes into cells derived from stomach cancer patient's non-cancer tissues>

The vectors POU5F1-pMXs, KLF4-pMXs, and SOX2-pMXs were constructed vectors (Table 33 below).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs, and 45 µL of FuGENE HD.

### <Preparation of a solution containing retroviral vectors for transducing the genes into cells derived from colon cancer patient's cancer tissues>

The vectors POU5F1-pMXs, KLF4-pMXs, and SOX2-pMXs were constructed vectors (Table 33 below).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs, and 45 µL of FuGENE HD.

The Plat-GP cells into which the retroviral vector plasmids had been transduced were cultured for at least 48 hours; thereafter, the supernatant was harvested three times every 24 hours and stored at 4°C, and filtration was performed using the Steriflip-HV Filter unit (pore size 0.45 µm filter; Millipore; Cat No. SE1M003M00). The above-noted procedure yielded a pantropic retroviral vector solution containing the three genes (POU5F1, KLF4, and SOX2 at a ratio of 4:2:1 in that order). The pantropic retroviral vector, which enables gene transfer into various cells, can efficiently transduce the genes into human cells as well.

**[Table 33]**

| **Details of constructed retroviral vector plasmids** | | | | | | |
|---|---|---|---|---|---|---|
| Gene | NCBI No. | Vector | 5' restriction enzyme | 3' restriction enzyme | Clone ID | Supplier |
| Human OCT3/4 | BC117435 | pMXs | EcoRI | EcoRI | 40125986 | Open Biosystems |
| Human KLF4 | BC029923 | pMXs | EcoRI | EcoRI | 5111134 | Open Biosystems |
| Human SOX2 | BC013923 | pMXs | EcoRI | XhoI | 2823424 | Open Biosystems |

### Example 2 Preparation of induced malignant stem cells from cells derived from cancer tissues of a stomach cancer patient

Somatic cells were isolated from fresh cancer tissues of a patient with (progressive) stomach cancer, which had been stored for several hours and transported in a preservation solution. To the resultant cells derived from the cancer tissues of the stomach cancer patient, the retroviral vector solution containing the three retroviral vectors of the three genes (POU5F1, KLF4, and SOX2 at a ratio of 4:2:1 in that order), which had been prepared in Example 1 so as to ensure that the relation of POU5F1 > KLF4 > SOX2 was achieved, was added for gene transfer, whereby human induced malignant stem cells were prepared. The details of the procedure are as described below.

Part of fresh stomach cancer tissues obtained during operation (from a 67-year-old Japanese male patient with progressive cancer) was washed with Hank's balanced salt solution (Phenol Red-free) (Invitrogen; Cat No. 14175-095) and minced with scissors into pieces of about 0.1-1 mm². The pieces were further washed with Hank's balanced salt solution (Phenol Red-free) until a supernatant became clear. Then, after removal of the supernatant, 5 mL of a mixture of 0.1% collagenase (Wako Pure Chemical; Cat No. 034-10533) and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the mixture was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the mixture was centrifuged again at 1000 rpm at 4°C for 5 minutes. Then, after removal of the supernatant, 5 mL of a D-MEM (high glucose) medium supplemented with 1X antibiotic/antimycotic and 10% FBS was added, and the mixture was seeded on a collagen-coated dish (60 mm) (Iwaki; Cat No. 11-018-004) to subject it to primary culture.

After 24 hours, the medium was removed, 5 mL of a retroviral vector solution containing the three genes was added, and infection was allowed to proceed at 37°C for one day. The viral supernatant was removed, and mitomycin treated mouse embryonic fibroblasts as feeder cells were suspended in 5 mL of a D-MEM (high glucose) medium supplemented with 1X antibiotic/antimycotic and 10% FBS, and the cells in the suspension were then seeded at a density of 5.0×10⁴ cells/cm² on a collagen-coated dish (60 mm) (Iwaki; Cat No. 11-018-004) on which the transduced cells derived from the cancer tissues of the stomach cancer patient had been cultured, whereby co-culture was performed.

Thereafter, the medium was repeatedly replaced with a MEF conditioned ES medium every three days, and from 15 days after the gene transfer, the medium was replaced everyday with maintenance medium for a feeder cell-free culture of human ES/iPS cells, mTeSR1.

The MEF conditioned ES medium and its preparation procedure which were used in Examples are as described below.

### <MEF conditioned ES medium>

### MEF

Mitomycin C-treated primary mouse embryonic fibroblasts (DS Pharma Biomedical; Cat No. R-PMEF-CF)
ES medium for MEF conditioning
Knockout D-MEM (Invitrogen; Cat No. 10829-018), 500 mL
2 mM GlutaMAX
10% knockout serum replacement (Invitrogen; Cat No. 10828-028)
50 µg/mL gentamicin (Invitrogen; Cat No. 15750-060)
MEM non-essential amino acid solution (Invitrogen; Cat No. 11140-050)
10 ng/mL bFGF (PeproTech; Cat No. 100-18B)

### <Preparation of a MEF conditioned ES medium>

First, 5×10⁶ mitomycin-treated mouse embryonic fibroblasts (DS Pharma Biomedical; Cat No. R-PMEF-CF) were suspended in 40 mL of a D-MEM (high glucose) medium supplemented with 1X antibiotic/antimycotic and 10% FBS, and the cells in the suspension were then seeded on four gelatin-coated 100 mm dishes (Iwaki; Cat No. 11-020-006). After 24 hours, the medium was removed and 10 mL of an ES medium for MEF conditioning was added.

To the supernatant harvested every 24 hours, 10% knockout serum replacement, 10 ng/mL bFGF, and 0.1 mM 2-mercaptoethanol were newly added, which was used as a MEF conditioned ES medium.

### [In vitro self-renewal culture of the human induced malignant stem cells derived from the cancer tissues of the stomach cancer patient]

One clone of an induced malignant stem cell colony was picked up after 25 days after the three-gene transduction (GC1-1), one clone of an induced malignant stem cell colony was picked up after 32 days after the three-gene transduction (GC1-3), and three three clones of an induced malignant stem cell colony were respectively picked up (GC1-5, -7 and -8). These induced malignant stem cell colonies were transferred onto mitomycin treated feeder cells in a gelatin-coated 24-well plate. The feeder cells, which were mitomycin-treated mouse embryonic fibroblasts, had been seeded at a density of 5.0×10⁴ cells/cm² on a gelatin-coated 24-well plate the day before the pickup of the induced malignant stem cells.

In the case of GC1-1, 32 days after the gene transfer, the human induced malignant stem cells (passage 1; p1) grown on the 24-well plate were subjected to passage culture onto a 6-well plate (p2); 43days after the gene transfer, the human induced malignant stem cells (p2) grown on a 6-well plate were subjected to passage culture onto a 10 cm culture dish (p3); 50 days after the gene transfer, a part of the human induced malignant stem cells (p3) grown on the 10 cm culture dish was subjected to passage culture onto another 10 cm culture dish (p4) and the reminder was cryopreserved; 55 days after the gene transfer, a part of the human induced malignant stem cells (p4) grown on the 10 cm culture dish were subjected to passage culture onto still another 10 cm culture dish (p5) and the reminder was cryopreserved; and 58 days after the gene transfer, the human induced malignant stem cells (p5) grown on the 10 cm culture dish were lysed in Buffer RLT (cell lysis solution before RNA purification) The same procedure as for GC1-1 was repeated with other clones, and the passage numbers (p), and the days (number of days after the gene transfer) when they were subjected to passage culture (p) and lysed in a buffer for an RNA collection kit (Buffer RLT) are summarized below.

In Examples of this application, cell cryopreservation was performed by the following procedure.

After removing the medium from the cultured cells, and washing the cells with 10 mL of PBS (-)/10 cm (about 60 cm²) culture dish, 2-3 mL of a cell dissociation solution was added to the 10 cm (about 60 cm²) culture dish. The following two types of cell dissociation solutions were used for passage culture in Examples:
(i) 0.25% trypsin/1 mM EDTA solution (Invitrogen; Cat No. 25200-056), and
(ii) Prepared cell dissociation solution [mixture of 10 mL of 10 mg/mL type IV Collagenase (Invitrogen; Cat No. 17104-019), 1 mL of a 100 mM calcium chloride solution (Sigma), 59 mL of PBS, 10 mL of a 2.5% trypsin solution (Invitrogen; Cat No. 15090-046), and 20 mL of knockout serum replacement (KSR; Invitrogen; Cat No. 10828-028), which was sterialized by passing through a 0.22 µm filter].

After placing at 37°C for 5 minutes, the cell dissociation solution was removed, 20 mL of a D-MEM (high glucose) medium supplemented with 1X antibiotic/antimycotic and 10% FBS was added, and the suspension was then centrifuged at 1000 rpm at 4°C for 5 minutes. Next, after removal of the supernatant, 1 mL of a cryopreservation solution was added, and the suspension was dispensed into two serum tubes. Thereafter, the serum tubes were placed into an animal cell cryopreservation container (BICELL), freezed to -80°C overnight, and then stored in liquid nitrogen.

The following two types of cryopreservation solutions were used:
(i) CELLBANKER 3 (Nippon Zenyaku Kogyo; Cat No. BLC-3S), and
(ii) Mixture of 50% mTeSR1, 40% KSR, and 10% DMSO.

### GC1-1

32 days after the genetic transfection (Day 32): 24-well plate (passage 1 (p1)) → 6-well plate (p2)
Day 43: 6-well plate (p2) → 10 cm dish (p3)
Day 50: Passage and cryopreservation (p4)
Day 55: Passage and cryopreservation (p5)
Day 58: Treatment with Buffer RLT (cell lysis solution before RNA purification)

### GC1-3

Day 44: 24-well plate (p1) → 6-well plate (p2)
Day 48: 6-well plate (p2) → 10 cm dish (p3)
Day 53: Passage and cryopreservation (p4)
Day 58: Treatment with Buffer RLT (cell lysis solution before RNA purification)

### GC1-5

Day 44: 24-well plate (p1) → 6-well plate (p2)
Day 52: 6-well plate (p2) → 10 cm dish (p3)
Day 61: Passage and cryopreservation (p4)
Day 63: Cryopreservation and treatment with Buffer RLT (cell lysis solution before RNA purification)

### GC1-7

Day 44: 24-well plate (p1) → 6-well plate (p2)
Day 52: 6-well plate (p2) → 10 cm dish (p3)
Day 61: Passage and cryopreservation (p4)
Day 62: Cryopreservation and treatment with Buffer RLT (cell lysis solution before RNA purification)

### GC1-8

Day 44: 24-well plate (p1) → 6-well plate (p2)
Day 48: 6-well plate (p2) → 10 cm dish (p3)
Day 53: Passage and cryopreservation (p4)
Day 55: Passage and cryopreservation (p5)
Day 58: Treatment with Buffer RLT (cell lysis solution before RNA purification)

As described above, the induced malignant stem cells derived from the cancer tissues of the stomach cancer patient were subjected to *in vitro* self-renewal using the feeder-free medium for human ES/iPS cells (mTeSR1) together with the feeder cells (MEFs).

### Example 3 Preparation of human induced malignant stem cells from cells derived from non-cancer tissues of a stomach cancer patient

Cells were isolated from fresh non-cancer tissues of a patient with (progressive) stomach cancer, which had been stored for several hours and transported in a preservation solution, and were subjected to primary culture. To the resultant cells derived from the non-cancer tissues of the stomach cancer patient, the retroviral vector solution containing the three retroviral vectors of the three genes (POU5F1, KLF4, and SOX2 at a ratio of 4:2:1 in that order), which had been prepared in Example 1, was added for gene transfer, whereby human induced malignant stem cells were prepared. The details of the procedure are as described below.

Part of fresh non-cancer tissues obtained during operation (from a 67-year-old Japanese male patient with progressive stomach cancer) was washed with Hank's balanced salt solution (Phenol Red-free) and minced with scissors into pieces of about 0.1-1 mm². The pieces were further washed with Hank's balanced salt solution (Phenol Red-free) until a supernatant became clear. Then, after removal of the supernatant, 5 mL of a mixture of 0.1% collagenase and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the mixture was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the mixture was centrifuged again at 1000 rpm at 4°C for 5 minutes. Then, after removal of the supernatant, 10 mL of a D-MEM (high glucose) medium supplemented with 1X antibiotic/antimycotic and 10% FBS was added, and the mixture was seeded on a collagen-coated dish (100 mm) (Iwaki; Cat No. 11-018-006).

After 24 hours, the medium was removed, 10 mL of a retroviral vector solution containing the three genes (POU5F1, KLF4, and SOX2 at a ratio of 4:2:1 in that order) was added, and the mixture was infected at 37°C for 24 hours. The viral supernatant was removed, and mitomycin treated mouse embryonic fibroblasts were suspended in 10 mL of a D-MEM (high glucose) medium supplemented with 1X antibiotic/antimycotic and 10% FBS, and the cells in the suspension were then seeded at a density of 5.0×10⁴ cells/cm² on a collagen-coated dish (100 mm) (Iwaki; Cat No. 11-018-006) on which the transduced cells derived from the non-cancer tissues of the stomach cancer patient had been cultured, whereby co-culture was performed.

### [In vitro self-renewal culture of the human induced malignant stem cells derived from the non-cancer tissues of the stomach cancer patient]

Thereafter, the medium was repeatedly replaced with a MEF conditioned ES medium every three days, and from 31 days after the three-gene transduction, the medium was replaced everyday with mTeSR1. Fourty-one days after the gene transfer, one clone (NGC1-1) of a colony was picked up and subjected to passage culture onto mitomycin treated mouse embryonic fibroblasts in a gelatin-coated 24-well plate. The feeder cells, which were mitomycin-treated mouse embryonic fibroblasts, had been seeded at a density of 5.0×10⁴ cells/cm² on a gelatin-coated 24-well plate the day before the pickup of the induced malignant stem cells.

Listed below are the days (number of days after the gene transfer) when the human induced malignant stem cells derived from the non-cancer tissues of the stomach cancer patient were subjected to passage culture (p) and lysed in a buffer for an RNA collection kit (Buffer RLT).

### NGC1-1

Day 52: 24-well plate (p1) → 6-well plate (p2)
Day 58: 6-well plate (p2) → 10 cm dish (p3)
Day 65: Passage, cryopreservation and treatment with Buffer RLT (cell lysis solution before RNA purification)

As described above, the human induced malignant stem cells derived from the non-cancer tissues of the stomach cancer patient were self-renewed *in vitro* using the feeder-free medium for human ES/iPS cells (mTeSR1) together with the feeder cells (MEFs).

### Example 4 Preparation of human induced malignant stem cells from cells derived from cancer tissues of a colon cancer patient

Cells were isolated from fresh cancer tissues of a patient with human colon cancer, which had been stored for several hours and transported in a preservation solution. To the resultant cells derived from the fresh cancer tissues of the human colon cancer patient, the retroviral vector solution containing the three retroviral vectors of the three genes (POU5F1, KLF4, and SOX2 at a ratio of 4:2:1 in that order), which had been prepared in Example 1, was added for gene transfer, whereby human induced malignant stem cells were prepared. The details of the procedure are as described below.

Part of colon cancer tissues obtained during operation (from a 55-year-old Japanese male patient with sigmoid colon cancer) was washed with Hank's balanced salt solution (Phenol Red-free) and minced with scissors into pieces of about 0.1-1 mm². The pieces were further washed with Hank's balanced salt solution (Phenol Red-free) until a supernatant became clear. Then, after removal of the supernatant, 5 mL of a mixture of 0.1% collagenase and 1X antibiotic/antimycotic was added to the tissue precipitate, and stirring was performed at 37°C for 60 minutes with a shaker.

After confirming that the precipitated tissue has been fully digested, 35 mL of a D-MEM (high glucose) medium supplemented with 1X antibiotic/antimycotic and 10% FBS was added, and the mixture was then centrifuged at 1000 rpm at 4°C for 5 minutes. Next, after removal of the supernatant, 40 mL of a D-MEM (high glucose) medium supplemented with 1X antibiotic/antimycotic and 10% FBS was added, and the mixture was centrifuged again at 1000 rpm at 4°C for 5 minutes. Then, after removal of the supernatant, 10 mL of a D-MEM (high glucose) medium supplemented with 1X antibiotic/antimycotic and 10% FBS was added, and the mixture was seeded on a collagen-coated dish (100 mm) (Iwaki; Cat No. 11-018-006).

After 24 hours, the medium was removed and 10 mL of a retroviral vector solution containing the three retroviral vectors of the three genes was added, and after 5 hours, 5 mL of a Luc-IRES-GFP retroviral vector was infected at 37°C for about 24 hours. The viral supernatant was removed, and mitomycin treated MEFs were suspended in 10 mL of a D-MEM (high glucose) medium supplemented with 1X antibiotic/antimycotic and 10% FBS, and the cells in the suspension were then seeded at a density of 5.0×10⁴ cells/cm² on a collagen-coated dish (100 mm) (Iwaki; Cat No. 11-018-006) on which the transduced cells derived from the cancer tissues of the colon cancer patient had been cultured, whereby co-culture was performed.

### [In vitro self-renewal culture of the human induced malignant stem cells derived from the cancer tissues of the colon cancer patient]

Thereafter, the medium was repeatedly replaced with a MEF conditioned ES medium every three days, and from 22 days after the gene transfer, the medium was replaced everyday with mTeSR1. Thirty-one days after the gene transfer, one clone (CC1-10) of a colony was picked up and subjected to passage culture onto mitomycin treated mouse embryonic fibroblasts in a gelatin-coated 24-well plate. The feeder cells, which were mitomycin-treated mouse embryonic fibroblasts, had been seeded at a density of 5.0×10⁴ cells/cm² on a gelatin-coated 24-well plate the day before the pickup of the induced malignant stem cells.

Listed below are the days (number of days after the gene transfer) when the human induced malignant stem cells derived from the cancer tissues of the colon cancer patient were subjected to passage culture (p) and lysed in a buffer for an RNA collection kit (Buffer RLT).

### CC1-10

Day 49: 24-well plate (p1) → 6-well plate (p2)
Day 54: 6-well plate (p2) → 10 cm dish (p3)
Day 59: Passage and cryopreservation (p4)
Day 63: Passage and cryopreservation (p5)
Day 68: Partial treatment with Buffer RLT (cell lysis solution before RNA purification)
Day 71: Partial treatment with Qiazol (cell lysis solution before RNA purification)
Day 75: Partial transplantation into NOD-SCID mice

As described above, the induced malignant stem cells derived from the cancer tissues of the colon cancer patient were self-renewed *in vitro* using the feeder-free medium for human ES/iPS cells (mTeSR1) together with the feeder cells (MEFs).

### Example 5 Microarray-based quantitative analysis

Genome-wide genetic expression (mRNA transcriptome) was analyzed using the Whole Human Genome Oligo DNA Microarray (4X44K) manufactured by Agilent Technologies. The microarray data for three human embryonic stem cells (i.e., hES_H9 (GSM194390), hES_BG03 (GSM194391) and hES_ES01 (GSM194392)) and for induced pluripotent stem cells (i.e., hiPS-201B7 (GSM241846)) to be used was downloaded from GEO.

### <Preparation of total RNAs and genomic DNAs>

The human induced malignant stem cells (GC1-1, GC1-3, GC1-5, GC1-7, GC1-8, NGC1-1, and CC1-10) prepared in Examples 2-4 were treated with Buffer RLT (cell lysis solution before RNA purification) to extract the total RNAs and genomic DNAs of the induced human malignant stem cells from the solution using the AllPrep DNA/RNA Mini Kit (50) (Qiagen; Cat No.80204).

### <Testing procedure>

### (i) Quality check of genomic DNAs

The DNA concentrations and purities were assessed using the NanoDrop ND-1000 (NanoDrop Technologies), and as a result, every sample was verified to have adequate concentration and high purity.

### (ii) Quality check of total RNAs

The total RNAs were checked for their quality on the Agilent 2100 Bioanalyzer (Agilent Technologies) using the RNA LabChip (registered trademark of Agilent Technologies) Kit, and all of the RNA samples were found to be of good quality. The RNA concentrations and purities were also assessed using the NanoDrop ND-1000 (NanoDrop Technologies), and as a result, every sample was verified to contain the total RNA in an amount required for cRNA synthesis and at a high level of purity.

### (iii) cRNA synthesis

According to the Agilent's protocol, double-stranded cRNA was synthesized from the total RNA (500 ng) of each sample using the Quick Amp Labeling kit (Agilent Technologies). From the prepared cDNA, cRNA was synthesized by *in vitro* transcription. During the synthesis, the cRNA was fluorescence-labeled by incorporating Cyanine-labeled CTP (Cyanine 3-CTP).

### (iv) Hybridization

With the use of the Gene Expression Hybridization Kit (Agilent Technologies), the hybridization labeled cRNA was added to a hybridization buffer to perform hybridization for 17 hours on the Whole Human Genome Oligo DNA Microarray (4X44K) manufactured by Agilent Technologies. After washing, DNA microarray images were scanned with an Agilent microarray scanner, and the fluorescent signals at each spot were converted to numerical values using the Feature Extraction Software (v.9.5.3.1).

### <Results of quantitative genetic analysis>

The analysis software used was GeneSpring GX 10.0 (Agilent Technologies, Inc.) and normalization was performed using the 50th percentile method.

The total genetic expression distributions (distribution of fluorescence values for respective probes) were presented with the median value being taken as 0. A probe that showed a value of more than 0 was regarded as a probe that detected the genetic expression, and the genetic expression was considered present. According to the analysis results, the human induced malignant stem cells (GC1-1, GC1-3, GC1-5, GC1-7, GC1-8, NGC1-1, and CC1-10) increased in the expression of the endodermal genes (GSC, and GATA4, FOXA2 or SOX17) as compared with the human induced pluripotent stem cells (hiPS201B7). In particular, the human induced malignant stem cells (GC1-2, GC1-5, GC1-7 and NGC1-1) increased by twice or more in the expression of the endodermal genes (GSC, and GATA4, FOXA2 or SOX17) as compared with the human induced pluripotent stem cells (hiPS201B7) and the human embryonic stem cells.

### 1) Genes Related to Angiogenesis

Among the probes for the genes related to angiogenesis contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, GenBank accession number, and probe name in Table 34 [hES_BG03 vs GC1-5] below. Further, the probes for the genes related to angiogenesis whose expressions increased at least twice are plotted in the figure given below (Fig. 1). It was shown that GC1-5, which are human induced malignant stem cells derived from the primary cultured somatic (non-embryonic) cells prepared from the cancer patient's fresh (stomach) cancer tissues, relatively highly expressed the genes related to angiogenesis (MDK, TIMP2, FGFR3, PLAU, and ID3) which are endogenous cancer-related genes.

**[Table 34]**

| GeneSymbol | GenbankAccession | ProbeName |
|---|---|---|
| MMP2 | NM_004530 | A_23_P163787 |
| ITGAV | NM_002210 | A_23_P50907 |
| PLAU | NM_002658 | A_23_P24104 |
| MMP9 | NM_004994 | A_23_P40174 |
| AKT1 | NM_005163 | A_23_P2960 |
| EFNA1 | NM_004428 | A_23_P113005 |
| TGFB1 | NM_000660 | A_24_P79054 |
| THBS1 | NM_003246 | A_23_P206212 |
| COL18A1 | NM_030582 | A_24_P57426 |
| SPHK1 | NM_021972 | A_23_P38106 |
| VEGFA | MM_001025366 | A_23_P81805 |
| TEK | NM_000459 | A_23_P374695 |
| MDK | NM_001012334 | A_23_P116235 |
| CCL2 | NM_002982 | A_23_P89431 |
| HAND2 | NM_021973 | A_23_P373521 |
| ANGPTL4 | NM_139314 | A_23_P159325 |
| FGFR3 | MM_000142 | A_23_P212830 |
| ANGPT12 | NM_001147 | A_23_P60079 |

| | | |
|---|---|---|
| FGF1 | NM_000800 | A_24_P111106 |
| ANPEP | NM_001150 | A_23_P88626 |
| EFNA1 | MM_004428 | A_23_P254512 |
| NRP1 | MM_003873 | A_24_P135322 |
| TIMP3 | NM_000362 | A_23_P399078 |
| NRP2 | NM_201266 | A_23_P209669 |
| PGF | NM_002632 | A_23_P76992 |
| ID3 | NM_002167 | A_23_P137381 |
| NRP2 | NM_201266 | A_23_P393727 |
| SERPINF1 | MM_002615 | A_23_P100860 |
| VEGFC | NM_005429 | A_23_P167096 |
| CXCL1 | NM_001511 | A_23_P7144 |
| TIMP2 | NM_003255 | A_23_P107401 |
| EFNB2 | NM_004093 | A_24_P355944 |
| TGFB2 | | A_24_P148261 |
| TNFAIP2 | NM_006291 | A_23_P421423 |
| ANGPT1 | NM_001146 | A_23_P216023 |
| FGFR3 | MM_000142 | A_23_P500501 |
| TIMP1 | NM_003254 | A_23_P62115 |
| PF4 | NM_002619 | A_24_P79403 |
| JAG1 | NM_000214 | A_23_P210763 |
| NRP1 | NM_003873 | A_24_P928052 |
| FGF1 | NM_000800 | A_23_P213336 |

### 2) Cancer-Related Pathway Genes

Among the probes for the cancer-related pathway genes contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) and the human induced pluripotent stem cells hiPS-201B7 were listed for gene symbol, GenBank accession number, and probe name in Tables 35 [hES_H9 vs NGC1-1] and 36 [hiPS-201B7 vs NGC1-1] below, respectively. It was shown that NGC1-1, which are human induced malignant stem cells derived from the primary cultured somatic (non-embryonic) cells prepared from the cancer patient's fresh (stomach) non-cancer tissues, relatively highly expressed the endogenous cancer-related pathway genes (MMP2, TIMP1, TIMP3, MMP1, CDKN1A, and S100A4).

**[Table 35]**

| GeneSymbol | GenbankAccession | ProbeName |
|---|---|---|
| MMP2 | NM_004530 | A_23_P163787 |
| ITGAV | NM_002210 | A_23_P50907 |
| ITGB1 | MM_133376 | A_23_P104199 |
| PLAU | NM_002658 | A_23_P24104 |
| ITGA3 | NM_002204 | A_23_P55251 |
| AKT1 | NM_005163 | A_23_P2960 |
| SERPINE1 | NM_000602 | A_24_P158089 |
| CDKN2A | NM_058197 | A_23_P43490 |
| ITGA1 | NM_181501 | A_23_P256334 |
| CDKN2A | NM_058197 | A_23_P43484 |
| MMP1 | NM_002421 | A_23_P1691 |
| TNFRSF10B | NM_003842 | A_24_P218265 |
| TGFB1 | NM_000660 | A_24_P79054 |
| THBS1 | NM_003246 | A_23_P206212 |
| COL18A1 | MM_030582 | A_24_P57426 |
| BAX | MM_138765 | A_23_P346311 |

| | | |
|---|---|---|
| VEGFA | MM_001025366 | A_23_P81805 |
| TEK | NM_000459 | A_23_P374695 |
| MCAM | NM_006500 | A_24_P326660 |
| CDKN1A | NM_078467 | A_24_P89457 |
| ITGB1 | NM_133376 | A_23_P104193 |
| ANGPT2 | MM_001147 | A_23_P60079 |
| MCAM | MM_006500 | A_23_P162171 |
| TWIST1 | NM_000474 | A_23_P71067 |
| CDKN1A | NM_000389 | A_23_P59210 |
| S100A4 | NM_002961 | A_23_P94800 |
| BAX | NM_138763 | A_23_P346309 |
| TIMP3 | NM_000362 | A_23_P399078 |
| TNFRSF1A | NM_001065 | A_24_P364363 |
| PLAUR | NM_001005377 | A_23_P16469 |
| NME4 | MM_005009 | A_24_P210829 |
| TIMP1 | NM_003254 | A_23_P62115 |
| TNFRSF10B | MM_003842 | A_23_P119030 |
| ANGPT1 | BC029406 | A_23_P431900 |

**[Table 36]**

| GeneSymbol | GenbankAccession | ProbeName |
|---|---|---|
| MMP2 | NM_004530 | A_23_P163787 |
| ITGAV | NM_002210 | A 23_P50907 |
| ITGA3 | NM_002204 | A_23_P55251 |
| SERPINE1 | NM_000602 | A_24_P158089 |
| CDKN2A | NM_058197 | A_23_P43490 |
| ITGA1 | NM_181501 | A_23_P256334 |
| FOS | NM_005252 | A_23_P106194 |
| CDKN2A | MM_058197 | A_23_P43484 |
| MMP1 | MM_002421 | A_23_P1691 |
| TP53 | MM_000546 | A_23_P26810 |
| BAX | NM_138764 | A_23_P208706 |
| TGFB1 | NM_000660 | A_24_P79054 |
| THBS1 | NM_003246 | A_23_P206212 |
| CASP8 | NM_033356 | A_23_P209389 |
| BCL2 | M13995 | A_23_P208132 |
| TNFRSF1A | NM_001065 | A_23_P139722 |
| VEGFA | MM_001025366 | A_23_P81805 |
| PIK3R1 | NM_181523 | A_24_P29401 |
| ANGPT2 | NM_001147 | A_23_P60079 |
| CDKN1A | NM_000389 | A_23_P59210 |
| S100A4 | NM_002961 | A_23_P94800 |
| BAX | NM_138763 | A_23_P346309 |
| NFKBIA | NM_020529 | A_23_P106002 |
| MTA1 | NM_004689 | A_24_P241370 |
| TIMP3 | NM_000362 | A_23_P399078 |
| ANGPT1 | NM_001146 | A_23_P216023 |
| TIMP1 | NM_003254 | A_23_P62115 |
| ANGPT1 | BC029406 | A_23_P431900 |

### 3) Genes Related to Stromal Barrier (Extracellular Matrix and Adhesion Molecule)

Among the probes for the genes related to stromal barrier contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, GenBank accession number, and probe name in Table 37 [hES_BG03 vs GC1-5] below. It was shown that GC1-5, which are human induced malignant stem cells derived from the primary cultured somatic (non-embryonic) cells prepared from the cancer patient's fresh (stomach) cancer tissues, relatively highly expressed the genes related to endogenous stromal barrier (COL4A2, FN1, COL1A1, and TGFB1).

**[Table 37]**

| GeneSymbol | GenbankAccession | ProbeName | GeneSymbol | GenbankAccession | ProbeName |
|---|---|---|---|---|---|
| MMP2 | NM_004530 | A_23_P163787 | COL5A1 | NM_000093 | A_23_P83818 |
| LAMA1 | NM_005559 | A_24_P100613 | LAMA1 | NM_005559 | A_32_P313405 |
| CD44 | NM_000610 | A_23_P24870 | TIMP3 | NM_000362 | A_23_P399078 |
| ITGAV | NM_002210 | A_23_P50907 | COL6A1 | NM_001848 | A_32_P32254 |
| ITGB1 | NM_133376 | A_23_P104199 | COL14A1 | MM_021110 | A_23_P216361 |
| ITGA3 | NM_002204 | A_23_P55251 | COL5A1 | MM_000093 | A_23_P158593 |
| MMP9 | NM_004994 | A_23_P40174 | TGFBI | MM_000358 | A_23_P156327 |
| THBS3 | NM_007112 | A_23_P201047 | COL4A2 | NM_001846 | A_23_P205031 |
| COL1A1 | 274615 | A_23_P207520 | FN1 | NM_212482 | A_24_P85539 |
| ITGA1 | NM_181501 | A_23_P256334 | CTNND2 | NM_001332 | A_23_P110624 |
| ICAM1 | NM_000201 | A_23_P153320 | CTNNB1 | NM_001904 | A_23_P29499 |
| COL6A2 | NM_001849 | A_23_P211233 | ECM1 | NM_004425 | A_23_P160559 |
| MMP1 | NM_002421 | A_23_P1691 | TIMP2 | NM_003255 | A_23_P107401 |
| FN1 | NM_212482 | A_24_P119745 | CTNND1 | CR749275 | A_24_P881527 |
| SPARC | NM_003118 | A_23_P7642 | COL8A1 | NM_001850 | A_23_P69030 |
| THBS1 | NM_003246 | A_23_P206212 | FN1 | MM_054034 | A_24_P334130 |
| COL14A1 | NM_021110 | A_32_P80850 | TIMP1 | NM_003254 | A_23_P62115 |
| ITGB1 | NM_133376 | A_23_P104193 | LAMB3 | NM_001017402 | A_23_P86012 |
| COL11A1 | NM_080629 | A_23_P11806 | COL12A1 | NM_004370 | A_23_P21416B |
| CTNND2 | NM_001332 | A_24_P380196 | COL16A1 | NM_001856 | A_23_P16031B |
| TNC | NM_002160 | A_23_P157865 | MMP10 | NM_002425 | A_23_P13094 |
| VCAM1 | MM_001078 | A_23_P34345 | ITGB1 | NM_002211 | A_32_P95397 |
| VTN | NM_000638 | A_23_P78099 | LAMA1 | NM_005559 | A_23_P118967 |
| ITGA5 | NM_002205 | A_23_P36562 | COL12A1 | NM_004370 | A_24_P291814 |
| MMP14 | MM_004995 | A_24_P82106 | | | |

### 4) Genes Related to Epithelial-Mesenchymal Transition

Among the probes for the genes related to epithelial-mesenchymal transition contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, probe name, and GenBank accession number in Table 38 [hES_BG03 vs GC1-5] below. Further, the probes for the genes related to the epithelial-mesenchymal transition which expressions increased at least twice are plotted in the figure given below (Fig. 2). It was shown that GC1-5, which are human induced malignant stem cells derived from the primary cultured somatic (non-embryonic) cells prepared from the cancer patient's fresh (stomach) cancer tissues, relatively highly expressed the genes related to endogenous epithelial-mesenchymal transition (VIM, COL3A1, and COL1A2).

**[Table 38]**

| GeneSymbol | GenbankAccession | ProbeName | GeneSymbol | GenbankAccession | ProbeName |
|---|---|---|---|---|---|
| MMP2 | NM_004530 | A_23_P163787 | BMP7 | | A_23_P154643 |
| ITGAV | NM_002210 | A_23_P50907 | ITGA5 | NM_002205 | A_23_P36562 |
| SNAI1 | NM_005985 | A_23_P131846 | AHNAK | NM_001620 | A_23_P426636 |
| ITGB1 | NM_133376 | A_23_P104199 | CAMK2N1 | NM_018584 | A_24_P117620 |
| PLEK2 | NM_016445 | A_23_P151506 | WNT11 | NM_004626 | A_24_P35643 |
| MMP9 | MM_004994 | A_23_P40174 | MSN | NM_002444 | A_23_P73593 |
| SERPINE1 | NM_000602 | A_24_P158089 | BMP1 | NM_006129 | A_24_P60930 |
| ZEB2 | MM_014795 | A_23_P142560 | GNG11 | NM_004126 | A_23_P111701 |
| FN1 | NM_212482 | A_24_P119745 | COL1A2 | NM_000089 | A_24_P265274 |
| TMEM132A | NM_017870 | A_23_P24716 | VIM | NM_003380 | A_23_P161194 |
| COL1A2 | NM_000089 | A_24_P277934 | FN1 | NM_212482 | A_24_P85539 |
| SPARC | NM_003118 | A_23_P7642 | MSN | NM_002444 | A_23_P73589 |
| BMP7 | NM_001719 | A_24_P91566 | AHNAK | NM_024060 | A_23_P21363 |
| NOTCH1 | NM_017617 | A_23_P60387 | TGFB2 | | A_24_P148261 |
| TGFB1 | NM_000660 | A_24_P79054 | COL5A2 | NM_000393 | A_23_P10391 |
| PDGFRB | NM_002609 | A_23_P421401 | FN1 | NM_054034 | A_24_P334130 |
| WNT11 | NM_004626 | A_24_P253003 | TIMP1 | NM_003254 | A_23_P62115 |
| AHNAK | NM_001620 | A_24_P943393 | TFPI2 | NM_006528 | A_23_P393620 |
| WNT5A | NM_003392 | A_23_P211926 | COL3A1 | NM_000090 | A_24_P402242 |
| EGFR | NM_005228 | A_23_P215790 | SNAI2 | NM_003068 | A_23_P169039 |
| BMP1 | NM_001199 | A_24_P129417 | COL3A1 | NM_000090 | A_24_P935491 |
| VIM | NM_003380 | A_23_P61190 | JAG1 | NM_000214 | A_23_P210763 |
| COL3A1 | MM_000090 | A_23_P142533 | BMP1 | NM_006128 | A_23_P33277 |
| RGS2 | NM_002923 | A_23_P114947 | ITGB1 | NM_002211 | A_32_P95397 |
| ITGB1 | NM_133376 | A_23_P104193 | COL5A2 | NM_000393 | A_23_P33196 |
| BMP1 | NM_006129 | A_24_P389409 | IGFBP4 | NM_001552 | A_24_P382187 |
| KRT19 | NM_002276 | A_23_P66798 | WNT5B | NM_030775 | A_23_P53588 |
| F11R | NM_144503 | A_24_P319369 | CDH2 | NM_001792 | A_23_P38732 |
| TWIST1 | NM_000474 | A_23_P71067 | CAMK2N1 | NM_018584 | A_23_P11800 |

Also, the results of the comparisons between the human induced endodermal malignant stem cells (NGC1-1) of the present invention and the human embryonic stem cells hES_H9 (GSM194390), between NGC1-1 and the human induced pluripotent stem cells hiPS-201B7, and between the human induced endodermal malignant stem cells (CC1-10) of the present invention and the human induced pluripotent stem cells hiPS-201B7 were listed in Tables 39 [hES_H9 vs NGC1-1], 40 [hiPS-201B7 vs NGC1-1] and 41 [hiPS-201B7 vs CC1-10], respectively. It was shown that NGC1-1, which are human induced malignant stem cells derived from the primary cultured somatic cells prepared from the cancer patient's fresh (stomach) non-cancer tissues, expressed the genes related to self-renewal (POU5F1, NANOG, SOX2, ZFP42, LIN28, and TERT). It was also shown that CC1-1, which are human induced malignant stem cells derived from the primary cultured somatic (non-embryonic) cells prepared from the cancer patient's fresh (colorectal) cancer tissues, expressed the genes related to self-renewal (POU5F1, NANOG, SOX2, ZFP42, LIN28, and TERT).

**[Table 39]**

| GeneSymbol | GenbankAccession | ProbeName | GeneSymbol | GenbankAccession | ProbeName |
|---|---|---|---|---|---|
| MMP2 | NM_004530 | A_23_P163787 | TGFB3 | NM_003239 | A_24_P373096 |
| ITGAV | MM_002210 | A_23_P50907 | ITGA5 | MM_002205 | A_23_P36562 |
| ITGB1 | NM_133378 | A_23_P104199 | AHNAK | NM_001620 | A_23_P426636 |
| PLEK2 | NM_016445 | A_23_P151506 | ILK | NM_001014795 | A_24_P406870 |
| AKT1 | NM_005163 | A_23_P2960 | CAMK2N1 | NM_018584 | A_24_P117620 |
| SERPINE1 | NM_000602 | A_24_P158089 | BMP1 | NM_006129 | A_24_P60930 |
| ZEB2 | NM_014795 | A_23_P142560 | GNG11 | MM_004126 | A_23_P111701 |
| FN1 | MM_212482 | A_24_P119745 | COL1A2 | NM_000089 | A_24_P2S5274 |
| COL1A2 | NM_000089 | A_24_P277934 | VIM | MM_003380 | A_23_P161194 |
| SPARC | NM_003118 | A_23_P7642 | FN1 | NM_212482 | A_24_P85539 |
| NOTCH1 | NM_017617 | A_23_P60387 | CTNNB1 | NM_001904 | A_23_P29499 |
| TGFB1 | NM_000660 | A_24_P79054 | KRT7 | NM_005556 | A_23_P381945 |
| START3 | NM_213662 | A_23_P107206 | AHNAK | NM_024060 | A_23_P21363 |
| PDGFRB | NM_002609 | A_23_P421401 | TGFB2 | | A_24_P148261 |
| WNT11 | NM_004626 | A_24_P253003 | COL5A2 | NM_000393 | A_23_P10391 |
| AHNAK | NM_001620 | A_24_P943393 | FN1 | NM_054034 | A_24_P334130 |
| ILK | NM_001014795 | A_23_P105066 | TIMP1 | NM_003254 | A_23_P62115 |
| WNT5A | NM_003392 | A_23_P211926 | TFPI2 | NM_006528 | A_23_P393620 |
| BMP1 | NM_001199 | A_24_P129417 | COL3A1 | NM_000090 | A_24_P402242 |
| VIM | NM_003380 | A_23_P161190 | TGFB3 | NM_003239 | A_23_P88404 |
| COL3A1 | NM_000090 | A_23_P142533 | SNAI2 | NM_003068 | A_23_P169039 |
| STAT3 | NM_213662 | A_24_P116805 | COL3A1 | NM_000090 | A_24_P935491 |
| RGS2 | MM_002923 | A_23_P114947 | TFPI2 | | A_24_P95070 |
| ITGB1 | MM_133376 | A_23_P104193 | BMP1 | NM_006128 | A_23_P33277 |
| BMP1 | MM_006129 | A_24_P389409 | ITGB1 | NM_00221 | A_32_P95397 |
| KRT719 | NM_002276 | A_23_P66798 | COL5A2 | NM_000393 | A_23_P33196 |
| F11R | NM_144503 | A_24_P319369 | IGFBP4 | NM_001552 | A_24_P382187 |
| TWIST1 | NM_000474 | A_23_P71067 | WNT5B | MM_030775 | A_23_P53588 |

**[Table 40]**

| GeneSymbol | GenbankAccession | ProbeName |
|---|---|---|
| NODAL | NM_018055 | A_23_P127322 |
| MMP2 | NM_004530 | A_23_P163787 |
| ITGAV | NM_002210 | A_23_P50907 |
| DSP | NM_004415 | A_32_P157945 |
| SNAI1 | NM_005985 | A_23_P31846 |
| PLEK2 | NM_016445 | A_23_P151506 |
| SERPINE1 | NM_000602 | A_24_P158089 |
| FN1 | NM_212482 | A_24_P119745 |
| COL1A2 | NM_000089 | A_24_P277934 |
| SPARC | NM_003118 | A_23_P7642 |
| BMP7 | NM_001719 | A_24_P91566 |
| NOTCH1 | NM_017617 | A_23_P60387 |
| TGFB1 | NM_000660 | A_24_P79054 |
| PDGFRB | NM_002609 | A_23_P421401 |
| WNT11 | NM_004626 | A_24_P253003 |
| AHNAK | NM_001620 | A_24_P943393 |
| WNT5A | NM_003392 | A_23_P211926 |
| BMP1 | MM_001199 | A_24_P129417 |
| COL3A1 | NM_000090 | A_23_P142533 |
| BMP7 | NM_001719 | A_23_P68487 |
| BMP1 | NM_006129 | A_24_P389409 |

| | | |
|---|---|---|
| TGFB3 | NM_003239 | A_24_P373096 |
| GSC | NM_173849 | A_24_P232809 |
| ITGA5 | NM_002205 | A_23_P36562 |
| GSC | NM_173849 | A_23_P76774 |
| CAMK2N1 | NM_018584 | A_24_P117620 |
| BMP1 | NM_006129 | A_24_P60930 |
| GNG11 | NM_004126 | A_23_P111701 |
| COL1A2 | NM_000089 | A_24_P265274 |
| FN1 | NM_212482 | A_24_P85539 |
| KRT7 | NM_005556 | A_23_P381945 |
| AHNAK | NM_024060 | A_23_P21363 |
| FOXC2 | NM_005251 | A_24_P82358 |
| TGFB2 | | A_24_P148261 |
| COL5A2 | NM_000393 | A_23_P10381 |
| FN1 | MM_054034 | A_24_P334130 |
| TIMP1 | NM_003254 | A_23_P62115 |
| COL3A1 | MM_000090 | A_24_P402242 |
| TGFB3 | NM_003239 | A_23_P88404 |
| SNAI2 | NM_003068 | A_23_P169039 |
| COL3A1 | NM_000090 | A_24_P935491 |
| BMP1 | NM_006128 | A_23_P33277 |
| COL5A2 | NM_000393 | A_23_P33196 |
| IGFBP4 | NM_001552 | A_24_P382187 |
| WNT5B | NM_030775 | A_23_P53588 |
| CDH2 | NM_001792 | A_23_P38732 |

**[Table 41]**

| GeneSymbol | GenbankAccession | ProbeName |
|---|---|---|
| NODAL | NM_018055 | A_23_P127322 |
| MMP2 | NM_004530 | A_23_P163787 |
| DSP | NM_404415 | A_32_P157945 |
| SNAI1 | NM_005985 | A_23_P131846 |
| MMP9 | NM_004994 | A_23_P40174 |
| FN1 | NM_212482 | A_24_P119745 |
| BMP7 | NM_001719 | A_24_P91566 |
| AHNAK | NM_001620 | A_24_P943393 |
| ILK | NM_001014795 | A_23_P105066 |
| F11R | NM_144503 | A_24_P319364 |
| COL3A1 | NM_000090 | A_23_P142533 |
| BMP7 | NM_001719 | A_23_P68481 |
| KRT19 | NM_002276 | A_23_P66798 |
| GSC | NM_173849 | A_24_P232809 |
| ITGA5 | NM_002205 | A_23_P36562 |
| GSC | NM_173849 | A_23_P76774 |
| CAMK2N1 | NM_018584 | A_24_P117620 |
| MSN | NM_002444 | A_23_P73593 |
| FN1 | NM_212482 | A_24_P85539 |
| MSN | NM_002444 | A_23_P73589 |
| TCF3 | NM_003200 | A_23_P67708 |
| KRT7 | NM_005556 | A_23_P381945 |
| AHNAK | NM_024060 | A_23_P21363 |
| TGFB2 | | A_24_P148261 |
| COL5A2 | NM_000393 | A_23_P10391 |
| FN1 | NM_054034 | A_24_P334130 |
| IGFBP4 | NM_001552 | A_24_P382187 |
| CAMK2N1 | NM_018584 | A_23_P11800 |

### 5) Genes Related to Stomach Cancer

Among the probes for the genes related to stomach cancer contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 42 [hES_H9 vs NGC1-1] below. It was shown that NGC1-1, which are human induced malignant stem cells derived from the primary cultured somatic (non-embryonic) cells prepared from the cancer patient's fresh (stomach) non-cancer tissues, relatively highly expressed the genes related to endogenous stomach cancer (CCND2, TIMP3, LOX, and RASSF1).

**[Table 42]**

| GeneSymbol | GenbankAccession | ProbeName |
|---|---|---|
| CDKN2A | NM_0588197 | 23_P43490 |
| LOX | NM_002317 | A_23_P122216 |
| CDKN2A | NM_058197 | A_23_P43484 |
| MGMT | NM_002412 | A_23_P104323 |
| NID1 | NM_002508 | A_23_P200928 |
| CDH13 | NM_001257 | A_32_P85999 |
| KLF4 | NM_004235 | A_23_P32233 |
| TIMP3 | NM_000362 | A_23_P399078 |
| DKK2 | NM_014421 | A_24_P311679 |
| RASSF1 | NM_17D713 | A_24_P148777 |
| CCND2 | NM_001759 | A_24_P270235 |

### 6) Genes Related to Autonomous Growth

Among the probes for the genes related to autonomous growth contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 43 [hES_H9 vs NGC1-1] below. It was shown that NGC1-1, which are human induced malignant stem cells derived from the primary cultured somatic (non-embryonic) cells prepared from the cancer patient's fresh (stomach) non-cancer tissues, relatively highly expressed the genes related to endogenous autonomous growth (IGF2, INHBA, MDK, INHBB, and BMP1).

**[Table 43]**

| GeneSymbol | GenbankAccession | ProbeName |
|---|---|---|
| IL1B | NM_000576 | A_23_P79518 |
| INHBA | NM_002192 | A_23_P122924 |
| LIF | MM_002309 | A_24_P122137 |
| TGFB1 | NM_000660 | A_24_P79054 |
| FGF7 | NM_002009 | A_23_P14612 |
| VEGFA | NM_001025366 | A_23_P81805 |
| MDK | NM_001012334 | A_23_P116235 |
| BMP1 | MM_001199 | A_24_P129417 |
| IGF2 | NM_001007139 | A_23_P150609 |
| GDF10 | NM_004962 | A_23_P52227 |
| BMP1 | NM_006129 | A_24_P389409 |
| BMP6 | NM_001718 | A_23_P19624 |
| IL11 | NM_000641 | A_23_P67169 |
| FGF7 | NM_002009 | A_24_P99244 |
| BMP5 | MM_021073 | A_23_P19723 |
| PGF | NM_002632 | A_23_P76992 |
| HBEGF | NM_001545 | A_24_PM0608 |
| NTF3 | NM_002527 | A_23_P360797 |
| BMP1 | NM_006129 | A_24_P60930 |
| BMP4 | NM_001202 | A_23_P54144 |
| VEGFC | NM_005429 | A_23_P167096 |
| CXCL1 | NM_001511 | A_23_P7144 |
| INHBB | NM_002193 | A_23_P153964 |
| PDGFC | NM_016205 | A_23_P58396 |
| BMP1 | NM_006128 | A_23_P33271 |
| IGF2 | NM_000612 | A_23_P421379 |
| BDNF | NM_170735 | A_23_P127891 |
| CSF1 | NM_172210 | A_23_P407012 |
| NRG1 | NM_013957 | A_23_P360777 |

### 8) Genes Related to TGFβ/BMP Signaling

Among the probes for the genes related to TGFβ/BMP signaling contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_ES01 (GSM194392) and the human induced pluripotent stem cells hiPS-201B7 were listed for gene symbol, GenBank accession number, and probe name in Tables 44 [hES_ES01 vs GC1-5] and 45 [hiPS-201B7 vs GC1-5] below, respectively.

**[Table 44]**

| GeneSymbol | GenbankAccession | ProbeName |
|---|---|---|
| JUNB | NM_002229 | A_24_P241815 |
| PLAU | MM_002658 | A_23_P24104 |
| IGFBP3 | NM_001013398 | A_23_P215634 |
| COL1A1 | Z74615 | A_23_P207520 |
| NOG | NM_005450 | A_23_P341938 |
| COL1A2 | NM_000089 | A_24_P277934 |
| TGFB1I1 | NM_015927 | A_23_P141055 |
| BMP7 | NM_001719 | A_24_P91566 |
| INHBA | NM_002192 | A_23_P122924 |
| TGFB1 | NM_000660 | A_24_P79054 |
| ID2 | NM_002166 | A_23_P143143 |
| LTBP1 | NM_206943 | A_23_P43810 |
| SMAD3 | NM_005902 | A_23_P48936 |
| BGLAP | NM_199173 | A_24_P336551 |
| BMP1 | NM_001199 | A_24_P129417 |
| COL3A1 | NM_000090 | A_23_P142533 |
| CDKN1A | NM_078467 | A_24_P89457 |
| ID2 | MM_002166 | A_32_P69368 |
| TSC22D1 | NM_183422 | A_23_P162739 |
| BMP1 | MM_006129 | A_24_P389409 |
| BMP6 | NM_001718 | A_23_P19624 |

| | | |
|---|---|---|
| CDKN1A | NM_000389 | A_23_P59210 |
| BMP7 | | A_23_P154643 |
| BMP5 | MM_021073 | A_23_P19723 |
| TGFBR3 | NM_003243 | A_23_P200780 |
| DLX2 | NM_004405 | A_24_P45980 |
| TGFBI | NM_000358 | A_23_P156327 |
| BMP1 | NM_006129 | A_24_P60930 |
| COL1A2 | NM_000089 | A_24_P265274 |
| BMP4 | NM_001202 | A_23_P54144 |
| FST | NM_013409 | A_23_P110531 |
| LTBP4 | NM_003573 | A_23_P141946 |
| SMURF1 | NM_020429 | A_23_P398254 |
| INHBB | NM_002193 | A_23_P153964 |
| TGFB2 | | A_24_P148261 |
| CDKN2B | NM_078487 | A_24_P360674 |
| JUNB | NM_002229 | A_23_P4821 |
| COL3A1 | NM_000090 | A_24_P402242 |
| ACVRL1 | NM_000020 | A_24_P945113 |
| COL3A1 | NM_000090 | A_24_P935491 |
| BMP1 | NM_006128 | A_23_P33277 |
| IGFBP3 | MM_001013398 | A_24_P320699 |
| BMPER | NM_133468 | A_23_P31287 |
| GDF3 | NM_020634 | A_23_P72817 |
| CST3 | NM_000099 | A_24_P216294 |
| BAMBI | NM_012342 | A_23_P52207 |

**[Table 45]**

| GeneSymbol | GenbankAccession | ProbeName | GeneSymbol | GenbankAccession | ProbeName |
|---|---|---|---|---|---|
| NODAL | MM_018055 | A_23_P127322 | DLX2 | NM_004405 | A_24_P45980 |
| IGFBP3 | NM_001013398 | A_23_P215634 | TGFBI | NM_000358 | A_23_P158327 |
| SERPINE1 | NM_000602 | A_24_P158089 | BMPR2 | NM_001204 | A_24_P753161 |
| COL1A1 | Z74615 | A_23_P207520 | COL1A2 | NM_000089 | A_24_P265274 |
| NOG | NM_005450 | A_23_P341938 | ENG | NM_000118 | A_23_P83328 |
| COL1A2 | NM_000089 | A_24_P277934 | BMP4 | NM_001202 | A_23_P54144 |
| TGFB1I1 | NM_015927 | A_23_P141055 | FST | NM_013409 | A_23_P110531 |
| BMP7 | MM_001719 | A_24_P91566 | LTBP4 | NM_003573 | A_23_P141946 |
| NR081 | NM_000475 | A_23_P73632 | BMP2 | NM_001200 | A_23_P143331 |
| TGFB1 | NM_000660 | A_24_P79054 | INHBB | NM_002193 | A_23_P153964 |
| ID2 | NM_002166 | A_23_P143143 | TGFB2 | | A_24_P148261 |
| LTBP1 | NM_206943 | A_23_P43810 | CDKN2B | NM_078487 | A_24_P360674 |
| SMAD3 | NM_005902 | A_23_P48936 | JUNB | NM_002229 | A_23_P4821 |
| BMP1 | NM_001199 | A_24_P129417 | COL3A1 | NM_000090 | A_24_P402242 |
| COL3A1 | NM_000090 | A_23_P142533 | RUNX1 | 7C90978 | A_24_P917783 |
| ID2 | NM_002166 | A_32_P69368 | ACVRL1 | NM_000020 | A_24_P945113 |
| BMP7 | NM_001719 | A_23_P68487 | COL3A1 | NM_000090 | A_24_P935491 |
| BMP1 | NM_006129 | A_24_P389409 | CER1 | NM_005454 | A_23_P329798 |
| SMAD3 | U68019 | A_23_P359091 | LTBP2 | NM_000428 | A_24_P176173 |
| BMP6 | NM_001718 | A_23_P19624 | BMP1 | NM_006128 | A_23_P33277 |
| CDKN1A | NM_000389 | A_23_P59210 | IGFBP3 | NM_001013398 | A_24_P320699 |
| BMP7 | | A_23_P154643 | BMPER | NM_133468 | A_23_P31287 |
| GSC | NM_173849 | A_24_P232809 | GDF3 | NM_020634 | A_23_P72817 |
| GSC | NM_173849 | A_23_P76774 | CST3 | NM_004099 | A_24_P216294 |
| BMP5 | NM_021073 | A_23_P19723 | BAMBI | NM_012342 | A_23_P52207 |
| TGFBR3 | NM_003243 | A_23_P200780 | | | |

Further, the probes for the genes related to TGFβ/BMP signaling whose expressions in the human induced endodermal malignant stem cells (GC1-5) of the present invention increased at least twice as much as those in the human induced pluripotent stem cells hiPS-201B7 are plotted in the figure given below (Fig. 3). It was shown that GC1-5, which are human induced malignant stem cells derived from the primary cultured somatic (non-embryonic) cells prepared from the cancer patient's fresh (stomach) cancer tissues, relatively highly expressed the genes related to endogenous TGFβ/BMP signaling (TGFB1, IGFBP3, INHBA, and INHBB).

Also, the results of the comparisons between the human induced endodermal malignant stem cells (NGC1-1) of the present invention and the human embryonic stem cells hES_ES01 (GSM194392), between NGC1-1 and the human induced pluripotent stem cells hiPS-201B7, between the human induced endodermal malignant stem cells (CC1-10) of the present invention and the human embryonic stem cells hES_ES01 (GSM194392), and between CC1-10 and the human induced pluripotent stem cells hiPS-201B7 were listed in Tables 46 [hES_ES01 vs NGC1-1], 47 [hiPS-201B7 vs NGC1-1], 48 [hES_ES01 vs CC1-10] and 49 [hiPS-201B7 vs CC1-10] below, respectively. It was shown that NGC1-1, which are human induced malignant stem cells derived from the primary cultured somatic (non-embryonic) cells prepared from the cancer patient's fresh (stomach) non-cancer tissues, expressed the genes related to endogenous TGFβ/BMP signaling (TGFB1, IGFBP3, INHBA, and INHBB). It was also shown that CC1-10, which are human induced malignant stem cells derived from the primary cultured somatic (non-embryonic) cells prepared from the cancer patient's fresh (colorectal) cancer tissues, expressed the genes related to endogenous TGFβ/BMP signaling (TGFB1, IGFBP3, INHBA, and INHBB).

**[Table 46]**

| GeneSymbol | GenbankAccession | ProbeName |
|---|---|---|
| JUN | NM_002228 | A_23_P201538 |
| JUNB | NM_002229 | A_24_P241815 |
| IGFBP3 | NM_001013398 | A_23_P215634 |
| COL1A1 | Z74615 | A_23_P207520 |
| COL1 A2 | NM_000089 | A_24_P277934 |
| TGFB111 | NM_015927 | A_23_P141055 |
| INHBA | NM_002192 | A_23_P122924 |
| TGFB1 | NM_000660 | A_24_P79054 |
| ID2 | NM_002166 | A_23_P143143 |
| LTBP1 | NM_206943 | A_23_P43810 |
| BGLAP | NM_199173 | A_24_P336551 |
| BMP1 | NM_001199 | A_24_P129417 |
| COL3A1 | MM_000090 | A_23_P142533 |
| CDKN1A | NM_078467 | A_24_PB8457 |
| ID2 | NM_002166 | A_32_P69368 |
| TSC22D1 | NM_183422 | A_23_P162739 |
| BMP1 | NM_006129 | A_24_P389409 |
| BMP6 | NM_001716 | A_23_P19624 |
| CDKN1A | NM_000389 | A_23_P59210 |
| RUNX1 | NM_001001890 | A_24_P96403 |
| TGFB3 | NM_003239 | A_24_P373096 |

| | | |
|---|---|---|
| BMP5 | NM_021073 | A_23_P19723 |
| TGFBR3 | NM_003243 | A_23_P200780 |
| TGFBI | NM_000358 | A_23_P156327 |
| BMPR2 | NM_001204 | A_24_P753161 |
| BMP1 | MM_006129 | A_24_P60930 |
| COL1A2 | NM_000089 | A_24_P265274 |
| BMP4 | NM_001202 | A_23_P54144 |
| LTBP4 | NM_003573 | A_23_P141946 |
| SMURF1 | NM_020429 | A_23_P398254 |
| INHBB | NM_002193 | A_23_P153964 |
| TGFB2 | | A_24_PM8261 |
| CDKN2B | NM_078487 | A_24_P360674 |
| JUNB | NM_002229 | A_23_P4821 |
| COL3A1 | NM_000090 | A_24_P402242 |
| ACVRL1 | NM_000020 | A_24_P945113 |
| TGFB3 | NM_003239 | A_23_P88404 |
| COL3A1 | NM_000090 | A_24_P935491 |
| LTBP2 | NM_000428 | A_24_P178173 |
| BMP1 | NM_006128 | A_23_P33277 |
| IGFBP3 | NM_001013398 | A_24_P320699 |
| CST3 | MM_000099 | A_24_P216294 |
| EVI1 | NM_005241 | A_23_P212688 |
| TGFBR2 | NM_001024847 | A_23_P211957 |
| BAMBI | NM_012342 | A_23_P52207 |

**[Table 47]**

| GeneSymbol | GenbankAccession | ProbeName | GeneSymbol | GenbankAccession | ProbeName |
|---|---|---|---|---|---|
| NODAL | NM_018055 | A_23_P127322 | TGFBR3 | NM_003243 | A_23_P200780 |
| JUNB | NM_002229 | A_24_P241815 | TGFBI | NM_000358 | A_23_P156327 |
| IGFBP3 | NM_001013398 | A_23_P215634 | BMPR2 | NM_001204 | A_24_P753161 |
| SERPINE1 | NM_000602 | A_24_P158089 | BMP1 | NM_006129 | A_24_P60930 |
| COL1A1 | Z74615 | A_23_P207520 | COL1A2 | NM_000089 | A_24_P265274 |
| FOS | NM_005252 | A_23_P106194 | ENG | NM_000118 | A_23_P83328 |
| COL1A2 | NM_000089 | A_24_P277934 | BMP4 | NM_001202 | A_23_P54144 |
| TGFB1I1 | NM_015927 | A_23_P141055 | FST | NM_013409 | A_23_P110531 |
| BMP7 | MM_001719 | A_24_P91566 | BMP2 | NM_001200 | A_23_P143331 |
| NR0B1 | NM_000475 | A_23_P73632 | INHBB | NM_002193 | A_23_P153994 |
| TGFB1 | NM_000660 | A_24_P79054 | TGFB2 | | A_24_P148261 |
| ID2 | NM_002166 | A_23_P143143 | CDKN2B | MM_078487 | A_24_P360674 |
| LTBP1 | MM_206943 | A_23_P43810 | JUNB | NM_002229 | A_23_P4821 |
| SMAD3 | NM_005902 | A_23_P48936 | COL3A1 | NM_000090 | A_24_P402242 |
| BMP1 | NM_001199 | A_24_P129417 | RUNX1 | X90978 | A_24_P917783 |
| GOL3A1 | NM_000090 | A_23_P142533 | ACVRL1 | NM_000020 | A_24_P945113 |
| ID2 | MM_002166 | A_32_P69368 | TGFB3 | NM_003239 | A_23_P88404 |
| BMP7 | NM_001719 | A_23_P68487 | COL3A1 | NM_000090 | A_24_P935491 |
| BMP1 | NM_006129 | A_24_P389409 | CER1 | NM_005454 | A_23_P329798 |
| BMP6 | NM_001718 | A_23_P19624 | LTBP2 | MM_000428 | A_24_P176173 |
| CDKN1A | NM_000389 | A_23_P59210 | BUMP1 | NM_006128 | A_23_P33277 |
| RUNX1 | NM_001001890 | A_24_P96403 | IGFBP3 | NM_001013398 | A_24_P320699 |
| TGFB3 | NM_003239 | A_24_P373096 | CST3 | NM_000099 | A_24_P216294 |
| GSC | NM_173849 | A_24_P232809 | EVI1 | NM_005241 | A_23_P212688 |
| GSC | NM_173849 | A_23_P76774 | TGFBR2 | NM_001024847 | A_23_P211957 |
| BMP5 | NM_021073 | A_23_P19723 | BAMBI | NM_012342 | A_23_P52207 |

**[Table 48]**

| GeneSymbol | GenbankAccession | ProbeName |
|---|---|---|
| JUN | NM_002228 | A_23_P201538 |
| JUNB | NM_002229 | A_24_P241815 |
| STAT1 | NM_139266 | A_24_P274270 |
| BMP7 | NM_001719 | A_24_P91566 |
| NR081 | NM_000475 | A_23_P73632 |
| TGFB1 | NM_000660 | A_24_P79054 |
| STAT1 | NM_007315 | A_23_P56630 |
| ID2 | NM_002166 | A_23_P143143 |
| LTBP1 | NM_206943 | A_23_P43810 |
| BGLAP | NM_199173 | A_24_P336551 |
| BMP1 | NM_001199 | A_24_P129417 |
| COL3A1 | NM_000090 | A_23_P142533 |
| TSC22D1 | NM_183422 | A_23_P162739 |
| BMP6 | NM_001718 | A_23_P19624 |
| SMAD5 | NM_001001419 | A_23_P144944 |
| BMP7 | | A_23_P154643 |

| | | |
|---|---|---|
| SOX4 | NM_003107 | A_23_P82169 |
| GSC | NM_173849 | A_24_P232809 |
| FKBP1B | NM_054033 | |
| GSC | NM_173849 | A_23_P76774 |
| TGFBI | NM_000358 | A_23_P156327 |
| BMP1 | NM_006129 | A_24_P60930 |
| BMP4 | NM_001202 | A_23_P54144 |
| LTBP4 | NM_003573 | A_23_P141946 |
| SMURF1 | NM_020429 | A_23_P398254 |
| BMP2 | NM_001200 | A_23_P143331 |
| INHBB | NM_002193 | A_23_P153964 |
| TGFB2 | | A_24_P148261 |
| BGLAP | NM_199173 | A_23_P160638 |
| CDKN2B | NM_078487 | A_24_P360674 |
| ID1 | NM_002165 | A_23_P252306 |
| BMP1 | NM_006128 | A_23_P33277 |
| SMAD2 | NM_005901 | A_24_P202521 |
| CST3 | NM_000099 | A_24_P216294 |
| BAMBI | NM_012342 | A_23_P52207 |

**[Table 49]**

| GeneSymbol | GenbankAccession | ProbeName |
|---|---|---|
| NODAL | NM_018055 | A_23_P127322 |
| BMP7 | NM_001719 | A_24_P91566 |
| NR0B1 | NM_000475 | A_23_P73632 |
| ID2 | NM_002166 | A_23_P143143 |
| LTBP1 | NM_206943 | A_23_P43810 |
| SMAD3 | NM_005902 | A_23_P48936 |
| COL3A1 | NM_000090 | A_23_P142533 |
| ID2 | NM_002166 | A_32_P69368 |
| BMP7 | NM_001719 | A_23_P68487 |
| BMP6 | NM_001718 | A_23_P19624 |
| SMAD5 | NM_001001419 | A_23_P144944 |
| GSC | NM_173849 | A_24_P232809 |
| GSC | NM_73849 | A_23_P76774 |
| TGFBI | NM_000358 | A_23_P156327 |
| BMP4 | NM_001202 | A_23_P54144 |
| FST | NM_013409 | A_23_P110531 |
| LTBP4 | NM_003573 | A_23_P141946 |
| BMP2 | NM_001200 | A_23_P143331 |
| INHBB | NM_002193 | A_23_P153964 |
| TGFB2 | | A_24_P148261 |
| CDKN2B | NM_078487 | A_24_P360674 |
| CER1 | NM_005454 | A_23_P329788 |
| IGFBP3 | NM_001013398 | A_24_P320699 |
| GDF3 | NM_020634 | A_23_P72817 |
| CST3 | NM_000099 | A_24_P216294 |

### 9) Genes Related to Tissue invasion/Metastasis

Among the probes for the genes related to tissue invasion/metastasis contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human induced pluripotent stem cells hiPS-201B7 were listed for gene symbol, GenBank accession number, and probe name in Table 50 [hiPS-201B7 vs GC1-5] below. It was shown that GC1-5, which are human induced malignant stem cells derived from the primary cultured somatic (non-embryonic) cells prepared from the cancer patient's fresh (stomach) cancer tissues, relatively highly expressed the genes related to endogenous tissue invasion/metastasis (FST, BMP7, TGFB1, and COL3A1).

**[Table 50]**

| GeneSymbol | GenbankAccession | ProbeName |
|---|---|---|
| CDH6 | NM_004932 | A_24_P687 |
| MMP2 | NM_004530 | A_23_P163787 |
| MMP9 | NM_004994 | A_23_P40174 |
| SRC | NM_005417 | A_23_P308603 |
| CDKN2A | NM_058197 | A_23_P43484 |
| NR4A3 | NM_73198 | A_23_P398566 |
| TP53 | NM_000546 | A_23_P26810 |
| FN1 | NM_212482 | A_24_P119745 |
| TGFB1 | NM_000660 | A_24_P79054 |
| CXCR4 | NM_001008540 | A_23_P102000 |
| CDM11 | NM_001797 | A_23_P152305 |
| HGF | NM_001010931 | A_23_P93780 |
| KISS1R | NM_032551 | A_23_P101761 |
| CD82 | MM_002231 | A_23_P1782 |
| CDH6 | | A_32_P134764 |
| MTA1 | NM_04689 | A_24_P241370 |
| TIMP3 | NM_000362 | A_23_P399078 |
| COL4A2 | NM_001846 | A_23_P205031 |
| CTSK | NM_000396 | A_23_P34744 |
| FN1 | NM_212482 | A_24_P85539 |
| FN1 | NM_054034 | A_24_P334130 |
| CDH6 | NM_004932 | A_23_P214011 |
| RPSA | BC010D54 | A_32_P156237 |
| MMP10 | NM_002425 | A_23_P13094 |

Also, the results of the comparison between the human induced endodermal malignant stem cells (NGC1-1) of the present invention and the human induced pluripotent stem cells hiPS-201B7 were listed in Table 51 [hiPS-201B7 vs NGC1-1] below. Further, the probes for the genes related to tissue invasion/metastasis whose expressions increased at least twice are plotted in the figure given below (Fig. 4). It was shown that the induced malignant stem cells, which are derived from the primary cultured somatic (non-embryonic) cells prepared from the cancer patient's fresh (stomach) non-cancer tissues, relatively highly expressed the genes related to endogenous tissue invasion/metastasis (FST, BMP7, TGFB1, and COL3A1).

**[Table 51]**

| GeneSymbol | GenbankAccession | ProbeName | GeneSymbol | GenbankAccession | ProbeName |
|---|---|---|---|---|---|
| NODAL | NM_018055 | A_23_P127322 | TGFBR3 | NM_003243 | A_23_P200780 |
| JUNB | NM_002229 | A_24_P241815 | TGFBI | NM_000358 | A_23_P156327 |
| IGFBP3 | NM_001013398 | A_23_P215634 | [BMPR2 | NM_{_}001204 | A_24_P753161 |
| SERPINE1 | NM_000602 | A_24_P158089 | BMP1 | NM_006129 | A_24_P60930 |
| COL1A1 | Z74615 | A_23_P207520 | COL1A2 | MM_000089 | A_24_P265274 |
| COL1A2 | NM_000089 | A_24_P277934 | ENG | NM_000118 | A_23_P83328 |
| TGFB1I1 | MM_015927 | A_23_P141055 | BMP4 | NM_001202 | A_23_P54144 |
| BMP7 | NM_001719 | A_24_P91566 | FST | MM_013409 | A_23_P110531 |
| NR0B1 | NM_000475 | A_23_P73632 | BMP2 | NM_001200 | A_23_P143331 |
| TGFB1 | NM_000660 | A_24_P79054 | INHBB | NM_002193 | A_23_P153964 |
| ID2 | NM_002166 | A_23_P143143 | TGFB2 | | A_24_P148261 |
| LTBP1 | NM_208943 | A_23_P43810 | CDKN2B | NM_078487 | A_24_P360674 |
| SMAD3 | NM_005902 | A_23_P48936 | JUNB | NM_02229 | A_23_P4821 |
| BMP1 | NM_001199 | A_24_P129417 | COL3A1 | NM_000090 | A_24_P402242 |
| COL3A1 | MM_000090 | A_23_P142533 | RUNX1 | X90978 | A_24_P917783 |
| ID2 | MM_002166 | A_32_P69368 | IACVRL1 | NM_000020 | A_24_P945113 |
| BMP7 | NM_001719 | A_23_P68487 | TGFB3 | NM_003239 | A_23_P88404 |
| BMP1 | NM_006129 | A_24_P389409 | COL3A1 | NM_000090 | A_24_P935491 |
| BUMP6 | NM_001718 | A_23_P19624 | CER1 | NM_005454 | A_23_P329798 |
| CDKN1A | MM_000389 | A_23_P59210 | LTBP2 | NM_000428 | A_24_P176173 |
| RUNX1 | NM_001001890 | A_24_P96403 | BMP1 | NM_006128 | A_23_P33277 |
| TGFB3 | NM_003239 | A_24_P373096 | IGFBP3 | NM_001013398 | A_24_P320699 |
| GSC | NM_173849 | A_24_P232809 | EVI1 | NM_005241 | A_23_P212688 |
| GSC | NM_173849 | A_23_P76774 | TGFBR2 | NM_001024847 | A_23_P211957 |
| BMP5 | NM_021073 | A_23_P19723 | BAMBI | NM_012342 | A_23_P52207 |

### 10) Genes Related to Wnt Signaling

Among the probes for the genes related to Wnt signaling contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 52 [hES_H9 vs NGC1-1] below. Further, the probes for the genes related to Wnt signaling whose expressions increased at least twice are plotted in the figure given below (Fig. 5). It was shown that the induced malignant stem cells relatively highly expressed the genes related to endogenous Wnt signaling (CCND2, SLC9A3R1, LEF1, CTNNB1, and FRZB).

**[Table 52]**

| GeneSymbol | GanbankAccession | ProbeName |
|---|---|---|
| CCND1 | NM_053056 | A_23_P202837 |
| WNT3A | NM_033131 | A_23_P385690 |
| SFRP4 | NM_003014 | A_23_P215328 |
| WNT11 | NM_004626 | A_24_P253003 |
| RHOU | MM_021205 | A_24_P62530 |
| WNT5A | NM_003392 | A_23_P211926 |
| CXXC4 | | A_32_P66908 |
| TCF7 | NM_003202 | A_23_P7582 |
| WNT6 | NM_006522 | A_23_P119916 |
| FRZB | NM_001463 | A_23_P363778 |
| FRZB | NM_001463 | A_23_P10902 |
| AES | NM_198970 | A_24_P416728 |
| WNT4 | NM_030761 | A_23_P11787 |
| RHOU | NM_021205 | A_23_P114814 |
| WISP1 | NM_080838 | A_23_P169097 |
| SLC9A3R1 | NM_004252 | A_23_P30851 |
| CCND3 | NM_001760 | A_23_P361773 |
| CTNNB1 | NM_001904 | A_23_P29499 |
| LEF1 | MM_016269 | A_24_P20630 |
| FZD2 | NM_001466 | A_23_P141362 |
| FZD1 | NM_003505 | A_24_P38276 |
| FBXW4 | NM_022039 | A_23_P104295 |
| CCND2 | NM_001759 | A_24_P270235 |
| PITX2 | NM_1 53426 | A_23_P167367 |
| CCND1 | NM_053056 | A_24_P193011 |
| CCND3 | NM_001760 | A_23_P214464 |
| WISP1 | NM_003882 | A_23_P354694 |
| WNT5B | NM_030775 | A_23_P53588 |

### 11) Genes Related to Signal Transduction

Among the probes for the genes related to signal transduction contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_H9 (GSM194390) were listed for gene symbol, GenBank accession number, and probe name in Table 53 [hES_H9 vs GC1-5] below. It was shown that the induced malignant stem cells relatively highly expressed the genes related to endogenous signal transduction (CCL2, CDKN1A, HSPB1, RBP1, CCND1, LEF1, GADD45A, and BAX).

**[Table 53]**

| GeneSymbol | GenbankAccessíon | ProbeName |
|---|---|---|
| IGFBP3 | MM_001013398 | A_23_P215634 |
| CCND1 | NM_053056 | A_23_P202837 |
| CDKN2A | NM_058197 | A_23_P43484 |
| FN1 | NM_212482 | A_24_P119745 |
| BAX | NM_138765 | A_23_P346311 |
| CCL2 | NM_002982 | A_23_P89431 |
| CDKN1A | NM_078467 | A_24_P89457 |
| HSPB1 | NM_001540 | A_32_P76247 |
| PRKCE | NM_005400 | A_23_P250564 |
| CDKN1A | NM_000389 | A_23_P59210 |
| VCAM1 | NM_001078 | A_23_P34345 |
| BAX | NM_138763 | A_23_P346309 |
| HSPB1 | NM_001540 | A_23_P257704 |
| WISP1 | NM_080838 | A_23_P169097 |
| FN1 | NM_212482 | A_24_P85539 |
| BMP4 | NM_001202 | A_23_P54144 |
| LEF1 | NM_016269 | A_24_P20630 |
| RBP1 | NM_002899 | A_23_P257649 |
| FN1 | NM_054034 | A_24_P334130 |
| CDKN2B | MM_078487 | A_24_P360674 |
| GADD45A | NM_001924 | A_23_P23221 |
| IGFBP3 | NM_001013398 | A_24_P320699 |
| HSPB1 | NM_001540 | A_24_P86537 |
| CCND1 | NM_053056 | A_24_P193011 |
| WISP1 | NM_003882 | A_23_P354694 |

### 12) Genes Related to Notch signaling

Among the probes for the genes related to Notch signaling contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_H9 (GSM194390) were listed for gene symbol, GenBank accession number, and probe name in Table 54 [hES_H9 vs GC1-5] below. It was shown that the induced malignant stem cells relatively highly expressed the the genes related to endogenous Notch signaling (CD44, FZD2, CCND1, HES1, and CDKN1A).

**[Table 54]**

| GeneSymbol | GenbankAccession | ProbeName |
|---|---|---|
| CD44 | NM_000610 | A_23_P24870 |
| CCND1 | NM_053056 | A_23_P202837 |
| NOTCH1 | NM_017617 | A_23_P60387 |
| LMO2 | NM_005574 | A_23_P53126 |
| WNT11 | NM_004626 | A_24_P253003 |
| CDKN1A | NM_078467 | A_24_P89457 |
| CDKN1A | MM_000389 | A_23_P59210 |
| AES | NM_198970 | A_24_P416728 |
| WNT11 | NM_004626 | A_24_P35643 |
| HOXB4 | NM_024015 | A_24_P416370 |
| HES1 | NM_005524 | A_23_P17998 |
| CFLAR | AF009616 | A_23_P209394 |
| WISP1 | NM_080838 | A_23_P169097 |
| AES | NM_198969 | A_23_P341312 |
| NEURL | NM_004210 | A_23_P138492 |
| HEY1 | NM_012258 | A_32_P83845 |
| FZD2 | NM_001466 | A_23_P141362 |
| FZD1 | NM_003505 | A_24_P38276 |
| DLL1 | NM_005618 | A_23_P167920 |
| JAG1 | NM_000214 | A_23_P210763 |
| RFNG | NM_002917 | A_23_P84629 |
| CCND1 | NM_053056 | A_24_P193011 |
| MFNG | NM_002405 | A_24_P224926 |
| WISP1 | NM_003882 | A_23_P354694 |

### 13) Genes Related to Breast cancer and estrogen receptor signaling

Among the probes for the genes related to breast cancer and estrogen receptor signaling contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_H9 (GSM194390) were listed for gene symbol, GenBank accession number, and probe name in Table 55 [hES_H9 vs GC1-5] below. It was shown that the induced malignant stem cells relatively highly expressed the genes related to the endogenous breast cancer and estrogen receptor signaling (KRT18, KRT19, GSN, TFF1, and CTSB).

**[Table 55]**

| GeneSymbol | GenbankAccession | ProbeName |
|---|---|---|
| CD44 | NM_000610 | A_23_P24870 |
| PLAU | NM_002658 | A_23_P24104 |
| KRT18 | NM_000224 | A_23_P99320 |
| CCND1 | NM_053056 | A_23_P202837 |
| SERPINE1 | MM_000602 | A_24_P158089 |
| CDKN2A | MM_058197 | A_23_P43484 |
| GNAS | MM_080425 | A_24_P418809 |
| ID2 | NM_002166 | A_23_P143143 |
| THBS1 | MM_003246 | A_23_P206212 |
| TFF1 | NM_003225 | A_23_P68759 |
| KRT18 | NM_000224 | A_24_P42136 |
| EGFR | NM_005228 | A_23_P215790 |
| CDKN1A | NM_078467 | A_24_P89457 |
| HSPB1 | MM_001540 | A_32_P76247 |
| PAPPA | MM_002581 | A_23_P216742 |
| ID2 | NM_002166 | A_32_P69368 |
| FGF1 | NM_000800 | A_24_P111106 |
| KRT18 | NM_000224 | A_32_P151544 |

| | | |
|---|---|---|
| RAC2 | MM_002872 | A_23_P218774 |
| KRT19 | NM_002276 | A_23_P66798 |
| CDKN1A | NM_000389 | A_23_P59210 |
| GNAS | NM_080425 | A_24_P273666 |
| CTSB | NM_147780 | A_24_P303770 |
| TFF1 | NM_003225 | A_24_P322771 |
| IL6R | NM_000565 | A_24_P379413 |
| HSPB1 | MM_001540 | A_23_P257704 |
| KLF5 | NM_001730 | A_24_P210406 |
| CTSD | NM_001909 | A_23_P52556 |
| DLC1 | NM_182643 | A_24_P940115 |
| CLU | MM_203339 | A_23_P215913 |
| DLC1 | NM_182643 | A_23_P252721 |
| CTSB | NM_147780 | A_23_P215944 |
| KLF5 | NM_001730 | A_23_P53891 |
| NGFR | NM_002507 | A_23_P389897 |
| HSPB1 | MM_001540 | A_24_P86537 |
| CCND1 | NM_053056 | A_24_P193011 |
| FGF1 | NM_000800 | A_23_P213336 |
| GSN | NM_198252 | A_23_P255884 |
| IGFBP2 | NM_000597 | A_23_P119943 |
| CTSB | NM_147780 | A_24_P397928 |

### 14) Genes Related to Colon cancer

Among the probes for the genes related to colon cancer contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_H9 (GSM194390) were listed for gene symbol, GenBank accession number, and probe name in Table 56 [hES_H9 vs GC1-5] below. It was shown that the induced malignant stem cells relatively highly expressed the genes related to endogenous colon cancer (DKK3, SPARC, and IGF2).

**[Table 56]**

| GeneSymbol | GenbankAccession | ProbeName |
|---|---|---|
| CDKN2A | NM_058197 | A_23_P43484 |
| SPARC | NM_003118 | A_23_P7642 |
| HIC1 | BY798288 | A_23_P129856 |
| IGF2 | NM_001007139 | A_23_P150609 |
| DKK3 | NM_015881 | A_24_P261417 |
| DKK3 | NM_015881 | A_24_P918317 |
| DKK3 | NM_015881 | A_23_P162047 |
| TMEFF2 | MM_016192 | A_23P125383 |
| RASSF1 | NM_170713 | A_24_P148777 |
| IGF2 | NM_000612 | A_23_P421379 |

### 15) Genes Related to Hypoxic signaling

Among the probes for the genes related to hypoxic signaling contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 57 [hES_H9 vs NGC1-1] below. It was shown that the induced malignant stem cells relatively highly expressed the genes related to endogenous hypoxic signaling (EPAS1, TUBA4, EEF1A1, and CDC42).

**[Table 57]**

| GeneSymbol | GenbankAccession | ProbeName |
|---|---|---|
| AGPAT2 | NM_006412 | A_32_P26103 |
| PLAU | NM_002658 | A_23_P24104 |
| COL1A1 | Z74615 | A_23_P207520 |
| PPARA | MM_005036 | A_24_P570049 |
| CDC42 | NM_044472 | A_24_P42633 |
| NOTCH1 | MM_017617 | A_23_P60387 |
| NPY | NM_000905 | A_23_P256470 |
| PTX3 | NM_002852 | A_23_P121064 |
| BAX | NM_138765 | A_23_P346311 |
| VEGFA | NM_001025366 | A_23_P81805 |
| TUBA4A | MM_006000 | A_23_P84448 |
| EEF1A1 | NM_001402 | A_32_P44316 |
| SLC2A4 | NM_001042 | A_23_P107350 |

| | | |
|---|---|---|
| IGF2 | NM_001007139 | A_23_P150609 |
| ANGPTL4 | NM_139314 | A_23_P159325 |
| PEA15 | NM_003768 | A_24_P410952 |
| GNA11 | L40630 | A_24_P927886 |
| TUBA4A | NM_006000 | A_23_P102109 |
| BAX | NM_138763 | A_23_P346309 |
| ECE1 | NM_001397 | A_24_P154080 |
| HIF3A | NM_152794 | A_23_P374339 |
| GNA11 | NM_002067 | A_23_P142289 |
| CDC42 | NM_001791 | A_32_P115015 |
| ARD1A | NM_003491 | A_23_P148546 |
| UCP2 | NM_003355 | A_23_P47704 |
| CAT | NM_001752 | A_23_P105138 |
| IGF2 | NM_000612 | A_23_P421379 |
| EPAS1 | NM_0001430 | A_23_P210210 |
| EEF1A1 | NM_001402 | A_32_P47701 |

### 16) Genes Related to GPCR signaling

Among the probes for the genes related to GPCR signaling contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 58 [hES_H9 vs NGC1-1] below. It was shown that the induced malignant stem cells relatively highly expressed the genes related to endogenous GPCR signaling (GNAS, RGS2, JUNB, and AGT).

**[Table 58]**

| GeneSymbol | GenbankAccession | ProbeName |
|---|---|---|
| JUNB | NM_002229 | A_24_P241815 |
| EDN1 | NM_001955 | A_23_P214821 |
| AKT1 | NM_005163 | A_23_P2960 |
| CCND1 | NM_053056 | A_23_P202837 |
| SERPINE1 | NM_000602 | A_24_P158089 |
| COL1A1 | Z74615 | A_23_P207520 |
| AGT | NM_000029 | A_23_P115261 |
| IL1B | NM_000576 | A_23_P79518 |
| GNAS | NM_080425 | A_24_P418809 |
| VEGFA | NM_001025366 | A_23_P81805 |
| CCL2 | NM_002982 | A_23_P89431 |
| CDKN1A | NM_078467 | A_24_P89457 |
| RGS2 | NM_002923 | A_23_P114947 |
| GNAS | NM_080425 | A_24_P168581 |
| CDKN1A | NM_000389 | A_23_P59210 |
| VCAM1 | NM_001078 | A_23_P34345 |
| MAX | NM_197957 | A_23_P151662 |
| GNAS | MM_080425 | A_24_P273666 |
| JUNB | MM_002229 | A_23_P4821 |
| CCND1 | MM_053056 | A_24_P193011 |

### 17) Genes Related to Drug Resistance

Among the probes for the genes related to drug resistance contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 59 [hES_H9 vs NGC1-1] below. It was shown that the induced malignant stem cells relatively highly expressed the genes related to endogenous drug resistance (AQP1, SLC16A3, ATP6VOC, MVP, ABCG2, and ATP7B).

**[Table 59]**

| GeneSymbol | GenbankAccession | ProbeName |
|---|---|---|
| SLCO4A1 | NM_016354 | A_23_P5903 |
| AQP1 | NM_198098 | A_23_P19894 |
| ABCA1 | NM_005502 | A_24_P235429 |
| AQP1 | NM_198098 | A_23_P372834 |
| ATP7B | NM_000053 | A_23_P205228 |
| SLC7A7 | NM_003982 | A_23_P99642 |
| ATP6V0C | NM_001694 | A_24_P279220 |
| SLCO3A1 | NM_013272 | A_24_P336276 |
| ABCG2 | NM_004827 | A_23_P18713 |
| SLC31A1 | NM_001859 | A_24_P321068 |
| MVP | NM_017458 | A_23_P88819 |
| SLC3A1 | NM_000341 | A_24_P217234 |
| SLC16A3 | NM_004207 | A_23_P158725 |
| SLCO2A1 | NM_005630 | A_23_P135990 |

### 18) Genes Related to Hedgehog signaling

Among the probes for the genes related to Hedgehog signaling contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 60 [hES_H9 vs NGC1-1] below. It was shown that the induced malignant stem cells relatively highly expressed the genes related to endogenous Hedgehog signaling (CTNNB1, FGFR3, and ERBB4).

**[Table 60]**

| GeneSymbol | GenbankAccession | ProbeName |
|---|---|---|
| WNT3A | NM_033131 | A_23_P385690 |
| ZIC1 | NM_003412 | A_23_P367618 |
| WNT11 | NM_004626 | A_24_P253003 |
| WNT5A | NM_003392 | A_23_P211926 |
| FGFR3 | NM_000142 | A_23_P212830 |
| BMP6 | NM_001718 | A_23_P19624 |
| WNT6 | NM_006522 | A_23_P119916 |
| ERBB4 | NM_005235 | A_32_P183765 |
| WNT4 | NM_030761 | A_23_P11787 |
| FKBP8 | NM_012181 | A_23_P39336 |
| BMP5 | NM_021073 | A_23_P19723 |
| BMP4 | MM_001202 | A_23_P54144 |
| GREM1 | NM_013372 | A_23_P432947 |
| CTNNB1 | NM_001904 | A_23_P29499 |
| FGFR3 | NM_000142 | A_23_P500501 |
| GAS1 | NM_002048 | A_23_P83134 |
| HHAT | NM_018194 | A_23_P136355 |
| WNT5B | NM_030775 | A_23_P53588 |

### 19) Genes Related to PI3K-AKT signaling

Among the probes for the genes related to PI3K-AKT signaling contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 61 [hES_H9 vs NGC1-1] below. It was shown that the induced malignant stem cells relatively highly expressed the genes related to endogenous PI3K-AKT signaling (HSPB1, ITGB1, CTNNB1, PDGFRA, and FKBP1A).

**[Table 61]**

| GeneSymbol | GenbankAccession | ProbeName |
|---|---|---|
| ITGB1 | NM_133376 | A_23_P104199 |
| AKT1 | NM_005163 | A_23_P2960 |
| CCND1 | NM_053056 | A_23_P202637 |
| PDGFRA | AA599881 | A_32_P100379 |
| TCL1A | NM_021966 | A_23_P357717 |
| RASA1 | NM_002890 | A_23_P16939 |
| MAPK14 | NM_139013 | A_24_P283288 |
| CDC42 | NM_044472 | A_24_P42633 |
| HRAS | NM_005343 | A_23_P98183 |
| GJA1 | NM_000165 | A_23_P93591 |
| ILK | NM_001014795 | A_23_P105066 |
| HSPB1 | NM_001540 | A_32_P76247 |
| PIK3R2 | NM_005027 | A_23_P142361 |

| | | |
|---|---|---|
| ITGB1 | NM_133376 | A_23_P104193 |
| MAPK3 | NM_002746 | A_23_P37910 |
| TLR4 | NM_138554 | A_32_P66881 |
| SHC1 | NM_183001 | A_24_P68585 |
| ILK | NM_001014795 | A_24_P406870 |
| HSPB1 | NM_001540 | A_23_P251704 |
| CDC42 | NM_001791 | A_32_P115015 |
| PDGFRA | NM_006206 | A_23_P300033 |
| CTNNB1 | NM_001904 | A_23_P29499 |
| EIF4B | NM_001417 | A_24_P93251 |
| FKBP1A | NM_000801 | A_23_P154667 |
| HSPB1 | NM_001540 | A_24_P86537 |
| CCND1 | NM_053056 | A_24_P193011 |
| FKBP1A | NM_054014 | A_24_P160001 |
| RHOA | MM_001664 | A_24_P174550 |

### 20) Drug metabolism genes

Among the probes for the drug metabolism genes contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 62 [hES_H9 vs NGC1-1] below. It was shown that the induced malignant stem cells relatively highly expressed the endogenous drug metabolism genes (PKM2, GSTM3, COMT, ALDH1A1, and BLVRB).

**[Table 62]**

| GeneSymbol | GenbankAccession | ProbeName |
|---|---|---|
| PKM2 | NM_182470 | A_32_P147241 |
| GSTM3 | MM_000849 | A_24_P914434 |
| GSR | BC035691 | A_32_P31618 |
| PON3 | MM_000940 | A_23_P215549 |
| GSTA3 | NM_000847 | A_23_P253495 |
| BLVR8 | NM_000713 | A_23_P153351 |
| CYB5R3 | NM_007326 | A_24_P100277 |
| ALDH1A1 | NM_000689 | A_23_P83098 |
| PKM2 | NM_182470 | A_23_P399501 |
| COMT | NM_000754 | A_23_P251680 |
| HSD17B2 | NM_002153 | A_23_P118065 |
| GSTM3 | MM_000849 | A_23_P12343 |
| ALAD | NM_001003945 | A_23_P324278 |

### 21) Genes Related to Molecular Mechanism of Cancer

Among the probes for the genes related to molecular mechanism of cancer contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human induced pluripotent stem cells hiPS-201B7 were listed for gene symbol, GenBank accession number, and probe name in Table 63 [hiPS-201B7 vs NGC1-1] below. It was shown that the induced malignant stem cells relatively highly expressed the genes related to endogenous molecular mechanism of cancer (BAX, NFKBIA, BCL2, and CASP8).

**[Table 63]**

| GeneSymbol | GenbankAccession | ProbeName |
|---|---|---|
| ITGAV | NM_002210 | A_23_P50907 |
| HGF | | A_24_P944788 |
| CDKN2A | NM_058197 | A_23_P43490 |
| COL1A1 | Z74615 | A_23_P207520 |
| HGF | NM_001010931 | A_23_P93787 |
| SRC | NM_005417 | A_23_P308603 |
| CDKN2A | NM_058197 | A_23_P43484 |
| RELA | BC014095 | A_23_P104689 |
| TP53 | NM_000546 | A_23_P26810 |
| BAX | NM_138764 | A_23_P208706 |
| MAPK14 | NM_139013 | A_24_P283288 |
| FN1 | NM_212482 | A_24_P119745 |

| | | |
|---|---|---|
| TGFB1 | NM_000660 | A_24_P79054 |
| CASP8 | NM_033356 | A_23_P209389 |
| BCL2 | M13995 | A_23_P208132 |
| ELK1 | NM_005229 | A_23_P171054 |
| VEGFA | NM_001025366 | A_23_P81805 |
| PIK3R1 | NM_181523 | A_24_P29401 |
| HGF | NM_001010931 | A_23_P93780 |
| CDKN1A | NM_000389 | A_23_P59210 |
| BAX | NM_138763 | A_23_P346309 |
| NFKBIA | NM_020529 | A_23_P106002 |
| FN1 | NM_212482 | A_24_P85539 |
| LEF1 | NM_016269 | A_24_P20630 |
| FN1 | NM_054034 | A_24_P334130 |
| FZD1 | NM_003505 | A_24_P38276 |
| CDKN2B | NM_078487 | A_24_P360674 |
| TGFBR2 | NM_001024847 | A_23_P211957 |

### 22) Genes Related to SMAD Signaling Network

Among the probes for the genes related to SMAD signaling network contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_ES01 (GSM194392) were listed for gene symbol, GenBank accession number, and probe name in Table 64 [hES_ES01 vs NGC1-1] below. It was shown that the induced malignant stem cells relatively highly expressed the genes related to endogenous SMAD signaling network (PSMC3, HDAC5, UBB, and ACTA2).

**[Table 64]**

| GeneSymbol | GenbankAccession | ProbeName |
|---|---|---|
| RAB5C | NM_201434 | A_23_P107211 |
| HDAC5 | MM_001015053 | A_23_P26916 |
| PSMC3 | NM_002804 | A_23_P104607 |
| TGFB1 | NM_000660 | A_24_P79054 |
| UBB | NM_018955 | A_23_P27215 |
| FLNC | NM_001458 | A_24_P77968 |
| DAB2 | NM_001343 | A_23_P257871 |
| BMP1 | NM_001199 | A_24_P129417 |
| RAB5C | NM_201434 | A_23_P107214 |
| ACTA2 | NM_001613 | A_23_P150053 |
| UBD | NM_006398 | A_23_P81898 |
| BMP1 | NM_006129 | A_24_P389409 |
| ZFYVE9 | NM_004799 | A_32_P143048 |
| BMP6 | NM_001718 | A_23_P19624 |

| | | |
|---|---|---|
| TGFB3 | NM_003239 | A_24_P373096 |
| PSMC5 | MM_002805 | A_23_P164035 |
| HDAC10 | NM_032019 | A_23_P368740 |
| BMP5 | NM_021073 | A_23_P19723 |
| ZFYVE9 | NM_004799 | A_23_P33768 |
| HDAC4 | NM_006037 | A_24_P359859 |
| BMP1 | NM_006129 | A_24_P60930 |
| BMP4 | NM_001202 | A_23_P54144 |
| SMURF1 | NM_020429 | A_23_P398254 |
| HDAC3 | NM_003883 | A_23_P7388 |
| TGFB2 | | A_24_P148261 |
| TGFB3 | NM_003239 | A_23_P88404 |
| BMP1 | NM_006128 | A_23_P33277 |
| HDAC8 | NM_018486 | A_23_P84922 |
| UBR2 | NM_015255 | A_23_P362637 |
| HDAC5 | NM_001015053 | A_23_P26922 |
| TGFBR2 | MM_001024847 | A_23_P211957 |

### 23) Genes Related to Pancreatic cancer

Among the probes for the genes related to pancreatic cancer contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human induced pluripotent stem cells hiPS-201B7 were listed for gene symbol, GenBank accession number, and probe name in Table 65 [hiPS-201B7 vs NGC1-1] below. It was shown that the induced malignant stem cells relatively highly expressed the genes related to endogenous pancreatic cancer.

**[Table 65]**

| GeneSymbol | GenbankAccession | ProbeName |
|---|---|---|
| MMP2 | NM_004530 | A_23_P163787 |
| CDKN2A | NM_058197 | A_23_P43490 |
| SRC | NM_005417 | A_23_P308603 |
| CDKN2A | MM_058197 | A_23_P43484 |
| MMP1 | NM_002421 | A_23_P1691 |
| RELA | BC014095 | A_23_P104689 |
| TP53 | NM_000546 | A_23_P26810 |
| NOTCH1 | NM_017617 | A_23_P60387 |
| TGFB1 | NM_000660 | A_24_P79054 |
| BCL2 | M13995 | A_23_P208132 |
| SMAD3 | NM_005902 | A_23_P48936 |
| ELK1 | NM_005229 | A_23_P171054 |
| VEGFA | NM_001025366 | A_23_P81805 |
| PIK3R1 | NM_181523 | A_24_P29401 |
| RAC2 | NM_002872 | A_23_P218774 |
| RHOB | NM_004040 | A_23_P51136 |
| CDKN1A | NM_000389 | A_23_P59210 |
| TGFB3 | NM_003239 | A_24_P373096 |
| VEGFC | NM_005429 | A_23_P167096 |
| TGFB2 | | A_24_P148261 |
| CDKN2B | NM_078487 | A_24_P360674 |
| TGFB3 | MM_003239 | A_23_P88404 |
| TGFBR2 | NM_001024847 | A_23_P211957 |

### 24) Genes Related to Prostate Cancer

Among the probes for the genes related to prostate cancer contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_BG03 (GSM194391) were listed for gene symbol, GenBank accession number, and probe name in Table 66 [hES_BG03 vs NGC1-1] below. It was shown that the induced malignant stem cells relatively highly expressed the genes related to endogenous prostate cancer (SFRP1, TIMP2, DKK3, and DLC1).

**[Table 66]**

| GeneSymbol | GenbankAccession | ProbeName |
|---|---|---|
| CDKN2A | NM_058197 | A_23_P43490 |
| CDKN2A | NM_058197 | A_23_P43484 |
| MGMT | NM_002412 | A_23_P104323 |
| SFRP1 | NM_003012 | A_23_P10127 |
| DKK3 | NM_015881 | A_24_P261417 |
| DKK3 | NM_015881 | A_24_P918317 |
| DKK3 | NM_015881 | A_23_P162047 |
| SFRP1 | NM_003012 | A_23_P10121 |
| ZNF185 | AK095258 | A_23_P11025 |
| RASSF1 | NM_170713 | A_24_P148777 |
| DLC1 | NM_182643 | A_24_P940115 |
| TIMP2 | MM_003255 | A_23_P107401 |
| MSX1 | NM_002448 | A_23_P110430 |
| DLC1 | NM_182643 | A_23_P252721 |
| PDLIM4 | NM_003687 | A_23_P144796 |

### 26) Genes Related to Liver Cancer

Among the probes for the genes related to liver cancer contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, GenBank accession number, and probe name in Table 67 [hES_BG03 vs GC1-5] below. It was shown that the induced malignant stem cells relatively highly expressed the genes related to endogenous liver cancer (CCND2, DLC1, CDKN1A, and DAB2IP).

**[Table 67]**

| GeneSymbol | GenbankAccession | ProbeName |
|---|---|---|
| CDKN1B | NM_004064 | A_24_P81841 |
| CDKN2A | MM_058197 | A_23_P43484 |
| CDKN1A | NM_078467 | A_24_P89457 |
| CDKN1A | MM_000389 | A_23_P59210 |
| CCND2 | NM_001759 | A_24_P278747 |
| CCND2 | MM_001759 | A_23_P139881 |
| FHIT | NM_002012 | A_23_P125164 |
| RASSF1 | NM_170713 | A_24_P148777 |
| DLC1 | NM_182643 | A_24_P940115 |
| DLC1 | NM_182643 | A_23_P252721 |
| CCND2 | NM_001759 | A_24_P270235 |
| DAB2IP | NM_032552 | A_23_P123848 |

### 27) Genes Related to Lung Cancer

Among the probes for the genes related to lung cancer contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, GenBank accession number, and probe name in Table 68 [hES_BG03 vs GC1-5] below. It was shown that the induced malignant stem cells relatively highly expressed the genes related to endogenous lung cancer (CDKN1C, MGMT, RASSF2, and CADM1).

**[Table 68]**

| GeneSymbol | GenbankAccession | ProbeName |
|---|---|---|
| CDKN2A | NM_058197 | A_23_P43484 |
| MGMT | NM_002412 | A_23_P104323 |
| CYP1B1 | NM_000104 | A_23_P209625 |
| CDH13 | NM_001257 | A_32_P85999 |
| CADM1 | NM_014333 | A_23_P203120 |
| FHIT | NM_002012 | A_23_P125164 |
| RASSF1 | NM_170713 | A_24_P148777 |
| DLC1 | NM_182643 | A_24_P940115 |
| CDKN1C | NM_000076 | A_23_P428129 |
| DLC1 | NM_182643 | A_23_P252721 |
| CADM1 | NM_014333 | A_24_P227230 |
| CDKN2B | NM_078487 | A_24_P360674 |
| RASSF2 | NM_014737 | A_23_P166087 |

### 28) Genes Related to Stress and Toxicity

Among the probes for the genes related to stress and toxicity contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_H9 (GSM194390) were listed for gene symbol, GenBank accession number, and probe name in Table 69 [hES_H9 vs GC1-5] below. It was shown that the induced malignant stem cells relatively highly expressed the genes related to endogenous stress and toxicity (GDF15, GSTM3, HMOX1, and HSPA5).

**[Table 69]**

| GeneSymbol | GenbankAccession | ProbeName |
|---|---|---|
| CCND1 | NM_053056 | A_23_P202837 |
| SERPINE1 | NM_000602 | A_24_P158089 |
| CRYAB | NM_001885 | A_24_P206776 |
| HMOX1 | NM_002133 | A_23_P120883 |
| HSPA5 | NM_005347 | A_24_P98411 |
| GSTM3 | NM_000849 | A_24_P914434 |
| GSR | BC035691 | A_32_P31618 |
| BAX | NM_138765 | A_23_P346311 |
| IGFBP6 | NM_002178 | A_23_P139912 |
| CDKN1A | NM_078467 | A_24_P89457 |
| HSPB1 | NM_001540 | A_32_P76247 |
| HSPA1A | NM_005345 | A_24_P123616 |
| DNAJB4 | NM_007034 | A_23_P51339 |
| HSPA1A | NM_005345 | A_24_P682285 |

| | | |
|---|---|---|
| CDKN1A | NM_000389 | A_23_P59210 |
| BAX | NM_138763 | A_23_P346309 |
| DDIT3 | NM_004083 | A_23_P21134 |
| HSPB1 | NM_001540 | A_23_P257704 |
| TNFRSF1A | NM_001065 | A_24_P364363 |
| PRDX2 | NM_181738 | A_24_P168416 |
| ATM | BC022307 | A_24_P103944 |
| EPHX2 | NM_001979 | A_23_P8834 |
| GADD45A | NM_001924 | A_23_P23221 |
| GDF15 | NM_004864 | A_23_P16523 |
| HSPB1 | NM_001540 | A_24_P86537 |
| CCND1 | NM_053056 | A_24_P193011 |
| CAT | NM_001752 | A_23_P105138 |
| GSTM3 | NM_000849 | A_23_P12343 |
| HSPA5 | NM_005347 | A_24_P18190 |

### 30) Genes Related to Epigenetics chromatin modification enzyme

Among the probes for the genes related to epigenetics chromatin modification enzyme contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 70 [hES_H9 vs NGC1-1] below. It was shown that the induced malignant stem cells relatively highly expressed the genes related to epigenetics chromatin modification enzyme (HDAC5, SMYD3, HDAC10, and PRMT5).

**[Table 70]**

| GeneSymbol | GenbankAccession | ProbeName |
|---|---|---|
| UBE2B | NM_003337 | A_24_P200583 |
| NEK6 | MM_014397 | A_23_P216920 |
| SMYD3 | NM_022743 | A_23_P51410 |
| PRMT5 | NM_006109 | A_24_P298420 |
| HDAC5 | NM_001015053 | A_23_P26916 |
| SUV39H1 | NM_003173 | A_23_P422193 |
| MBD2 | NM_003927 | A_23_P15864 |
| SETD8 | MM_020382 | A_32_P82807 |
| RPS6KA3 | NM_004586 | A_32_P517749 |
| SETD8 | MM_020382 | A_32_P191859 |
| HDAC10 | MM_032019 | A_23_P368740 |
| USP22 | BC110499 | A_23_P207068 |
| HDAC4 | NM_006037 | A_24_P359859 |
| SETD8 | NM_020382 | A_24_P238855 |
| SMYD3 | NM_022743 | A_32_P103291 |
| PRMT2 | NM_206962 | A_23_P80156 |
| HDAC8 | NM_018486 | A_23_P84922 |
| HDAC5 | NM_001015053 | A_23_P26922 |
| MYST4 | NM_012330 | A_23_P388851 |

### 31) Stem cell transcription factor genes

Among the probes for the stem cell transcription factor genes contained in the Whole Human Genome Oligo DNA Microarray (4X44K) manufactured by Agilent Technologies, the genes whose expressions in the human induced endodermal malignant stem cells (NGC1-1) of the present invention increased at least twice as much as those in the human embryonic stem cells hES_H9 (GSM194390) were listed for gene symbol, GenBank accession number, and probe name in Table 71 [hES_H9 vs NGC1-1] below. It was shown that the induced malignant stem cells relatively highly expressed the stem cell transcription factor genes (NR2F2, PITX2, HAND1, and ZIC1).

**[Table 71]**

| GeneSymbol | GenbankAccession | ProbeName |
|---|---|---|
| ZFPM2 | NM_012082 | A_23_P168909 |
| ZIC1 | NM_003412 | A_23_P367618 |
| FOXA1 | NM_004496 | A_23_P37127 |
| STAT3 | NM_213662 | A_23_P107206 |
| HAND1 | NM_004821 | A_23_P58770 |
| STAT3 | NM_213662 | A_24_P116805 |
| HOXB3 | NM_002146 | A_24_P399220 |
| RUNX1 | NM_001001890 | A_24_P96403 |
| HOXB5 | NM_002147 | A_23_P363316 |
| KLF4 | NM_004235 | A_23_P32233 |
| NR2F2 | NM_021005 | A_24_P313354 |
| FOXA1 | NM_004496 | A_24_P347431 |
| NR2F2 | NM_021005 | A_23_P88589 |
| DACH1 | NM_080759 | A_23_P32577 |
| HTR7 | NM_019859 | A_23_P500381 |
| KLF2 | NM_016270 | A_23_P119196 |
| PITX2 | NM_153426 | A_23_P167367 |
| HOXC9 | NM_006897 | A_23_P25150 |

### 32) Hepatocyte related genes

Among the probes for the hepatocyte related genes contained in the Whole Human Genome Oligo DNA Microarray (4X44K) manufactured by Agilent Technologies, the genes whose expressions in the human induced endodermal malignant stem cells (GC1-5) of the present invention increased at least twice as much as those in the human embryonic stem cells hES_BG03 (GSM194391) were listed for gene symbol, GenBank accession number, and probe name in Table 72 [hES_BG03 vs GC1-5] below. It was shown that the induced malignant stem cells relatively highly increased in the expressions of not only any of the genes 1) to 31) but also the hepatocyte related genes (TTR, DLK1, AFP, and TF) as compared with the human embryonic stem cells hES_H9 (GSM194390).

**[Table 72]**

| GeneSymbol | GenbankAccession | ProbeName | GeneSymbol | GenbankAccession | ProbeName |
|---|---|---|---|---|---|
| HPX | NM_000613 | A_23_P161998 | PAG1 | NM_018440 | A_23_P347070 |
| TF | NM_001063 | A_23_P212500 | VTN | NM_000638 | A_23_P78099 |
| TTR | NM_000371 | A_23_P130333 | H19 | NR_002196 | A_24_P52697 |
| FABP1 | NM_001443 | A_23_P79562 | PDZK1 | NM_002614 | A_23_P52121 |
| APOA1 | NM_000039 | A_23_P203191 | ART4 | NM_021071 | A_23_P116902 |
| APOB | NM_000384 | A_23_P79591 | MAF | AF055376 | A_24_P256219 |
| AK124281 | AK124281 | A_32_P23525 | GJB1 | NM_000166 | A_23_P250444 |
| F2 | NM_000506 | A_23_P94879 | SLC40A1 | NM_014585 | A_23_P102391 |
| TF | NM_001063 | A_23_P212508 | C13orf15 | NM_014059 | A_24_P10137 |
| FOXA1 | NM_004496 | A_23_P37127 | RNF43 | NM_017763 | A_23_P3934 |
| AGT | NM_000029 | A_23_P115261 | NNMT | NM_006169 | A_23_P127584 |
| FGA | NM_021871 | A_23_P375372 | AK126405 | AK126405 | A_24_P766716 |
| C5 | MM_001735 | A_23_P71855 | ALB | NM_000477 | A_23_P257834 |
| A2M | NM_000014 | A_23_P116898 | FLRT3 | NM_98391 | A_23_P166109 |
| AK074614 | AK074614 | A_32_P56661 | DLK1 | NM_003836 | A_24_P236251 |
| SERPINA5 | MM_000624 | A_24_P321766 | NTF3 | NM_002527 | A_23_P360797 |
| SERINC2 | NM_178865 | A_24_P145629 | IL32 | NM_001012631 | A_23_P15146 |
| FGB | NM_005141 | A_23_P136125 | VIL1 | MM_007127 | A_23_P16866 |
| COLEC11 | NM_199235 | A_23_P120125 | SEPP1 | MM_005410 | A_23_P121926 |

| | | | | | |
|---|---|---|---|---|---|
| UBD | NM_006398 | A_23_P81898 | ALDH1A1 | NM_000689 | A_23_P83098 |
| C11orf9 | NM_013279 | A_23_P75790 | GATA4 | NM_002052 | A_23_P384761 |
| IGF2 | NM_001007139 | A_23_P150609 | LGALS2 | NM_006498 | A_23_P120902 |
| APOA2 | NM_001643 | A_24_P302249 | SERPINA5 | NM_000624 | A_23_P205355 |
| AHSG | NM_001622 | A_23_P155509 | CA414006 | CA414006 | A_32_P213103 |
| UGT2B11 | NM_001073 | A_23_P212968 | GATM | NM_001482 | A_23_P129064 |
| UGT2B7 | NM_001074 | A_23_P136671 | FOXA1 | NM_004496 | A_24_P347431 |
| MTTP | NM_000253 | A_23_P213171 | INHBB | NM_002193 | A_23_P153964 |
| SERPINA1 | MM_001002236 | A_23_P218111 | STARD10 | MM_006645 | A_23_P36345 |
| HMGCS2 | NM_005518 | A_23_P103588 | APOA4 | NM_000482 | A_23_P87036 |
| ATAD4 | NM_024320 | A_23_P118894 | PRG4 | MM_005807 | A_23_P160286 |
| FGG | NM_000509 | A_23_P148088 | M27126 | M27126 | A_24_P845223 |
| ASGR2 | NM_080912 | A_23_P130113 | AREG | NM_001657 | A_23_P259071 |
| SLC13A5 | MM_177550 | A_23_P66739 | S100A14 | NM_020672 | A_23_P124619 |
| RASD1 | MM_016084 | A_23_P118392 | KYNU | NM_003937 | A_23_P56898 |
| CXCR7 | NM_020311 | A_23_P131676 | LOC132205 | AK091178 | A_24_P178834 |
| F10 | NM_000504 | A_23_P205177 | ANXA8 | NM_001630 | A_32_P105549 |
| GSTA3 | NM_000847 | A_23_P253495 | RBP4 | NM_006744 | A_23_P75283 |
| C13orf15 | MM_014059 | A_23_P204937 | FTCD | NM_206965 | A_23_P91552 |
| AFP | NM_001134 | A_23_P58205 | LOC285733 | AK091900 | A_24_P463929 |
| VCAM1 | NM_001078 | A_23_P34345 | GPRC5C | NM_022036 | A_32_P109029 |

### 33) Genes Related to Self-replication

Among the probes for the genes related to self-renewal contained in the Whole Human Genome Oligo DNA Microarray (4X44K) manufactured by Agilent Technologies, the genes which the human induced endodermal malignant stem cells (GC1-5) of the present invention expressed almost (half to twice) as much as the human embryonic stem cells hES_H9 (GSM194390) were listed for gene symbol, GenBank accession number, and probe name in Table 73 [hES_H9 = GC1-5] below. Further, the probes for the genes related to the self-renewal which the human induced endodermal malignant stem cells (NGC1-1) of the present invention expressed almost (half to twice) as much as the human embryonic stem cells hES_BG03 (GSM194391) are plotted in the figure given below (Fig. 6). All of the genes related to the self-renewal listed in Table 1 were expressed almost (an eighth to eight times) as much by the human induced endodermal malignant stem cells (GC1-5) of the present invention as by the human embryonic stem cells hES_H9 (GSM194390).

**[Table 73]**

| GeneSymbol | GenbankAccession | ProbeName |
|---|---|---|
| NODAL | NM_018055 | A_23_P127322 |
| GABRB3 | NM_000814 | A_23_P14821 |
| DNMT3B | NM_175850 | A_23_P28953 |
| TERT | NM_198253 | A_23_P110851 |
| PODXL | NM_005397 | A_23_P215060 |
| GRB7 | NM_005310 | A_23_P163992 |
| TDGF1 | NM_003212 | A_32_P135985 |
| POU5F1 | NM_002701 | A_24_P144601 |
| POU5F1 | NM_002701 | A_23_P59138 |
| CDH1 | NM_004360 | A_23_P206359 |
| ACVR2B | NM_001106 | A_24_P231132 |
| ZIC3 | NM_003413 | A_23_P327910 |
| SOX2 | NM_003106 | A_23_P401055 |
| NANOG | NM_024865 | A_23_P204640 |
| FLT1 | NM_002019 | A_24_P42755 |
| ACVR2B | NM_001106 | A_23_P109950 |
| LIN28 | NM_024674 | A_23_P74895 |
| SALL4 | NM_020436 | A_23_P109072 |
| POU5F1 | NM_002701 | A_32_P132563 |
| DPPA4 | NM_018189 | A_23_P380526 |
| ACVR2B | NM_001106 | A_32_P134209 |
| SOX2 | NM_003106 | A_24_P379969 |
| CD24 | L33930 | A_23_P85250 |
| POU5F1 | NM_002701 | A_24_P214841 |

Also, the results of the comparisons between the human induced endodermal malignant stem cells (NGC1-1) of the present invention and the human embryonic stem cells hES_BG03 (GSM194391), and between the human induced endodermal malignant stem cells (CC1-10) of the present invention and the human embryonic stem cells hES_BG03 (GSM194391) were listed in Tables 74 [hES_BG03=NGC1-1] and 75 [hES_BG03=CC1-10], respectively. All of the genes related to the self-renewal listed in Table 1 were expressed almost (an eighth to eight times) as much by the human induced endodermal malignant stem cells (NGC1-1) and the human induced endodermal malignant stem cells (CC1-10) of the present invention as by the human embryonic stem cells hES_H9 (GSM194390). Therefore, it was shown that the induced malignant stem cells expressed not only any of the genes 1) to 31) but also the genes related to the self-renewal (POU5F1, NANOG, SOX2, ZFP42, LIN28, and TERT), and that their expression levels were almost (an eighth to eight times) as much as those in the human embryonic stem cells hES_H9 (GSM194390).

**[Table 74]**

| GeneSymbol | GenbankAccession | ProbeName |
|---|---|---|
| NODAL | NM_018055 | A_23_P127322 |
| EDNRB | NM_003991 | A_23_P2831 |
| GABRB3 | MM_000814 | A_23_P14821 |
| DNMT3B | NM_175850 | A_23_P28953 |
| TERT | NM_198253 | A_23_P110851 |
| PODXL | NM_005397 | A_23_P215060 |
| GRB7 | NM_005310 | A_23_P163992 |
| TDGF1 | NM_003212 | A_32_P135985 |
| POU5F1 | NM_002701 | A_24_P144601 |
| POU5F1 | MM_002701 | A_23_P59138 |
| ZFP42 | NM_174900 | A_23_P395582 |
| CDH1 | NM_004360 | A_23_P206359 |
| GABRB3 | MM_000814 | A_23_P10966 |
| ACVR2B | NM_001106 | A_24_P231132 |

| | | |
|---|---|---|
| ZIC3 | MM_003413 | A_23_P327910 |
| SOX2 | MM_003106 | A_23_P401055 |
| NANOG | NM_024865 | A_23_P204640 |
| FLT1 | NM_002019 | A_24_P42755 |
| ACVR2B | NM_001106 | A_23_P109950 |
| LIN28 | NM_024674 | A_23_P74895 |
| SALL4 | NM_020436 | A_23_P109072 |
| POU5F1 | NM_002701 | A_32_P132563 |
| DPPA4 | MM_018189 | A_23_P380526 |
| ACVR2B | NM_001106 | A_32_P134209 |
| SOX2 | NM_003106 | A_24_P379969 |
| ICYP26A1 | NM_057157 | A_23_P138655 |
| GATA6 | NM_005257 | A_23_P304450 |
| GDF3 | MM_020634 | A_23_P72817 |
| CD24 | L33930 | A_23_P85250 |
| POU5F1 | NM_002701 | A_24_P214841 |

**[Table 75]**

| GeneSymbol | GenbankAccession | ProbeName |
|---|---|---|
| NODAL | NM_018055 | A_23_P127322 |
| GABRB3 | NM_000814 | A_23_P14821 |
| DNMT3B | NM_175850 | A_23_P28953 |
| PODXL | NM_005397 | A_23_P215060 |
| TDGF1 | NM_003212 | A_32_P135985 |
| POU5F1 | NM_002701 | A_23_P59138 |
| CDH1 | NM_004360 | A_23_P206359 |
| GABRB3 | NM_000814 | A_23_P10966 |
| ACVR2B | NM_001106 | A_24_P231132 |
| ZIC3 | NM_003413 | A_23_P327910 |
| SOX2 | NM_003106 | A_23_P401055 |
| NANOG | NM_024865 | A_23_P204640 |
| FLT1 | NM_002019 | A_24_P42755 |
| ACVR2B | MM_001106 | A_23_P109950 |
| TDGF1 | NM_003212 | A_23_P366376 |
| LIN28 | MM_024674 | A_23_P74895 |
| SALL4 | NM_020436 | A_23_P109072 |
| DPPA4 | NM_018189 | A_23_P380526 |
| ACVR2B | NM_001106 | A_32_P134209 |
| GATA6 | NM_005257 | A_23_P304450 |
| GDF3 | NM_020634 | A_23_P72817 |
| CD24 | L33930 | A_23_P85250 |

Further, the probes for the genes related to the self-renewal which the human induced endodermal malignant stem cells (NGC1-1) of the present invention expressed almost (half to twice) as much as the human embryonic stem cells hES_BG03 (GSM194391) are plotted in the figure given below (Fig. 7), and the probes for the genes related to the self-renewal which the human induced endodermal malignant stem cells (CC1-10) of the present invention expressed almost (a fourth to four times) as much as the human embryonic stem cells hES_BG03 (GSM194391) are plotted in the figure given below (Fig. 8).

Therefore, it was shown that the induced malignant stem cells expressed not only any of the genes 1) to 31) but also the genes related to the self-renewal (POU5F1, NANOG, SOX2, ZFP42, LIN28, and TERT), and that their expression levels were almost (half to twice) as much as those in the human embryonic stem cells hES_H9 (GSM194390). It was also shown that the induced malignant stem cells expresses not only any of the genes 1) to 31) but also the genes related to the self-renewal (POU5F1, NANOG, SOX2, ZFP42, LIN28, and TERT), and that their expression level was almost (a fourth to four times) as much as that in the human embryonic stem cells hES_H9 (GSM194390).

The above results experimentally verified not only that the human induced malignant stem cells increased in the expression of the malignancy marker genes (cancer-related genes) which indicate the nature of cancers, as compared with the human embryonic stem cells and other cells, but also that the human induced malignant stem cells expressed the genes related to the self-renewal (POU5F1, NANOG, SOX2, ZFP42, LIN28, and TERT), which are characteristic of the embryonic stem cells, at comparable levels to the human embryonic stem cells.

### Example 6 Identification of the karyotype of the human induced malignant stem cells by band staining and mode analysis

The induced malignant stem cells (NGC1-1) of Example 3 were analyzed for karyotype and number of chromosomes by G band staining (20 cells/line) and by mode analysis (50 cells/line). As a result, all of these induced malignant stem cells were found to be normal, having a 46XY karyotype. The induced malignant stem cells (NGC1-1: Day 76 after the gene transfer) were analyzed after culture in mTeSR1 or other medium on MEF layer. The induced malignant stem cells were of a type that has a normal karyotype.

### Example 7 Chromosomal analysis of the human induced malignant stem cells by multi-color FISH

The induced malignant stem cells (NGC1-1) of Example 3 were analyzed for chromosome deletion and translocation by multi-color FISH (10 cells/line). As a result, all of these induced malignant stem cells had normal chromosomes (no chromosomal abnormalities detected). The induced malignant stem cells (NGC1-1: Day 76 after the gene transfer) were analyzed after culture in mTeSR1 or the like on MEF layer. The induced malignant stem cells were of a type that has no chromosomal abnormalities (no chromosome deletion or translocation).

It was confirmed that the self-renewal of the induced malignant stem cells (NGC1-1) of the present invention could be maintained for a long time (for at least several months to one year or longer).

### Example 8 Tumor markers in the culture supernatant of the human induced malignant stem cells

An analysis of the culture supernatant of the induced malignant stem cells (NGC1-1) as commissioned to SRL, a contract research organization, revealed the induced malignant stem cells had produced TGFβ1, AFP, procollagen type III peptide (P-III-P), type IV collagen, apolipoprotein C-II, prealbumin (TTR), and IGF-I protein. The results showed that the induced malignant stem cells were of a type that produced tumor markers.

### Example 9 Tumorigenesis in experimental animals by the human induced (malignant stem cells (preparation of a tumor bearing model)

The following experiments were performed in order to transplant the induced malignant stem cells (NGC1-1) of Example 3 into mice, observe the tissue images of the cancer cells induced by the malignant cells, and prepare a tumor bearing model.

The induced malignant stem cells (NGC1-1) together with 200 µL of Matrigel were subcutaneously transplanted into the back of NOD/SCID mice which were immunologically deficient animals, at a concentration of 5×10⁶ cells/100 µL per mouse. The cells were also transplanted into the abdominal cavity of the mice at a concentration of 5×10⁶ cells/500 µL. After 2 or 3 months, tumor was formed in the respective tumor bearing mice into which the induced malignant stem cells (NGC1-1) had been transplanted. Unlike common teratomas (benign tumors) formed from normal pluripotent stem cells, the tumors formed from the induced malignant stem cells (NGC1-1) were tissues that underwent an epithelial mesenchymal transition and formed stromal barriers. Therefore, it was shown that some of the induced malignant stem cells (NGC1-1) would form stromal barriers.

After the mice were euthanized, the malignant tumor tissues were fixed with formalin, and then paraffin sections were prepared, stained with Hematoxylin and Eosin, and examined under a microscope. Along with gut-like tissues, cancer tissues were observed which were rich in extracellular matrixes and storomal cells and formed stromal barriers. Therefore, it was confirmed that the transplanted cells were human induced malignant stem cells of a type that would undergo an epithelial mesenchymal transition.

### Example 10 Single sorting (1 cell/well) of the human induced malignant stem cells (unstained)

The induced malignant stem cells (GC1-2 and NGC1-1) prepared in Example 3 were single sorted into 96-well plates (1 cell/well) using the PERFLOW^{™} Sort manufactured by Furukawa Electric. As a result, the monoclonal induced malignant stem cells (GC1-2-1, GC1-2-2, GC1-2-3, GC1-2-4, NGC1-1-1, NGC1-1-2, NGC1-1-3, and NGC1-1-4) were established.

### Example 11 Single sorting (1 cell/well) of the human induced malignant stem cells (stained with specific antibodies)

The induced malignant stem cells (NGC1-1) prepared in Example 3 were stained with CD34 (Alexa fluor 488-conjugated mouse monoclonal anti-human CD34 antibody; Biolegend; clone: 581; mouse IgG1), VEGFR2 (Alexa fluor 647-conjugated mouse monoclonal anti-human CD309 antibody; Biolegend; clone: HKDR-1; mouse IgG1), PDGFRα (PE-conjugated anti-human CD140a; Biolegend; clone: 16A1; mouse IgG1), DLK-1 (mouse monoclonal anti-human Pref-1/DLK-1/FA1 antibody; R&D; clone#: MAB1144; mouse IgG2B), CXCR4 (Carboxyfluorescein (CFS)-conjugated mouse monoclonal anti-human CXCR4 antibody; R&D; clone#: 12G5; mouse IgG2A), E-cadherin (APC-conjugated anti-human CD324; Biolegend; clone: 67A4; mouse IgG1), IGF-R1 (mouse monoclonal anti-human IGF-IR antibody; R&D; clone#: MAB391; mouse IgG1), FABP1 (mouse monoclonal anti-human FABP1 antibody; R&D; clone#: MAB2964; mouse IgG2a), or ALB (mouse monoclonal anti-human serum albumin antibody; Sigma; clone: HAS-11; mouse IgG2a). Thereafter, the stained cells were measured for the positive stained rate using the PERFLOW^{™} Sort manufactured by Furukawa Electric. Given a high positive stained rate, positive fractions were single sorted into 96-well plates at a concentration of one cell/well.

### Example 12 Preparation of retroviral vectors for transducing the genes into cells derived from the cancer tissues of familial tumor patients

As in Example 1, a retroviral vector solution was prepared so as to enture that the relation of POU5F1 > SOX2 wasachieved. The details of the procedure are as described below.

### <Preparation of a retroviral vector solution for transducing the genes into cells derived from familial adenomatous polyposis coli (APC) patient's skin tissues>

The vectors POU5F1-pMXs, KLF4-pMXs, and SOX2-pMXs were constructed vectors (Table 33 above).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs (Cell Biolab), and 45 µL of FuGENE HD.

### <Preparation of a retroviral vector solution for transducing the genes into cells derived from retinoblastoma (RB) patient's skin tissues>

The vectors POU5F1-pMXs, KLF4-pMXs, and SOX2-pMXs were constructed vectors (Table 33 above).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs (Cell Biolab), and 45 µL of FuGENE HD.

### <Preparation of a retroviral vector solution for transducing the genes into cells derived from retinoblastoma (RB) patient's cancer tissues>

The vectors POU5F1-pMXs, KLF4-pMXs, and SOX2-pMXs were constructed vectors (Table 33 above).

The amounts of the respective vectors were as follows: 5 µg of POU5F1-pMXs, 2.5 µg of KLF4-pMXs, 1.25 µg of SOX2-pMXs, 1.25 µg of Venus-pCS2, 5 µg of VSV-G-pCMV, 1.25 µg of GFP-pMXs (Cell Biolab), and 45 µL of FuGENE HD.

### <Preparation of a retroviral vector solution for transducing the genes into cells derived from retinoblastoma (RB) patient's skin tissues>

POU5F1-KLF4-SOX2-pMXs (8.4 kb) was a vector constructed by cutting out the EcoRI-EcoRI insert fragment (3700 bp) from pCX-OKS-2A (8478 bp) and replacing it with the EcoRI-EcoRI fragment (1122 bp) of pMXs (5871 bp). Further, the fragment was confirmed to be inserted in the forward direction from 5' end to 3' end (Table 76 below).

**[Table 76]**

| **pCX-OKS-2A** | | | | | |
|---|---|---|---|---|---|
| Gene | Vector | 5' restriction enzyme | 3' restriction enzyme | Clone ID | Supplier |
| Mouse OCT3/4-2A-KLF4-2A-SOX2 | pCX | EcoRI | EcoRI | | addgene |

The amounts of the respective vectors were as follows: 3 µg of POU5F1-KLF4-SOX2-pMXs, 0.5 µg of Venus-pCS2, 2 µg of VSV-G-pCMV, and 15 µL of FuGENE HD. The use of POU5F1-KLF4-SOX2-pMXs resulted in using the genes POU5F1, KLF4, and SOX2 at a ratio of 1:1:1 in that order. The ratio of 1:1:1 may be achieved when the genes are introduced into packaging cells or may be achieved by preparing separate retroviral vector solutions for the three genes POU5F1-pMXs, KLF4-pMXs, and SOX2-pMXs, and mixing these solutions at a ratio of 1:1:1 in that order.

### Example 13 Preparation of human induced precancer stem cells from cells derived from APC patient's skin tissues

From somatic cells (frozen at passage 2)from the skin tissues of an APC patient (APC3223; a 25-year-old Caucasian male patient having an APC gene with a mutation on the 541th glutamine [541 Gln, Q: CAA or CAG] of the APC gene in which a C base was replaced by a T base to generate a stop codon), human induced endodermal precancer stem cells bearing a mutation for APC, which is an endogenous tumor suppressor gene, were established by the following procedure. One vial of cryopreserved cells derived from the skin tissues of the familial adenomatous polyposis coli patient (Coriell Institute for Medical Research, a U.S. NPO; Cat No. GM03223) was thawed in a water bath at 37°C and suspended in a D-MEM (high glucose) medium supplemented with 1X antibiotic/antimycotic and 10% FBS to give 10 mL of a cell suspension. The cells derived from the skin tissues of a familial adenomatous polyposis coli (APC) patient were precancer cells carrying a germline (genetic) mutation (541 Gln → ter: C → T) for APC in one of a pair of alleles.

Next, the resultant cell suspension was centrifuged at 1000 rpm at 4°C for 5 minutes to remove the supernatant, and thereafter the remaining cells were suspended in 20 mL of a D-MEM (high glucose) medium supplemented with 1X antibiotic/antimycotic and 10% FBS to give a cell suspension. The resultant cell suspension was added at a volume of 10 mL per well onto 90 mm diameter cell culture dishes (Nunc; Cat No. 172958) whose bottoms had been coated with matrigel at a concentration of 20 µg/cm² for at least 30 minutes, whereby the cells were seeded.

After one day, the medium was removed, and 10 mL of a retroviral vector solution containing the three genes (POU5F1, KLF4, and SOX2 at a ratio of 4:2:1 in that order) was added, and the cells were infected at 37°C for 24 hours. The viral supernatant was removed, 10 mL of a MEF conditioned ES medium was added, and the mixture was cultured at 37°C for one day. Thereafter, the medium was repeatedly replaced with a MEF conditioned ES medium every two days.

From 18 days after the gene transfer, the medium was replaced everyday with a feeder-free maintenance medium for human ES/iPS cells, mTeSR1. From 28 to 32 days after the gene transfer, the medium was repeatedly replaced everyday with a MEF conditioned ES medium. From 33 days after the gene transfer, the medium was further replaced everyday with a feeder-free maintenance medium for human ES/iPS cells, mTeSR1. Thirty-six and fourty-eight days after the gene transfer, one clone (1-1) and three clones (1-2, 1-3 and 1-4) of an human induced endodermal precancer stem cell colony were respectively picked up with forceps and transferred onto the layer of feeder cells. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of the induced malignant stem cells.

Listed below are the days (Day) when the respective clones were subjected to passage culture (p) and lysed in a buffer for an RNA collection kit (Buffer RLT).

### <Induced human endodermal precancer stem cells derived from APC patient's skin tissues>

### APC (3223) 1-1

Day 54: 24-well plate (p1) → 6-well plate (p2)
Day 60: 6-well plate (p2) → 10 cm culture dish (p3)
Day 67: Passage (p4) and cryopreservation
Day 72: Treatment with Buffer RLT (cell lysis solution before RNA purification) and cryopreservation (p5)

### APC (3223) 1-2

Day 59: 24-well plate (p1) → 6-well plate (p2)
Day 75: 6-well plate (p2) → 10 cm culture dish (p3)
Day 79: Cryopreservation

### APC (3223) 1-3

Day 59: 24-well plate (p1) → 6-well plate (p2)
Day 75: 6-well plate (p2) → 10 cm culture dish (p3)
Day 79: Cryopreservation

### APC (3223) 1-4

Day 59: 24-well plate (p1) → 6-well plate (p2)
Day 75: 6-well plate (p2) → 10 cm culture dish (p3)
Day 79: Cryopreservation

As described above, human induced precancer stem cells bearing a mutation for an endogenous tumor suppressor gene were prepared. These clones were human induced endodermal precancer stem cells carrying a germline mutation for the endogenous tumor suppressor gene (APC) in one of a pair of alleles.

### Example 14 Preparation of human induced precancer stem cells from cells derived from APC patient's skin tissues

From the skin tissues (frozen passage 2) of an APC patient (APC3946, 22-year-old Caucasian female patient who has an APC gene with a mutation on the 541th glutamine [541 1 Gln, Q: CAA or CAG] of the APC gene in which a C base was replaced by a T base to generate a stop codon and who is a younger sister of APC3223), human induced endodermal precancer stem cells were established by the following procedure. One vial of cryopreserved cells derived from the skin tissues of the familial adenomatous polyposis coli patient (Coriell; Cat No. GM03946) was thawed in a water bath at 37°C and suspended in a D-MEM (high glucose) medium supplemented with 1X antibiotic/antimycotic (Invitrogen; Cat No. 15240-062) and 10% FBS to give 10 mL of a cell suspension.

Then, the resultant cell suspension was centrifuged at 1000 rpm at 4°C for 5 minutes to remove the supernatant, and thereafter the remaining cells were resuspended in 20 mL of a D-MEM (high glucose) medium supplemented with 1X antibiotic/antimycotic and 10% FBS to give a cell suspension. The resultant cell suspension was added at a volume of 10 mL per well onto 90 mm diameter cell culture dishes whose bottoms had been coated with matrigel at a concentration of 20 µg/cm² for at least 30 minutes, whereby the cells were seeded.

After one day, the medium was removed, and 10 mL of a retroviral vector solution containing the three genes (POU5F1, KLF4, and SOX2 at a ratio of 4:2:1 in that order) was added, and the cells were infected at 37°C for 24 hours. The viral supernatant was removed, 10 mL of a MEF conditioned ES medium was added, and the mixture was cultured at 37°C for one day. Thereafter, the medium was repeatedly replaced with a MEF conditioned ES medium every two days.

From 18 days after the gene transfer, the medium was replaced everyday with a feeder-free maintenance medium for human ES/iPS cells, mTeSR1. From 28 to 32 days after the gene transfer, the medium was repeatedly replaced everyday with a MEF conditioned ES medium. From 33 days after the gene transfer, the medium was further replaced everyday with a feeder-free maintenance medium for human ES/iPS cells, mTeSR1. Thirty-six and fourty days after the gene transfer, each one clone (1-1 and 1-2) of a colony was picked up with forceps and transferred onto the layer of feeder cells to culture it with mTeSR1. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of the induced malignant stem cells.

Listed below are the days (Day) when the respective clones were subjected to passage culture (p) and lysed in a buffer for an RNA collection kit (Buffer RLT).

As described above, human induced endodermal precancer stem cells bearing a mutation for an endogenous tumor suppressor gene were prepared. These clones were human induced endodermal precancer stem cells carrying a germline mutation for the endogenous tumor suppressor gene (APC) in one of a pair of alleles.

### <Induced human endodermal precancer stem cells derived from APC patient's skin tissues>

### APC (3946) 1-1

Day 54: 24-well plate (p1) → 6-well plate (p2)
Day 61: 6-well plate (p2) → 10 cm culture dish (p3)
Day 72: Passage (p4) and cryopreservation
Day 77: Treatment with Buffer RLT (cell lysis solution before RNA purification) and cryopreservation (p5).

### APC (3946) 1-2

Day 59: 24-well plate (p1) → 6-well plate (p2)
Day 61: 6-well plate (p2) → 10 cm culture dish (p3)
Day 72: Passage (p4) and cryopreservation
Day 77: Treatment with Buffer RLT (cell lysis solution before RNA purification) and cryopreservation (p5).

As described above, human induced endodermal precancer stem cells bearing a mutation for an endogenous tumor suppressor gene were prepared. These clones were human induced endodermal precancer stem cells carrying a germline mutation for the endogenous tumor suppressor gene (APC) in one of a pair of alleles.

### Example 15 Preparation of human induced precancer stem cells from cells derived from RB patient's skin tissues

From the skin tissues (passage 3) of an RB patient (1-year-old Japanese male patient having an RB1 gene with a mutation on the 706th codon [706: TGT] of the RB1 gene in which a G base was replaced by an A base), human induced endodermal precancer stem cells carrying a mutation for the endogenous tumor suppressor gene were established by the following procedure. One vial of cryopreserved cells derived from the skin tissues of the retinoblastoma patient (National Institute of Biomedical Innovation; Resource No. KURB2358; Lot No. 080786) was thawed in a water bath at 37°C and suspended in a D-MEM (high glucose) medium supplemented with 1X antibiotic/antimycotic and 10% FBS to give 10 mL of a cell suspension.

Then, the resultant cell suspension was centrifuged at 1000 rpm at 4°C for 5 minutes to remove the supernatant, and thereafter the remaining cells were resuspended in 20 mL of the FGM-2 BulletKit to give a cell suspension. The resultant cell suspension was added at a volume of 10 mL per well onto 90 mm diameter cell culture dishes whose bottoms had been coated with matrigel at a concentration of 20 µg/cm² for at least 30 minutes, whereby the cells were seeded.

After one day, the medium was removed, the cells were washed with PBS (-), a 0.25% trypsin/1 mM EDTA solution (Invitrogen; Cat No. 25200-056) was added, and the mixture was left to stand at 37°C for 5 minutes. Then, after the 0.25% trypsin/1 mM EDTA solution was removed, 20 mL of a D-MEM (high glucose) medium supplemented with 1X antibiotic/antimycotic and 10% FBS (Invitrogen; Cat No. 26140-079) was added, and the mixture was centrifuged at 1000 rpm at 4°C for 5 minutes. After removing the supernatant, the remaining cells were suspended in 80 mL of the FGM-2 BulletKit to give a cell suspension. The resultant cell suspension was added at a volume of 10 mL per well onto 90 mm diameter cell culture dishes whose bottoms had been coated with matrigel at a concentration of 20 µg/cm² for at least 30 minutes, whereby the cells were seeded.

After one day, the medium was removed, 10 mL of a retroviral vector solution containing the three genes was added, and the cells were infected at 37°C for 24 hours. The viral supernatant was removed, 10 mL of a MEF conditioned ES medium was added, and the mixture was cultured at 37°C for one day. Thereafter, the medium was repeatedly replaced with a MEF conditioned ES medium every two days.

From 16 days after the gene transfer, the medium was replaced everyday with a feeder-free maintenance medium for human ES/iPS cells, mTeSR1. Twenty-six and thirty-three days after the gene transfer, four clones (1-4, 1-7, 1-8 and 1-9) and three clones (1-2, 1-5 and 1-6) of a colony were respectively picked up with forceps and transferred onto the layer of feeder cells. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of the induced malignant stem cells.

Listed below are the days (Day) when the respective clones were subjected to passage culture (p) and lysed in a buffer for an RNA collection kit (Buffer RLT).

### <Induced human precancer stem cells derived from RB patient's skin tissues>

### RB (203) 1-2

Day 49: 24-well plate (p1) → 6-well plate (p2)
Day 77: 6-well plate (p2) → 10 cm culture dish (p3)
Day 82: Cryopreservation

### RB (203) 1-4

Day 49: 24-well plate (p1) → 6-well plate (p2)
Day 77: 6-well plate (p2) → 10 cm culture dish (p3)
Day 82: Cryopreservation

### RB203 (1-5)

Day 53: 24-well plate (p1) → 6-well plate (p2)
Day 60: 6-well plate (p2) → 10 cm culture dish (p3)
Day 66: Passage (p4) and cryopreservation
Day 69: Treatment with Buffer RLT (cell lysis solution before RNA purification) and cryopreservation

### RB203 (1-6)

Day 53: 24-well plate (p1) → 6-well plate (p2)
Day 59: 6-well plate (p2) → 10 cm culture dish (p3)
Day 63: Passage (p4) and cryopreservation
Day 66: Treatment with Buffer RLT (cell lysis solution before RNA purification) and cryopreservation

### RB203 (1-7)

Day 56: 24-well plate (p1) → 6-well plate (p2)
Day 77: 6-well plate (p2) → 10 cm culture dish (p3)
Day 82: Cryopreservation

### RB203 (1-8)

Day 56: 24-well plate (p1) → 6-well plate (p2)
Day 77: 6-well plate (p2) → 10 cm culture dish (p3)
Day 82: Cryopreservation

### RB203 (1-9)

Day 56: 24-well plate (p1) → 6-well plate (p2)
Day 59: 6-well plate (p2) → 10 cm culture dish (p3)
Day 82: Cryopreservation

As described above, human induced endodermal precancer stem cells bearing a mutation for an endogenous tumor suppressor gene were prepared. These clones were human induced endodermal precancer stem cells carrying a monoallelic germline mutation for the endogenous tumor suppressor gene (RB1).

### Example 16 Preparation of human induced malignant stem cells from cells derived from RB patient's skin tissues

From the cancer tissues (retinoblastoma) of a familial retinoblastoma (RB) patient (1-year-old Japanese male patient having an RB1 gene with a mutation on the 706th codon [706: TGT] of the RB1 gene in which a G base was replaced by an A base), human induced malignant stem cells carrying a mutation for the endogenous tumor suppressor gene were established by the following procedure. One vial of cryopreserved cells derived from the retinoblastoma of the retinoblastoma patient (National Institute of Biomedical Innovation; Resource No. KURB2359; Lot No. 091285) was thawed in a water bath at 37°C and suspended in a D-MEM (high glucose) medium supplemented with 1X antibiotic/antimycotic and 10% FBS to give 10 mL of a cell suspension.

Then, the resultant cell suspension was centrifuged at 1000 rpm at 4°C for 5 minutes to remove the supernatant, and thereafter 40 mL of a D-MEM (high glucose) medium supplemented with 1X antibiotic/antimycotic and 10% FBS was added, and the mixture was recentrifuged again at 1000 rpm at 4°C for 5 minutes. Next, after removal of the supernatant, 20 mL of a D-MEM (high glucose) medium supplemented with 1X antibiotic/antimycotic and 10% FBS was added, and the mixture was seeded on a collagen-coated dish (100 mm) (Iwaki; Cat No. 11-018-006).

After one day, the medium was removed, and 10 mL of a retroviral vector solution containing the three genes (POU5F1, KLF4, and SOX2 at a ratio of 4:2:1 in that order) was added, and the cells were infected at 37°C for 48 hours. The viral supernatant was removed, mitomycin treated MEFs (DS Pharma Biomedical; Cat No. R-PMEF-CF) were suspended at a density of 50×10⁴ cell/cm² in 10 mL of a D-MEM (high glucose) medium supplemented with 1X antibiotic/antimycotic and 10% FBS, and then the suspension was seeded on a collagen-coated dish (100 mm) on which the transduced cells derived from the cancer tissues of the RB patient had been cultured, whereby co-culture was performed.

Then, the medium was repeatedly replaced with a MEF conditioned ES medium every three days, and from 21 days after the gene transfer, the medium was replaced everyday with a feeder-free maintenance medium for human ES/iPS cells, mTeSR1.

From twenty-one days after the gene transfer, five clones (1-1, 1-2, 1-3, 1-4 and 1-6) of a colony was picked up with forceps and transferred onto the layer of feeder cells. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of the induced malignant stem cells.

Listed below are the days (Day) when the respective clones were subjected to passage culture (p) and lysed in a buffer for an RNA collection kit (Buffer RLT).

### <Induced human malignant stem cells derived from RB patient's cancer tissues>

### RBT203 (1-1)

Day 28: 24-well plate (p1) → 6-well plate (p2)
Day 33: 6-well plate (p2) → 10 cm culture dish (p3)
Day 38: Treatment with Buffer RLT (cell lysis solution before RNA purification) and cryopreservation

### RBT203 (1-2)

Day 32: 24-well plate (p1) → 6-well plate (p2)
Day 56: 6-well plate (p2) → 10 cm culture dish (p3)
Day 61: Cryopreservation

### RBT203 (1-3)

Day 32: 24-well plate (p1) → 6-well plate (p2)
Day 56: 6-well plate (p2) → 10 cm culture dish (p3)
Day 61: Cryopreservation

### RBT203 (1-4)

Day 32: 24-well plate (p1) → 6-well plate (p2)
Day 39: 6-well plate (p2) → 10 cm culture dish (p3)
Day 45: Passage and cryopreservation
Day 48: Treatment with Buffer RLT (cell lysis solution before RNA purification) and cryopreservation

### RBT203 (1-6)

Day 32: 24-well plate (p1) → 6-well plate (p2)
Day 39: 6-well plate (p2) → 10 cm culture dish (p3)
Day 45: Passage and cryopreservation
Day 48: Treatment with Buffer RLT (cell lysis solution before RNA purification) and cryopreservation

As described above, human induced malignant stem cells bearing a mutation for an endogenous tumor suppressor gene were prepared. These clones were human induced malignant stem cells carrying a monoallelic germline mutation for the endogenous tumor suppressor gene (RB1).

### Example 17 Preparation of human induced precancer stem cells from cells derived from RB patient's skin tissues

From the skin tissues (passage 1) of an RB patient (2-year-old Japanese female patient), human induced precancer stem cells were established by the following procedure. One vial of cryopreserved somatic cells derived from the skin tissues of the retinoblastoma patient (National Institute of Biomedical Innovation; Resource No. KURB2435; Lot No. 080687) was thawed in a water bath at 37°C and suspended in a D-MEM (high glucose) medium supplemented with 1X antibiotic/antimycotic and 10% FBS to give 20 mL of a cell suspension.

Then, the resultant cell suspension was centrifuged at 1000 rpm at 4°C for 5 minutes to remove the supernatant, and thereafter the remaining cells were suspended in 20 mL of the FGM-2 BulletKit to give a cell suspension. The resultant cell suspension was added at a volume of 10 mL per well onto 90 mm diameter cell culture dishes whose bottoms had been coated with matrigel at a concentration of 20 µg/cm² for at least 30 minutes, whereby the cells were seeded.

After two days, the medium was removed, and 10 mL of a retroviral vector solution containing the three genes was added, and the cells were infected at 37°C for 24 hours. The viral supernatant was removed, 10 mL of a MEF conditioned ES medium was added, and the mixture was cultured at 37°C for one day. Thereafter, the medium was repeatedly replaced with a MEF conditioned ES medium every two days.

Fourteen days after the gene transfer, the medium was replaced with a feeder-free maintenance medium for human ES/iPS cells, mTeSR1. Again, from 18 days after the gene transfer, the medium was repeatedly replaced with a MEF conditioned ES medium every two days. Thirty-six days after the gene transfer, the medium was replaced with a feeder-free maintenance medium for human ES/iPS cells, mTeSR1. Thirty-eight days after the gene transfer, the medium was replaced with a MEF conditioned ES medium, and from 51 days after the gene transfer, the medium was repeatedly replaced everyday with a feeder-free maintenance medium for human ES/iPS cells, mTeSR1. Fifty-three days after the gene transfer, two clones (1-1 and 1-2) of a colony were picked up with forceps and transferred onto the layer of feeder cells. It should be noted that the feeder cells, which were mitomycin treated mouse embryonic fibroblasts (DS Pharma Biomedical; Cat No. R-PMEF-CF), had been seeded on a gelatin-coated 24-well plate (Iwaki; Cat No. 11-020-012) at 5.0×10⁴ cells/cm² on the day before the pickup of the induced malignant stem cells.

Listed below are the days (Day) when the respective clones were subjected to passage culture (p) and lysed in a buffer for an RNA collection kit (Buffer RLT).

### <Induced human precancer stem cells derived from RB patient's skin tissues>

### RB (243) 1-1

Day 61: 24-well plate (p1) → 6-well plate (p2)
Day 65: 6-well plate (p2) → 10 cm culture dish (p3)
Day 74: Cryopreservation

### RB (243) 1-2

Day 61: 24-well plate (p1) → 6-well plate (p2)
Day 65: 6-well plate (p2) → 10 cm culture dish (p3)
Day 74: Cryopreservation

As described above, human induced precancer stem cells bearing a mutation for an endogenous tumor suppressor gene were prepared. These clones were human induced precancer stem cells carrying a germline mutation for the endogenous tumor suppressor gene (RB 1) in one of a pair of alleles.

### Example 18 Microarray-based quantitative analysis of induced malignant stem cells derived from the cancer tissues of a familial tumor patient

The human induced malignant stem cells (RBT203 (1-1)) was analyzed using the Whole Human Genome Oligo DNA Microarray (4X44K) manufactured by Agilent Technologies. The analysis software used was GeneSpring GX 10.0 (Agilent Technologies, Inc.) and normalization was performed using the 50th percentile method. The testing procedure was the same as in Example 5.

### <Preparation of total RNAs and genomic DNAs>

The total RNAs and genomic DNAs of the human induced malignant stem cells (RBT203 (1-1)) prepared in Example 16 were extracted from the solutions that had been treated with Buffer RLT (cell lysis solution before RNA purification), using the AllPrep DNA/RNA Mini Kit (50) (Qiagen; Cat No. 80204).

### (1) Quality check of genomic DNAs

The DNA concentrations and purities were assessed using the NanoDrop ND-1000 (NanoDrop Technologies), and as a result, every sample was verified to have adequate concentration and high purity.

### (2) Quality check of total RNAs

The total RNAs were checked for their quality on the Agilent 2100 Bioanalyzer (Agilent Technologies) using the RNA LabChip (registered trademark of Agilent Technologies) Kit, and all of the RNA samples were found to be of good quality. The RNA concentrations and purities were also assessed using the NanoDrop ND-1000 (NanoDrop Technologies), and as a result, every sample was verified to contain the total RNA in an amount required for cRNA synthesis and at a high level of purity.

### (1) Genes Related to Angiogenesis

Among the probes for the genes related to angiogenesis contained in the Whole Human Genome Oligo DNA Microarray (4X44K) manufactured by Agilent Technologies, the genes whose expressions in the human induced malignant stem cells RBT203 (1-1) of the present invention increased at least twice as much as those in the human embryonic stem cells hES_ES01 (GSM194392) were listed for gene symbol, GenBank accession number, and probe name in Table 77 [hES_ES01 vs RBT203 (1-1)] below. Further, the probes for the genes related to angiogenesis whose expressions increased at least twice are plotted in the figure given below (Fig. 9). These results showed that the human induced malignant stem cells were of a type that increased in the expressions in the genes related to angiogenesis at least twice.

**[Table 77]**

| GeneSymbol | GenbankAccession | ProbeName |
|---|---|---|
| MMP2 | NM_004530 | A_23_P163787 |
| PLAU | NM_002658 | A_23_P24104 |
| AKT1 | NM_005163 | A_23_P2960 |
| EFNA1 | NM_004428 | A_23_P113005 |
| TGFB1 | NM_040660 | A_24_P79054 |
| KDR | NM_002253 | A_24_P71973 |
| COL18A1 | NM_030582 | A_24_P57426 |
| SPHK1 | NM_021972 | A_23_P38106 |
| EFNB2 | NM_004093 | A_23_P428139 |
| VEGFA | NM_001025366 | A_23_P81805 |
| CXCL3 | MM_002090 | A_24_P183150 |
| MDK | NM_001012334 | A_23_P116235 |
| VEGFA | NM_003376 | A_23_P70398 |
| ANGPTL4 | NM_139314 | A_23_P159325 |
| FGFR3 | MM_000142 | A_23_P212830 |
| ANGPT2 | NM_001147 | A_23_P60079 |
| ANPEP | NM_001150 | A_23_P88626 |

| | | |
|---|---|---|
| EFNA1 | NM_004428 | A_23_P254512 |
| NRP1 | NM_003873 | A_24_P135322 |
| NRP2 | NM_201266 | A_23_P209669 |
| ID3 | NM_002167 | A_23_P137381 |
| VEGFA | MM_001025366 | A_24_P12401 |
| NRP2 | NM_201266 | A_23_P393727 |
| SERPINF1 | NM_002615 | A_23_P100660 |
| VEGFA | NM_003376 | A_24_P179400 |
| EFNB2 | NM_004093 | A_24_P355944 |
| TGFB2 | | A_24_P148261 |
| TNFAIP2 | NM_006291 | A_23_P421423 |
| FGFR3 | NM_000142 | A_23_P500501 |
| T1MP1 | MM_003254 | A_23_P62115 |
| ID1 | NM_002165 | A_23_P252306 |
| JAG1 | MM_000214 | A_23_P210763 |
| PDGFA | NM_002607 | A_23_P113701 |
| NRP1 | NM_043873 | A_24_P928052 |
| KDR | NM_002253 | A_23_P58419 |
| NOTCH4 | NM_004557 | A_23_P365614 |

### (2) Genes Related to Signal Transduction

Among the probes for the genes related to signal transduction contained in the Whole Human Genome Oligo DNA Microarray (4X44K) manufactured by Agilent Technologies, the genes whose expressions in the human induced malignant stem cells RBT203 (1-1) increased at least twice as much as those in the human induced pluripotent stem cells hiPS-201B7 were listed for gene symbol, GenBank accession number, and probe name in Table 78 [hiPS-201B7 vs RBT203-1-1] below. Further, the probes for the genes related to signal transduction whose expressions increased at least twice are plotted in the figure given below (Fig. 10). These results showed that the human induced malignant stem cells were of a type that increased in the expressions in the genes related to signal transduction at least twice.

**[Table 78]**

| GeneSymbol | GenbankAccession | ProbeName |
|---|---|---|
| CCND1 | NM_053056 | A_24_P124550 |
| CCND1 | MM_053056 | A_23_P202837 |
| FOS | NM_005252 | A_23_P106194 |
| TP53 | NM_000546 | A_23_P26810 |
| BAX | NM_138764 | A_23_P208706 |
| EGR1 | MM_001964 | A_23_P214080 |
| FN1 | MM_212482 | A_24_P119745 |
| BAX | NM_138765 | A_23_P346311 |
| FOXA2 | NM_021784 | A_24_P365515 |
| CCL2 | NM_002982 | A_23_P89431 |
| HSPB1 | NM_001540 | A_32_P76247 |
| TCF7 | NM_003202 | A_23_P7582 |
| VEGFA | NM_003376 | A_23_P70398 |
| BAX | NM_138763 | A_23_P346309 |
| HSPB1 | MM_001540 | A_23_P257704 |
| FN1 | NM_212482 | A_24_P85539 |
| VEGFA | MM_003376 | A_24_P179400 |
| BMP4 | NM_001202 | A_23_P54144 |
| BMP2 | NM_001200 | A_23_P143331 |
| LEF1 | NM_016269 | A_24_P20630 |
| FN1 | NM_054034 | A_24_P334130 |
| FOXA2 | MM_021784 | A_24_P365523 |
| FOXA2 | NM_021784 | A_23_P500936 |
| FASN | MM_004104 | A_23_P44132 |
| IGFBP3 | MM_001013398 | A_24_P320699 |
| HSPB1 | MM_001540 | A_24_P86537 |
| GYS1 | NM_002103 | A_23_P208698 |

### (3) Genes Related to Self-renewal

Among the probes for the genes related to self-renewal contained in the Whole Human Genome Oligo DNA Microarray (4X44K) manufactured by Agilent Technologies, the genes which the human induced malignant stem cells RBT203 (1-1) expressed almost (half to twice) as much as the human embryonic stem cells HES_ES01 (GSM194392) were listed for gene symbol, GenBank accession number, and probe name in Table 79 [hES_ESO1=RBT203-1-1] below. Further, the probes for the genes related to the self-renewal which RBT203 expressed almost (half to twice) as much as hES_ES01 are plotted in the figure given below (Fig. 11).

These results showed that the human induced malignant stem cells were of a type that expressed the six types of genes (genes related to self-renewal) consisting of POU5F1, NANOG, SOX2, ZFP42, LIN28, and TERT genes.

**[Table 79]**

| GeneSymbol | GenbankAccession | ProbeName |
|---|---|---|
| NODAL | NM_018055 | A_23_P127322 |
| GABRB3 | NM_000814 | A_23_P14821 |
| DNMT3B | MM_175850 | A_23_P28953 |
| TERT | NM_198253 | A_23_P110851 |
| PODXL | NM_005397 | A_23_P215060 |
| TDGF1 | NM_003212 | A_32_P135985 |
| POU5F1 | NM_002701 | A_23_P59138 |
| CDH1 | NM_004360 | A_23_P206359 |
| GABRB3 | NM_000814 | A_23_P10966 |
| ACVR2B | MM_001106 | A_24_P231132 |
| ZIC3 | NM_003413 | A_23_P327910 |
| SOX2 | NM_003106 | A_23_P401055 |
| NANOG | MM_024865 | A_23_P204640 |
| FLT1 | NM_002019 | A_24_P42755 |
| ACVR2B | NM_001106 | A_23_P109950 |
| TDGF1 | NM_003212 | A_23_P366376 |
| LIN28 | NM_024674 | A_23_P74895 |
| SALL4 | NM_020436 | A_23_P109072 |
| DPPA4 | NM_018189 | A_23_P380526 |
| ACVR2B | NM_001106 | A_32_P134209 |
| CYP26A1 | MM_057157 | A_23_P138655 |
| CD24 | L33930 | A_23_P85250 |

The above results experimentally verified not only that the human induced malignant stem cells derived from the cancer tissues of a patient with a familial tumor, more specifically retinoblastoma (RB), increased in the expression of the cancer-related genes, but also that the human induced malignant stem cells expressed the genes that are characteristic of the embryonic stem cells, at comparable levels to the human embryonic stem cells.

### INDUSTRIAL APPLICABILITY

The induced cancer stem cells of the present invention maintain (keep intact) the aberrations inherent in the starter somatic cell, such as (a) a mutation in a tumor suppressor gene or (b) increased expression of a cancer-related gene and they are also capable of self-renewal without limit. Hence, the induced cancer stem cells of the present invention can be effectively cultured in the passage culture condition for an extended period and easily induced to cancer cells having the properties of tissue cells and, as a result, they are extremely useful in cancer therapy research and the research for cancer-related drug discovery, as applicable in methods of screening such as a method of screening for targets of anti-cancer drug discovery, a method of screening for anti-cancer therapeutic drugs, and a method of screening for cancer diagnostic drugs, as well as in methods of preparing anti-cancer vaccines and cancer model animals.

## Claims

1. An induced cancer stem cell which is an induced precancer stem cell or an induced malignant stem cell, wherein the induced cancer stem cell has the following two characteristics:
(1) expressing the six genes *POU5F1, NANOG,* SOX2, *ZFP42, LIN28,* and *TERT*; and
(2) having an aberration which is either (a) a mutation in an endogenous tumor suppressor gene or (b) increased expression of an endogenous cancer-related gene.

2. The induced cancer stem cell according to claim 1, wherein the self-renewal related genes as referred to in (1) above are expressed in the induced cancer stem cell in amounts ranging from one-eighth to eight times the amounts of the genes that are expressed in an embryonic stem cell.

3. The induced cancer stem cell according to claim 1 or 2, which is an induced precancer stem cell.

4. The induced cancer stem cell according to claim 3, wherein the tumor suppressor gene referred to (a) is *APC* or *RB1.*

5. The induced cancer stem cell according to claim 1 or 2, which is an induced malignant stem cell.

6. The induced cancer stem cell according to claim 5, wherein the cancer-related gene referred to (b) is within at least one group of genes selected from the groups of genes consisting of a group of genes related to angiogenesis, a group of cancer-related pathway genes, a group of genes related to stromal barrier, a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer.

7. The induced cancer stem cell according to claim 5 or 6, wherein in addition to the endogenous cancer-related gene referred to in (b), at least one endogenous gene selected from the groups of genes consisting of a group of genes related to stress and toxicity, a group of genes for epigenetics of chromatin modifying enzyme, and a group of genes for stem cell transcription factor undergoes an increased genetic expression.

8. The induced cancer stem cell according to any one of claims 5 to 7, wherein in addition to the endogenous cancer-related gene as referred to in (b), at least one endogenous gene selected from the group of hepatocyte-specific genes undergoes an increased genetic expression.

9. The induced cancer stem cell according to any one of claims 5 to 8, which further expresses a gene characteristic of mesendodermal stem cells or endodermal stem cells.

10. The induced cancer stem cell according to claim 9, wherein the gene characteristic of mesendodermal stem cells is *GSC* and the gene characteristic of endodermal stem cells is at least one member selected from *GSC, GATA4, FOXA2,* and *SOX17.*

11. A process for producing an induced cancer stem cell, which is either an induced precancer stem cell or an induced malignant stem cell and has the characteristics (1) and (2) recited in claim 1, from a starter somatic cell consisting of a somatic cell isolated from a mammal having (a) a mutation in a tumor suppressor gene or a a non-embryonic starter somatic cell that is isolated from a carcinogenic mammal, the process being **characterized by** performing an induction step in which the starter somatic cell is brought to such a state that the genetic products of *POU5F1, KLF4,* and *SOX2* are present therein.

12. The process for producing an induced cancer stem cell according to claim 11, wherein the genetic products of *POU5F1, KLF4,* and *SOX2* are such that their relative abundances in the starter somatic cell satisfy the relation of *POU5F1* > *SOX2.*

13. The process for producing an induced cancer stem cell according to claim 11 or 12, which uses *POU5F1*, *KLF4,* and *SOX2* or genetic products of these genes.

14. The process for producing an induced cancer stem cell according to any one of claims 11 to 13, which includes the step of sorting a single cell in one well and proliferating the cell.

15. The process for producing an induced cancer stem cell according to any one of claims 11 to 14, which further includes a selection step in which the malignancy or a specific marker of the induced cancer stem cell capable of self-renewal *in vitro* is identified to select the cell of interest.

16. The process for producing an induced cancer stem cell according to claim 15, wherein the selection step is such that a cell obtained by induction treatment of a starter somaic cell selected from the group consisting of a somatic cell isolated from a mammal having (a) a mutation in a tumor suppressor gene and a non-embryonic somatic cell that is isolated from a carcinogenic mammal is compared with an induced mesendodermal stem cell, an induced endodermal stem cell or an induced pluripotent stem cell as induced from a reference somatic cell isolated from a mammal, or an embryonic stem cell, and malignancy or a specific marker is identified to select the cell of interest.

17. The process for producing an induced cancer stem cell according to claim 15 or 16, wherein the selection step is conducted by identifying the increased expression of a cancer-related gene which is within at least one group of genes selected from the groups of genes consisting of a group of genes related to angiogenesis, a group of cancer-related pathway genes, a group of genes related to stromal barrier, a group of genes related to epithelial-mesenchymal transition, a group of genes related to stomach cancer, a group of genes related to autonomous growth, a group of genes related to TGF β/BMP signaling, a group of genes related to tissue invasion/metastasis, a group of genes related to Wnt signaling, a group of genes related to signal transduction, a group of genes related to Notch signaling, a group of genes related to breast cancer and estrogen receptor signaling, a group of genes related to colon cancer, a group of genes related to hypoxic signaling, a group of genes related to GPCR signaling, a group of genes related to drug resistance, a group of genes related to Hedgehog signaling, a group of genes related to PI3K-AKT signaling, a group of drug metabolism genes, a group of genes related to molecular mechanism of cancer, a group of genes related to SMAD signaling network, a group of genes related to pancreatic cancer, a group of genes related to prostate cancer, a group of genes related to liver cancer, and a group of genes related to lung cancer.

18. A method of screening which is selected from a method of screening for a target in anti-cancer drug discovery, a method of screening for an anti-cancer therapeutic drug, and a method of screening for a cancer diagnostic drug, wherein the method is **characterized by** using the induced cancer stem cell according to any one of claims 1 to 10.

19. A method of preparing an anti-cancer vaccine which is **characterized by** using the induced cancer stem cell according to any one of claims 1 to 10.

20. A method of preparing a cancer model animal which is **characterized in that** the induced cancer stem cell according to any one of claims 1 to 10 is transplanted to an experimental animal.
